# EUROPÄISCHE PATENTSCHRIFT

(11) **EP 3 390 886 B1**
(45) Veröffentlichungstag und Bekanntmachung des Hinweises auf die Patenterteilung: **13.05.2020**
(21) Anmeldenummer: 16831619.8
(22) Anmeldetag: 14.12.2016
(51) Int. Cl.: F16M 11/04, B60R 11/00, A61F 2/68, A61F 2/50, A61F 2/76, A43B 1/00, F16B 7/20, F16B 1/00, A61F 2/78, A43C 11/14, F16M 13/02

(54) **VERSCHLUSSVORRICHTUNG MIT EINER VERSTELLEINRICHTUNG ZUM SELBSTTÄTIGEN DREHEN EINES VERBINDUNGSELEMENTS EINES VERSCHLUSSTEILS IN EINE SCHLIESSSTELLUNG**
CLOSURE DEVICE WITH AN ADJUSTING DEVICE FOR AUTOMATICALLY ROTATING A CONNECTION ELEMENT OF A CLOSURE PART INTO A CLOSED POSITION
SYSTÈME DE FERMETURE MUNI D'UN DISPOSITIF DE DÉPLACEMENT PERMETTANT UNE ROTATION AUTOMATIQUE D'UN ÉLÉMENT DE RACCORDEMENT D'UNE PIÈCE DE FERMETURE DANS UNE POSITION DE FERMETURE

(30) Priorität: 16.12.2015 DE 102015225438
(43) Veröffentlichungstag der Anmeldung: 24.10.2018
(73) Patentinhaber: Fidlock GmbH, 30659 Hannover (DE)
(72) Erfinder: RICHTER, Friedemann, 30453 Hannover (DE); BOTKUS, Breido, 30175 Hannover (DE); FIEDLER, Joachim, 30175 Hannover (DE)
(74) Vertreter: Maikowski & Ninnemann Patentanwälte Partnerschaft mbB
(86) Internationale Anmeldenummer: PCT/EP2016/081060
(87) Internationale Veröffentlichungsnummer: WO 2017/102878

(56) Entgegenhaltungen:
- WO-A2-2009/127196
- WO-A2-2014/086874
- DE-A1- 19 922 618
- DE-A1-102008 006 135

## Beschreibung

Die Erfindung betrifft eine Verschlussvorrichtung nach dem Oberbegriff des Anspruchs 1.

Eine gattungsgemäße Verschlussvorrichtung weist dabei wenigstens ein erstes und ein zweites Verschlussteil auf, die miteinander verbindbar sind, um die Verschlussvorrichtung zu schließen, und die voneinander lösbar sind, um die Verschlussvorrichtung zu öffnen. Hierbei ist das zweite Verschlussteil zum Schließen der Verschlussvorrichtung entlang einer Verbindungsachse in einer Schließrichtung an das erste Verschlussteil ansetzbar. Das erste und das zweite Verschlussteil weisen ferner jeweils ein Verbindungselement auf, wobei das erste und zweite Verschlussteil über die Verbindungselemente bei geschlossener Verschlussvorrichtung aneinander gehalten sind.

Eine gattungsgemäße Verschlussvorrichtung weist ferner wenigstens zwei Magnetelemente auf, die beim Ansetzen der Verschlussteile aneinander magnetisch anziehend zwischen dem ersten Verschlussteil und dem zweiten Verschlussteil wirken. Die wenigstens zwei Magnetelemente unterstützen die Annäherung der Verschlussteile aneinander und sorgen somit für ein leichtes und vorzugsweise selbsttätiges Verschließen der Verschlussvorrichtung, nachdem die beiden Verschlussteile aneinander angesetzt wurden.

Aus der WO 2015/004278 A1 ist beispielsweise eine Verschlussvorrichtung mit zwei Verschlussteilen bekannt, die unter Einwirkung wenigstens zweier Magnetelemente der Verschlussvorrichtung selbsttätig in eine Relativlage zueinander überführt werden, in der die Verschlussvorrichtung bestimmungsgemäß geschlossen ist. Hierbei werden die beiden Verschlussteile nach Art einer Schraubverbindung miteinander verbunden, sodass beispielsweise ein zweites Verschlussteil entlang einer Drehrichtung in das erste Verschlussteil eingedreht wird. Ist das zweite Verschlussteil maximal eingedreht, sind die beiden Verschlussteile über Sperrflächen in axialer Richtung aneinander gehalten. Dabei sieht eine in der WO 2015/004278 A1 vorgeschlagene Verschlussvorrichtung vor, dass die beiden Verschlussteile in einem geschlossenen Zustand axial relativ zueinander um ein definiertes Spiel verlagerbar sind, um die beiden Verschlussteile leicht, d.h., nahezu ohne zusätzlichen Kraftaufwand miteinander verschrauben zu können, indem die beiden Verschlussteile in einer ersten Belastungsrichtung aufeinander zu gedrückt werden. Über die Sperrflächen ist dann aber in dem geschlossenen Zustand der Verschlussvorrichtung sichergestellt, dass ein Aufdrehen der Verschlussvorrichtung blockiert ist, wenn an den Verschlussteilen eine Kraft in eine entgegengesetzte Belastungsrichtung angreift. Werden die Verschlussteile somit beispielsweise im geschlossenen Zustand der Verschlussvorrichtung lediglich auseinandergezogen, kann sich die Verschraubung nicht lösen.

Die aus der WO 2015/004278 A1 bekannte und nach Art eines Schraubverschlusses ausgestaltete Verschlussvorrichtung eignet sich für eine Vielzahl von Anwendungsfällen. In bestimmten Anwendungsfällen wird jedoch eine Verschlussvorrichtung bevorzugt, bei der eine möglichst spielfreie mechanische Verbindung gefordert ist.

Die DE19922618A offenbart eine Art Bajonettverschluß, wobei nach Einführen des ersten Kupplungsteiles in das zweiten Kupplungsteil infolge einer durch Magnete herbeigeführten Relativdrehung der beiden Kupplungsteile unabhängig von deren Anfangsdrehlage der Bajonettverschluss geschlossen wird.

Der Erfindung liegt daher die Aufgabe zugrunde, eine Verschlussvorrichtung mit zwei Verschlussteilen, deren Annäherung einander durch wenigstens zwei Magnetelemente unterstützt ist, zu verbessern und insbesondere eine Alternative zu einer nach Art eines Schraubverschlusses ausgestalteten Verschlussvorrichtung bereitzustellen.

Diese Aufgabe wird mit der Verschlussvorrichtung des Anspruchs 1 oder des Anspruchs 2 gelöst.

Bei einer erfindungsgemäßen Verschlussvorrichtung weist ein erstes Verbindungselement des ersten Verschlussteils mindestens einen Führungsabschnitt auf und ein zweites Verbindungselement des zweiten Verschlussteils weist mindestens einen Verschlussabschnitt auf, die ein Halten und eine Arretierung der ersten und zweiten Verschlussteile aneinander nach Art eines Bajonettverschlusses ermöglichen. Hierbei ist vorgesehen, dass
- der Führungsabschnitt eine zur Verbindungsachse der Verschlussteile geneigte Führungsfläche aufweist, mit der der Verschlussabschnitt beim Ansetzen des zweiten Verschlussteils an das erste Verschlussteil in Kontakt tritt und die dem zweiten Verbindungselement eine Drehung um die Verbindungsachse relativ zu dem ersten Verbindungselement entlang einer ersten Drehrichtung aufzwingt, wenn die ersten und zweiten Verschlussteile - unter Einwirkung der wenigstens zwei Magnetelemente der Verschlussvorrichtung - einander weiter angenähert werden,
- der Führungsabschnitt in der ersten Drehrichtung ein Ende aufweist, sodass der Verschlussabschnitt in Schließrichtung an dem Führungsabschnitt vorbeiführbar ist, bevor das zweite Verbindungselement eine Zwischenstellung relativ zu dem ersten Verbindungselement erreicht,
- die Verschlussvorrichtung eine, zum Beispiel federkraftbasierte und/oder magnetkraftbasierte Verstelleinrichtung aufweist, über die das zweite Verbindungselement in der Zwischenstellung mit einer Kraft in eine zu der erste Drehrichtung entgegengesetzte, zweite Drehrichtung beaufschlagt ist, so dass das zweite Verbindungselement aus der Zwischenstellung selbsttätig relativ zu dem ersten Verbindungselement und um die Verbindungsachse entlang der zweiten Drehrichtung in eine Schließstellung gedreht wird, und
- in der Schließstellung (a) einerseits der Verschlussabschnitt den Führungsabschnitt zumindest teilweise hintergreift, um die ersten und zweiten Verbindungselemente und damit die ersten und zweiten Verschlussteile aneinander zu halten, und (b) andererseits für ein Lösen der ersten und zweiten Verschlussteile das zweite Verbindungselement in die erste Drehrichtung - entgegen der von der Verstelleinrichtung aufgebrachten Kraft - um die Verbindungsachse relativ zu dem ersten Verbindungselement drehbar ist. , um die Verschlussvorrichtung zu offnen.

Über den wenigstens einen Führungsabschnitt des ersten Verschlussteils wird somit zunächst eine Drehung des zweiten Verbindungselements des zweiten Verschlussteils in eine erste Drehrichtung erzwungen, bevor über die Verstelleinrichtung selbsttätig ein Drehen in die entgegengesetzte, zweite Drehrichtung erfolgt, sobald das zweite Verbindungselement durch den Führungsabschnitt an einer solchen Drehung nicht mehr gehindert ist. Durch den konstruktiv erzwungenen Drehrichtungswechsel beim Schließen der Verschlussvorrichtung ist ein definierter, sicherer Eingriff der beiden Verbindungselemente ineinander bei geschlossener Verschlussvorrichtung sichergestellt. Darüber hinaus muss das zweite Verbindungselement in einer ersten Phase des Schließvorgangs lediglich um wenige Grad in die erste Drehrichtung verdreht werden. Ein Ein- oder Aufschrauben ist nicht nötig. Über die Verstelleinrichtung wird zudem ein selbsttätiges Überführen des zweiten Verbindungselements in die Schließstellung zum zumindest teilweisen Hintergreifen des Führungsabschnitts erreicht, so dass die Verschlussvorrichtung schnell und ohne Kraftaufwand geschlossen werden kann.

Unter Zugrundelegung desselben Erfindungsgedankens wird weiterhin eine Verschlussvorrichtung vorgeschlagen, bei der
- das erste Verbindungselement mindestens einen Führungsabschnitt und einen Halteabschnitt aufweist und das zweite Verbindungselement mindestens einen ersten und einen zweiten Verschlussabschnitt, also mindestens zwei Verschlussabschnitte, aufweist, wobei
- der Führungsabschnitt eine zur Verbindungsachse geneigte Führungsfläche aufweist, mit der der erste Verschlussabschnitt beim Ansetzen des zweiten Verschlussteils an das erste Verschlussteil in Kontakt tritt und die dem zweiten Verbindungselement eine Drehung um die Verbindungsachse relativ zu dem ersten Verbindungselement entlang einer ersten Drehrichtung aufzwingt, wenn die ersten und zweiten Verschlussteile einander weiter angenähert werden,
- der Führungsabschnitt und der Halteabschnitt in der ersten Drehrichtung jeweils ein Ende aufweisen, so dass der erste Verschlussabschnitt in Schließrichtung an dem Führungsabschnitt und der andere, zweite Verschlussabschnitt an dem Halteabschnitt vorbeiführbar sind, bevor das zweite Verbindungselement eine Zwischenstellung relativ zu dem ersten Verbindungselement erreicht,
- die Verschlussvorrichtung eine Verstelleinrichtung aufweist, über die das zweite Verbindungselement in der Zwischenstellung mit einer Kraft in eine zu der ersten Drehrichtung entgegengesetzte, zweite Drehrichtung beaufschlagt ist, so dass das zweite Verbindungselement aus der Zwischenstellung selbsttätig relativ zu dem ersten Verbindungselement und um die Verbindungsachse entlang der zweiten Drehrichtung in eine Schließstellung gedreht wird, und
- in der Schließstellung der zweite Verschlussabschnitt den Halteabschnitt zumindest teilweise hintergreift, um die ersten und zweiten Verbindungselemente und damit die ersten und zweiten Verschlussteile aneinander zu halten, und für ein Lösen der ersten und zweiten Verschlussteile voneinander das zweite Verbindungselement in die erste Drehrichtung um die Verbindungsachse relativ zu dem ersten Verbindungselement drehbar ist, um die Verschlussvorrichtung zu öffnen.

Bei dieser erfindungsgemäßen Verschlussvorrichtung fallen somit derjenige Abschnitt, der die Führungsfläche bereitstellt, und derjenige Abschnitt, der von einem Verschlussabschnitt hintergriffen wird, um die beiden Verschlussteile aneinander zu halten, auseinander. Sind beispielsweise in einer Ausführungsvarianten nach dem ersten Erfindungsaspekt an einem ersten Verbindungselement entlang eines Umfang lediglich zueinander äquidistant beabstandete Führungsabschnitte für die Generierung der Drehung vorgesehen, können sich bei einer Verschlussvorrichtung nach dem zweiten Erfindungsaspekt entlang eines Umfang des ersten Verbindungselements Führungsabschnitte und Halteabschnitte abwechseln, wobei die Halteabschnitte keine Führungsfläche für die Generierung einer Drehbewegung durch das zweite Verbindungselement aufweisen, aber in der Schließstellung jeweils von einem Verschlussabschnitt des zweiten Verbindungselements hintergriffen sind. Zum Beispiel sind in einer Ausführungsvariante nach dem ersten Erfindungsaspekt an einem ersten Verbindungselement vier identisch ausgebildete Führungsabschnitte entlang einer im Querschnitt kreisförmigen Mantelfläche alle 90° vorgesehen, d.h., bei 90° (3 Uhr), 180° (6 Uhr), 270° (9 Uhr) und 360° (12 Uhr). In einer Ausführungsvariante nach dem zweiten Erfindungsaspekt sind an einem ersten Verbindungselement zwei Führungsabschnitte und zwei Halteabschnitte entlang einer im Querschnitt kreisförmigen Mantelfläche alle 90° einander abwechselnd vorgesehen, d.h., bei 90° (3 Uhr) und 270° (9 Uhr) jeweils ein Führungsabschnitt und bei 180° (6 Uhr) und 360° (12 Uhr) jeweils ein Halteabschnitt.

Die Verstelleinrichtung ist beispielsweise federkraftbasiert und/oder magnetkraftbasierte, um das zweite Verbindungselement in der Zwischenstellung mit einer Federkraft und/oder einer Magnetkraft in die zweite Drehrichtung zu beaufschlagen.

In einer Ausführungsvariante umfasst eine magnetkraftbasierte Verstelleinrichtung die wenigstens zwei Magnetelemente, die auch beim Ansetzen der Verschlussteile aneinander magnetisch anziehend zwischen dem ersten Verschlussteil und dem zweiten Verschlussteil wirken. Um über diese Magnetelemente dann auch eine Magnetkraft zum Drehen des zweiten Verbindungselements in seine Schließstellung zu erzeugen, ist beispielsweise wenigstens ein Magnetelement vorgesehen, das zwei verschieden, d.h., ungleichnamige Magnetpole (Nordpol, Südpol) in einer im Wesentlichen senkrecht zur Schließrichtung verlaufenden Ebene aufweist. Hierfür ist dieses Magnetelement in einem Ausführungsbeispiel quer zur Schließrichtung magnetisiert, sodass eine Trennlinie zwischen einem Nordpol und einem Südpol dieses Magnetelements quer zur Schließrichtung verläuft. Bei einem im Querschnitt kreisförmigen Magnetelement, zum Beispiel also bei einem scheibenförmigen Magnetelement, ist dieses Magnetelement dann diametral magnetisiert, also entlang seines Durchmessers. Die Trennlinie zwischen Nord- und Südpol dieses Magnetelements verläuft hier im bestimmungsgemäß in der Verschlussvorrichtung eingebauten Zustand quer zur Schließrichtung.

Anstelle eines einzelnen Magnetelements mit zwei verschiedenen Magnetpolen, die in einer im Wesentlichen senkrecht zur Schließrichtung verlaufenden Ebene angeordnet sind, können an einem Verschlussteil auch wenigstens zwei Magnetelemente derart vorgesehen sein, dass hierüber zwei verschiedene Magnetpole in einer im Wesentlichen senkrecht zur Schließrichtung verlaufenden Ebene vorliegen.

Über die Anordnung zweier verschiedener Magnetpole in einer senkrecht zur Schließrichtung verlaufenden Ebene kann beim Ansetzen der Verschlussteile entlang der Schließrichtung in Abhängigkeit davon, in welcher Drehlage sich die Verschlussteile relativ zueinander um die Verbindungsachse befinden, nicht nur gesteuert werden, ob sich die in unterschiedlichen Verschlussteilen vorgesehenen Magnetelemente anziehen oder abstoßen. Vielmehr ist hierüber auch die Erzeugung eines Drehmoments möglich.

Vor diesem Hintergrund sieht beispielsweise eine Ausführungsvariante vor, dass für ein Öffnen der Verschlussvorrichtung eine Drehung des zweiten Verbindungselements relativ zu dem ersten Verbindungselement aus der Schließstellung in die erste Drehrichtung über die Zwischenstellung hinaus zugelassen ist und jedes Verschlussteil wenigstens ein Magnetelement mit zwei verschiedenen Magnetpolen oder zwei Magnetelemente derart aufweist, dass dem Verschlussteil zwei verschiedene Magnetpole in einer im Wesentlichen senkrecht zur Schließrichtung verlaufenden Ebene vorliegen. Die Magnetelemente der ersten und zweiten Verschlussteile sind hierbei dann derart an den Verschlussteilen angeordnet, dass die Magnetelemente
(a) beim Schließen der Verschlussvorrichtung magnetisch anziehend wirken und in der Zwischenstellung des zweiten Verbindungselements derart zueinander angeordnet sind, dass die beiden Verbindungselemente aufgrund der wirkenden Magnetkräfte bestrebt sind, eine der Schließstellung entsprechende Relativlage zueinander einzunehmen, und
(b) beim Öffnen der Verschlussvorrichtung magnetisch abstoßend wirken, wenn das zweite Verbindungselement aus der Schließstellung relativ zu dem ersten Verbindungselement in die erste Drehrichtung oder in die zweite Drehrichtung über die Zwischenstellung hinaus gedreht wird.

In dieser Variante kann an jedem Verschlussteile folglich z.B. jeweils ein quer zur Schließrichtung magnetisiertes Magnetelement vorgesehen sein, wobei dann die zwei quer zur Schließrichtung magnetisierten Magnetelemente der zwei Verschlussteile Teil der Verstelleinrichtung sind und somit nicht nur eine Annäherung der beiden Verschlussteile beim Schließen der Verschlussvorrichtung unterstützen, sondern auch das Drehen des zweiten Verbindungselements in die zweite Drehrichtung, um die Schließstellung einzunehmen. Beim Öffnen der Verschlussvorrichtung wiederum werden die wenigstens zwei Magnetelemente der zwei Verschlussteile durch das Drehen des zweiten Verbindungselements über die Zwischenstellung hinaus so zueinander ausgerichtet, dass sie einander abstoßend wirken und damit das Lösen der beiden Verschlussteile voneinander unterstützen.

In einer Ausführungsvariante weist der Führungsabschnitt des ersten Verbindungselements zwei verschiedene Führungsflächen derart auf, dass
- beim Schließen der Verschlussvorrichtung eine Führung des zweiten Verbindungselements an der einen zur Verbindungsachse geneigten Führungsfläche über den hieran anliegenden Verschlussabschnitt für die Drehung des zweiten Verbindungselements in die erste Drehrichtung erfolgt und
- beim Öffnen der Verschlussvorrichtung eine Führung des zweiten Verbindungselements an der anderen Führungsfläche für die Drehung in die erste Drehrichtung erfolgt.

An einem einzelnen Führungsabschnitt des ersten Verbindungselements sind somit zwei Führungsflächen vorgesehen und beispielsweise hieran ausgebildet, die in Abhängigkeit davon, ob die Verschlussvorrichtung geöffnet oder geschlossen wird, eine körperliche Führung für das zweite Verbindungselement bereitstellen.

Beide Führungsflächen sind hierbei vorzugsweise geneigt zur Schließrichtung ausgeführt, um hierüber eine bestimmte Führungsbahn einerseits für die weitere Annäherung der beiden Verschlussteile aneinander beim Schließen der Verschlussvorrichtung und andererseits für das definierte Lösen der Verschlussteile voneinander beim Öffnen der Verschlussvorrichtung bereitzustellen. Die Neigungen der Führungsflächen bezüglich der Schließrichtung und der Verbindungsachse können hierbei zueinander unterschiedlich sein. Beispielsweise weist die (erste) Führungsfläche des Führungsabschnitts, mit der eine Führung in die erste Drehrichtung beim Ansetzen des zweiten Verschlussteils an das erste Verschlussteil bereitgestellt ist, eine stärkere Neigung auf als die andere (zweite) Führungsfläche des Führungsabschnitts, über die eine Führung beim Öffnen der Verschlussvorrichtung bereitgestellt ist. Die Neigungen der Führungsflächen können aber auch im Wesentlichen identisch sein.

In einem Ausführungsbeispiel verlaufen die unterschiedlichen Führungsflächen an einem Führungsabschnitt ferner unter einem Winkel von mehr als 90° zueinander.

Bei mehreren entlang einer Umfangslinie um die Verbindungsachse oder die Schließrichtung zueinander (äquidistant) beabstandeten Führungsabschnitten an dem ersten Verbindungselement sind die einzelnen Führungsabschnitte vorzugsweise zueinander identisch ausgebildet und weisen somit gegebenenfalls jeweils zwei unter einem Winkel zueinander verlaufende, unterschiedliche Führungsflächen für das zweite Verbindungselement auf.

Sind an dem zweiten Verbindungselement mehrere Verschlussabschnitte vorgesehen, die entlang einer Umfangslinie um die Schließrichtung oder die Verbindungsachse zueinander (äquidistant) beabstandet sind, kann in einer Ausführungsvariante vorgesehen sein, dass beim Schließen der Verschlussvorrichtung zunächst derjenige Verschlussabschnitt an der ersten Führungsfläche eines Führungsabschnitts für die Drehung in der ersten Drehrichtung geführt wird, der in der Schließstellung des zweiten Verbindungselements diesen Führungsabschnitt zumindest teilweise hintergreift. Während beim Öffnen der Verschlussvorrichtung ein anderer, benachbarter Verschlussabschnitt an der zweiten Führungsfläche dieses Führungsabschnitts anliegt. Derart kann dem zweiten Verbindungselement über unterschiedliche Verschlussabschnitte stets eine definierte Drehbewegung mit überlagerter Verstellbewegung in Schließ- oder Öffnungsrichtung vorgegeben werden, indem die unterschiedlichen Verschlussabschnitte zwar an demselben Führungsabschnitt und für eine Drehung des zweiten Verbindungselements in dieselbe (erste) Drehrichtung relativ zu dem ersten Verbindungselement, aber je nachdem, ob die Verschlussvorrichtung geschlossen oder geöffnet wird, an unterschiedlichen Führungsflächen dieses Führungsabschnitts anliegen.

Ein Verschlussabschnitt des zweiten Verbindungselements weist eine hieran ausgebildete Gleitfläche auf, über die der Verschlussabschnitt mit einer Führungsfläche des ersten Verbindungselements in Kontakt. tritt. Um hierbei ein selbsttätiges Drehen eines der Verbindungselemente relativ zu dem anderen Verbindungselement infolge der Magnetelemente zu unterstützen und auch ein leichtgängiges Aufdrehen der Verschlussvorrichtung für einen Nutzer zu ermöglichen, ist eine Gleitfläche in einer Variante um mehr als 55° zur Verbindungsachse geneigt ausgeführt, beispielsweise um mehr als 60° und insbesondere um etwa 70° .

In einer Ausführungsvariante umfasst das erste Verschlussteil mindestens einen ersten Verdrehsicherungsabschnitt und das zweite Verschlussteil umfasst mindestens einen zweiten Verdrehsicherungsabschnitt, wobei an dem zweiten Verschlussteil das zweite Verbindungselement relativ zu dem zweiten Verdrehsicherungsabschnitt drehbar ist und der mindestens eine erste Verdrehsicherungsabschnitt und der mindestens eine zweite Verdrehsicherungsabschnitt beim Schließen der Verschlussvorrichtung drehfest miteinander verbindbar sind. Durch die drehfeste Verbindung behalten die Verdrehsicherungsabschnitte sowie alle damit starr verbundenen Teile während der Verbindung der Verbindungselemente (insbesondere bezüglich einer Drehung um die Schließrichtung herum) eine gleichbleibende relative Stellung zueinander. Dies ist insbesondere von Vorteil, wenn über die Verschlussvorrichtung zwei Elemente aneinander befestigt werden sollen, wobei ein erstes Element mit dem ersten Verschlussteil und ein zweites Element mit dem zweiten Verschlussteil verbunden ist. Aufgrund der Verdrehsicherungsabschnitte und der Drehbarkeit des zweiten Verbindungselements relativ zu dem zweiten Verdrehsicherungsabschnitt an dem zweiten Verschlussteil können die beiden aneinander zu befestigenden Elemente in einer gewünschten oder vorbestimmten Ausrichtung relativ zueinander verbleiben, wenn die Verschlussvorrichtung geschlossen oder geöffnet wird. So kann auf diese Weise zum Beispiel das Befestigen eines elektronischen Geräts an einem Trägerelement (zum Beispiel an einem Fahrradlenker) in einer gewünschten oder vorbestimmten Ausrichtung relativ zu dem Trägerelement erleichtert werden. Das mit dem zweiten Verschlussteil verbundene elektronische Gerät kann beispielsweise während des Schließens der Verschlussvorrichtung gleichbleibend in einer Stellung (bezüglich Drehung um die Schließrichtung herum) verbleiben, die es beim Ansetzen der Verschlussteile aneinander einnimmt.

So kann zum Beispiel das erste Verschlussteil ein mit dem ersten Verbindungselement verbundenen erstes Befestigungselement zum Befestigen des ersten Verschlussteils an einem Trägerelement aufweisen, wobei das erste Befestigungselement mit dem mindestens einen ersten Verdrehsicherungsabschnitt drehfest bezüglich einer Drehung um die Schließrichtung herum verbunden ist. Beispielsweise kann dabei das erste Befestigungselement zum Befestigen des ersten Verschlussteils an einem rohrförmigen Trägerelement, insbesondere an einem Fahrradlenker ausgebildet sein. Es ist selbstverständlich auch denkbar, dass das erste Befestigungselement zum Befestigen des ersten Verschlussteils an einem anderen Trägerelement ausgebildet ist, wie zum Beispiel einem Motorradlenker. Grundsätzlich kann eine erfindungsgemäße Verschlussvorrichtung insbesondere für jede Art von Halterung für die Befestigung eines Gegenstands an einem Zwei- oder Dreirad ausgebildet und eingerichtet sein. Dies schließt neben der Verwendung als Teil einer Gerätehalterung an einem Lenker unter anderem die Verwendung für eine Taschenhalterung an einem Fahrrad, Motorrad oder Trike ein.

In einer möglichen Ausführungsvariante weist das zweite Verschlussteil ein zweites Befestigungselement zum Befestigen eines Gegenstands, zum Beispiel eines elektronischen Gerätes oder einer Tasche, an dem zweiten Verschlussteil auf. Das zweite Befestigungselement kann hierbei mit einem Verdrehsicherungselement des zweiten Verschlussteils drehfest verbunden, das den mindestens einen zweiten Verdrehsicherungsabschnitt aufweist. Grundsätzlich kann der zweite Verdrehsicherungsabschnitt des zweiten Verschlussteils an einem separaten Verdrehsicherungselement vorgesehen sein, relativ zu dem das zweite Verbindungselement drehbar gelagert ist. Das zweite, mehrteilig aufgebaute Verschlussteil weist somit neben dem Verbindungselement wenigstens noch eine weitere hiermit verbundene Komponente auf. Das Verdrehsicherungselement kann wiederum drehfest mit einem Befestigungselement des zweiten Verschlussteils verbunden sein, über das das zweite Verschlussteil mit einem Element, wie zum Beispiel einem an einem Trägerelement festzulegenden Gegenstand, verbunden ist.

Beispielsweise ist eine Verbindung des Verdrehsicherungselements mit einem Befestigungselement über eine Bajonettverbindung vorgesehen. In einer Variante bildet das Verdrehsicherungselement beispielsweise Verschlusshaken aus, die beim Ansetzen an das Befestigungselement in zugeordnete Ballettöffnungen des Befestigungselements eingreifen und durch Drehung des Verdrehsicherungselements relativ zu dem Befestigungselement mit diesen verrasten. Für diese während des Zusammenbaus des Verschlussteils vorgesehene Verrastung bildet das Befestigungselement zum Beispiel an seinen Bajonettöffnungen jeweils Rastlaschen aus, die nach einem Drehen des Befestigungselements relativ zu dem Verdrehsicherungselement jeweils einen durch die zugehörige Bajonettöffnung hindurchgreifenden Verschlusshaken hintergreifen und in der Bajonettöffnung formschlüssig sichern.

Gemäß einer Ausführungsform weist der mindestens eine erste Verdrehsicherungsabschnitt und/oder der mindestens eine zweite Verdrehsicherungsabschnitt eine hohlzylindrische Grundform auf, die sich axial entlang der Schließrichtung erstreckt. Durch eine hohlzylindrische Grundform beider Verdrehsicherungsabschnitte können diese eine große mechanische Stabilität aufweisen und zugleich vergleichsweise kostengünstig hergestellt werden. Die Verdrehsicherungsabschnitte können hierbei derart ausgebildet sein, dass beim Ansetzen der Verschlussteile aneinander der mindestens eine erste Verdrehsicherungsabschnitt den mindestens einen zweiten Verdrehsicherungsabschnitt oder der mindestens eine zweite Verdrehsicherungsabschnitt den mindestens einen ersten Verdrehsicherungsabschnitt mindestens abschnittsweise rings um die Schließrichtung herum umgibt. Durch eine solche Anordnung kann ein Formschluss der Verdrehsicherungsabschnitte miteinander und darüber der Verschlussteile insgesamt radial bezüglich der Schließrichtung hergestellt werden. Ein Führungsabschnitt ist beispielsweise an einer äußeren Mantelfläche des ersten Verbindungselements ausgebildet. Ein Verschlussabschnitt wiederum ist beispielsweise an einer inneren Mantelfläche des zweiten Verbindungselements ausgebildet, das im geschlossenen Zustand der Verschlussvorrichtung die äußere Mantelfläche des ersten Verbindungselements umlaufend umgibt.

Der mindestens eine erste Verdrehsicherungsabschnitt und der mindestens eine zweite Verdrehsicherungsabschnitt stehen in einer Ausführungsvariante beim Schließen der Verschlussvorrichtung zum Herstellen einer drehfesten Verbindung formschlüssig miteinander in Eingriff. Hierbei kann ein Verdrehsicherungsabschnitt eines Verschlussteils mindestens ein entlang der Schließrichtung längserstrecktes Formschlusselement aufweisen, während ein Verdrehsicherungsabschnitt des anderen Verschlussteils mindestens eine entlang der Schließrichtung längserstreckte Ausnehmung zur Aufnahme des mindestens einen längserstreckten Formschlusselements aufweist. Über das Eingreifen des Formschlusselementes in eine Ausnehmung oder das Eingreifen mehrerer Formschlusselemente in mehrere Ausnehmungen und eine damit verbundene Arretierung des jeweiligen Formschlusselements quer zur Schließrichtung (und einer damit zusammenfallenden Verbindungsachse der Verschlussvorrichtung) ist eine drehfeste Verbindung der Verdrehsicherungsabschnitte hergestellt.

Ein Formschlusselement und/oder eine Ausnehmung können zumindest abschnittsweise konisch zur Schließrichtung geformt sein. In einem Ausführungsbeispiel sind mehrere Formschlusselemente und die zugehörigen Ausnehmungen jeweils konisch und sich in Schließrichtung verjüngend ausgebildet. Die Formschlusselemente sind somit keilförmig ausgebildet und sind nach dem bestimmungsgemäßen Schließen der Verschlussvorrichtung vorzugsweise auch kraftschlüssig in der jeweiligen Ausnehmung gehalten. Derart ist nach dem Schließen der Verschlussvorrichtung eine zusätzliche kraft- und formschlüssige Bauteilverbindung für die miteinander verbundenen ersten und zweiten Verschlussteile über die Formschlusselemente und die sie aufnehmenden Ausnehmungen bereitgestellt.

Mehrere Formschlusselemente und/oder mehrere Ausnehmungen sind in einem Ausführungsbeispiel an dem jeweils zugehörigen Verdrehsicherungsabschnitt entlang einer Umfangslinie um die Schließrichtung zueinander beabstandet. Für eine gleichmäßige Verteilung auftretender Belastungen und eine formschlüssige, drehfeste Verbindung der beiden Verdrehsicherungsabschnitte in mehreren Relativlagen um die Schließrichtung sind hierbei die Formschlusselemente und Ausnehmungen beispielsweise äquidistant zueinander beabstandet; zum Beispiel sind bei vier Formschlusselementen diese um jeweils 90° zueinander versetzt.

In einer Ausführungsvariante wird ein den mindestens einen zweiten Verdrehsicherungsabschnitt aufweisendes Verdrehsicherungselement des zweiten Verschlussteils zusätzlich als Abstützung für ein Federelement einer federkraftbasierten Verstelleinrichtung genutzt. Das an dem zweiten Verschlussteil vorgesehene Federelement stützt sich hierbei dann einerseits an dem Verdrehsicherungselement und andererseits an dem zweiten Verbindungselement ab, um das zweite Verbindungselement in der Zwischenstellung mit einer Federkraft in die zweite Drehrichtung zu beaufschlagen. Über das wenigstens eine Federelement wird somit auf das zweite Verbindungselement eine Rückstellkraft in die zweite Drehrichtung um die Schließrichtung und um die Verbindungsachse ausgeübt, nachdem beim Schließen der Verschlussvorrichtung das zweite Verbindungselement aufgrund des wenigstens einen Führungsabschnitts zunächst in die erste Drehrichtung relativ zu dem ersten Verbindungselement und dem hiermit über die Verdrehsicherungsabschnitte drehfest verbundenen zweiten Verdrehsicherungselement gedreht und damit das wenigstens eine Federelement (stärker) gespannt wurde. Sobald nach Abschluss einer ersten Phase des Schließvorgangs das zweite Verbindungselement seine Zwischenstellung erreicht, und über den Führungsabschnitt oder die Führungsabschnitte des ersten Verbindungselements eine Drehung des zweiten Verbindungselements relativ dem ersten Verbindungselement nicht mehr blockiert ist, wird das zweite Verbindungselement unter Wirkung des Federelements in seine Schließrichtung gedreht, in der der Führungsabschnitt (jeweils) von dem (zugeordneten) Verschlussabschnitt zumindest teilweise - bezogen auf die Verbindungsachse - hintergriffen wird.

Mit anderen Worten ist folglich das zweite Verbindungselement über das wenigstens eine Federelement an dem zweiten Verschlussteil relativ zu dem Verdrehsicherungselement vorgespannt. Beim Ansetzen an das erste Verschlussteil werden die Verschlussteile unter der Wirkung der wenigstens zwei Magnetelemente aneinander gezogen. Durch die geneigt verlaufende Führungsfläche des Führungsabschnitts wird dann infolge der anziehenden Wirkung der Magnetelemente der wenigstens eine Verschlussabschnitt des zweiten Verschlussteils an der geneigten Führungsfläche des Führungsabschnitts des ersten Verschlussteils entlang geführt und hierdurch das zweite Verbindungselement relativ zu dem bereits drehfest mit dem ersten Verbindungselement des ersten Verschlussteils verbundenen zweiten Verdrehsicherungsabschnitt des zweiten Verschlussteils in die erste Drehrichtung gedreht. Diese Drehung in die erste Drehrichtung erfolgt gegen die Rückstellkraft des Federelements. In der Zwischenstellung ist das Federelement dann (stärker) gespannt und drückt oder zieht damit das zweite Verbindungselement in die zweite Drehrichtung und damit in die Schließstellung.

In einer Ausführungsvariante, die auf einer Verschlussvorrichtung mit Verdrehsicherungsabschnitten aufgebaut, weist das zweite Verschlussteil (zusätzlich) ein Betätigungselement auf, dass sowohl relativ zu dem zweiten Verdrehsicherungsabschnitt als auch relativ zu dem zweiten Verbindungselement an dem zweiten Verschlussteil drehbar ist. Bei bestimmungsgemäß geschlossener Verschlussvorrichtung ist dieses Betätigungselement in die erste Drehrichtung um die Verbindungsachse drehbar, um auf das zweite Verbindungselement einzuwirken und das zweite Verbindungselement relativ zu dem ersten Verbindungselement zu drehen. Das Betätigungselement ist somit von einem Nutzer betätigbar und überträgt eine Verstellkraft zum Entriegeln der Verschlussvorrichtung an das zweite Verbindungselement, das in einer solchen Ausführungsvariante bei geschlossener Verschlussvorrichtung für einen Nutzer üblicherweise nicht direkt zugänglich ist. Das Betätigungselement kann hierbei beispielsweise mit dem zweiten Verbindungselement derart zusammenwirken, dass das Betätigungselement das zweite Verbindungselement nach Art eines Mitnehmers für das Öffnen der Verschlussvorrichtung mitnimmt.

In einer hierauf basierenden Weiterbildung ist das Betätigungselement bei geschlossener Verschlussvorrichtung in einer Relativlage zu dem zweite Verbindungselement gehalten, aus der das Betätigungselement einen vorgegebenen Leerdrehweg in die erste Drehrichtung überbrücken muss, bevor das Betätigungselement auf das zweite Verbindungselement einwirkt und die Verschlussvorrichtung bestimmungsgemäß geöffnet werden kann. Indem das Betätigungselement zunächst einen vorgegebenen Leerdrehweg zurücklegen muss, bevor es auf das zweite Verbindungselement einwirkt und damit dessen einen Verschlussabschnitt oder dessen mehrere Verschlussabschnitte verstellt, sodass hierüber ein Führungsabschnitt oder mehrere Führungsabschnitte des ersten Verbindungselements nicht mehr hintergriffen werden und entlang der Verbindungsachse ein Trennen der Verschlussteile voneinander ermöglicht wird, ist das Risiko für ein unbeabsichtigtes Öffnen der Verschlussvorrichtung reduziert. So muss ein Nutzer zunächst das Betätigungselement um den vorgegebenen Leerdrehweg in die erste Drehrichtung drehen, bis hierüber überhaupt auf das zweite Verbindungselement zum Lösen der Verbindung zwischen den beiden Verschlussteilen eingewirkt werden kann. Außerdem ist auf diese Weise eine Art "Umpolung" der wirkenden Magnetkräfte vor dem Öffnen der Verschlussvorrichtung möglich. So kann bei der Verwendung von mehreren Magnetelementen, die an jeweils einem Verschlussteil derart angeordnet sind, dass hierüber in einer quer zur Schließrichtung verlaufenden Ebene verschiedene (ungleichnamige) Magnetpole vorliegen, das Überbrücken des Leerdrehwergs einschließen, dass die Magnetelemente eines Verschlussteils mitgedreht werden und hierdurch in eine Relativlage zu den Magnetelementen des anderen Verschlussteils gedreht werden, in der die Magnetelemente beider Verschlussteile einander abstoßend wirken und damit die Öffnungsbewegung unterstützen.

Das Betätigungselement ist in einer Weiterbildung bei geschlossener Verschlussvorrichtung mittels wenigstens zweier Magnetelemente in der Relativlage zu dem Verbindungselement gehalten, aus der das Betätigungselement den vorgegebenen Leerdrehweg in die erste Drehrichtung überbrücken muss, bevor das Betätigungselement auf das zweite Verbindungselement einwirkt und dieses mitdreht. Zum Beispiel kann über die Wirkung der entsprechend angeordneten Magnetelemente der ersten und zweiten Verschlussteile sichergestellt sein, dass das Betätigungselement bei bestimmungsgemäß geschlossener Verschlussvorrichtung in der gewünschten Relativlage vorliegt und somit ein Leerdrehweg überbrückt werden muss.

Das Betätigungselement und das zweite Verbindungselement können derart ausgebildet und angeordnet sein, dass das zweite Verbindungselement bei einer Drehung in die Zwischenstellung entlang der ersten Drehrichtung auf das Betätigungselement einwirkt und das Betätigungselement in die erste Drehrichtung um die Verbindungsachse dreht. Das zweite Verbindungselement nimmt somit in einer ersten Phase des Schließvorgangs das Betätigungselement mit und dreht dieses in die erste Drehrichtung. Bei der anschließenden Drehung des zweiten Verbindungselements in die entgegengesetzte zweite Drehrichtung ist in einer Weiterbildung eine Kraftübertragung von dem zweiten Verbindungselement an das Betätigungselement nicht vorgesehen. Beispielsweise sind an dem Betätigungselement und dem zweiten Verbindungselement wechselseitig lediglich lokal radial vorspringende Abschnitte derart vorgesehen, dass hierüber beim Schließen der Verschlussvorrichtung ein Mitdrehen des Betätigungselements beim Drehen des zweiten Verbindungselements in die erste Drehrichtung sichergestellt ist, aber beim anschließenden Drehen des zweiten Verbindungselements in die entgegengesetzte zweite Drehrichtung dieses nicht auf das Betätigungselement einwirkt.

Eine Ausführungsvariante sieht, gegebenenfalls in Kombination mit den vorgenannten Merkmalen, vor, dass die Verschlussvorrichtung eine Betätigungselement aufweist, das selbsttätig in die zweite Drehrichtung gedreht wird. Beispielsweise ist vorgesehen, dass das Betätigungselement in der Zwischen- oder Schließstellung des zweiten Verbindungselements über wenigstens zwei Magnetelemente der Verschlussvorrichtung ein Drehmoment in die zweite Drehrichtung erfährt. So kann das Betätigungselement ebenso wie das erste Verbindungselement des ersten Verschlussteils ein quer zur Schließrichtung magnetisiertes Magnetelement derart aufweisen, dass hierüber nicht nur eine Anziehung der beiden Verschlussteile beim Schließen der Verschlussvorrichtung erzeugt wird, sondern auch das Betätigungselement bezüglich des ersten Verbindungselements selbsttätig in eine bestimmte Relativlage ausgerichtet wird, wenn sich das zweite Verbindungselement ein der Zwischenstellung oder der Schließstellung befindet. Das Betätigungselement wird auf diese Art und Weise in einer zweiten Phase des Schließvorgangs (Verstellen des zweiten Verbindungselements aus der Zwischenstellung in die Schließstellung) aufgrund der wirkenden Magnetkräfte in die zweite Drehrichtung gedreht. Das zweite Verbindungselement kann in einer solchen Variante unter Einwirkung wenigstens eines Federelements entlang der zweiten Drehrichtung um die Verbindungsachse in seine Schließstellung gezogen oder gedrückt werden, so dass in der Zwischenstellung das zweite Verbindungselement durch wenigstens ein Federelement in die zweite Drehrichtung mechanisch vorgespannt ist, das Betätigungselement aber durch wenigstens zwei Magnetelemente magnetisch vorgespannt ist.

In einer Ausführungsvariante weist die Verschlussvorrichtung eine Stellwegbegrenzung mit wenigstens einem Anschlag an dem zweiten Verbindungselement und wenigstens einem Gegenanschlag derart auf, dass eine Drehung des zweiten Verbindungselements beim Öffnen und/oder Schließen der Verschlussvorrichtung durch Kontakt von Anschlag und Gegenanschlag auf einen vorgegebenen maximalen Drehwinkel begrenzt ist. Das Vorsehen einer Stellwegbegrenzung wird hierbei insbesondere einerseits bei der Verwendung einzelner quer zur Schließrichtung magnetisierter Magnetelemente als vorteilhaft erachtet, wie auch andererseits bei der Verwendung von mehreren Magnetelementen, die an jeweils einem Verschlussteil derart angeordnet sind, dass hierüber in einer quer zur Schließrichtung verlaufenden Ebene verschiedene (ungleichnamige) Magnetpole vorliegen. Über die Stellwegbegrenzung kann sichergestellt werden, dass die Magnetelemente der ersten und zweite Verschlussteile in der jeweiligen Ausrichtung der Verschlussteile oder ihrer Verbindungselemente zueinander beim Schließen und Öffnen der Verschlussvorrichtung das gewünschte Maß an magnetischer Abstoßung und das gewünschte Maß eines Rückdrehmomentes bereitstellen. Dabei ist jedoch über die Anordnung und Ausrichtung der Magnetelemente ausgeschlossen, dass gleichzeitig eine magnetische Abstoßung und ein Rückstellmoment maximal sind.

In einem Ausführungsbeispiel weist das zweite Verschlussteil ein durch einen Anschlagring definiertes Befestigungselement auf, das an einem mit dem zweiten Verschlussteil versehenen Element festgelegt ist, zum Beispiel einer Verschlusslasche. Das zweite Verbindungselement ist dann relativ zu diesem Befestigungselement drehbar gelagert und über hieran ausgebildete Gegenanschläge sowohl in der ersten Drehrichtung als auch in der zweiten Drehrichtung in seiner Drehbarkeit relativ zu dem Befestigungselement begrenzt. Die Gegenanschläge des Befestigungselements schränken hierbei die Drehbarkeit des zweiten Verbindungselements beispielsweise auf einen Drehwinkelbereich unter 270°, vorzugsweise unter 180°, insbesondere unter 150° und z.B. auf einen Bereich von etwa 120° ein.

Die Einschränkung der Drehbarkeit ist dabei auch insoweit sinnvoll, da bei einem Ausführungsbeispiel mit magnetkraftbasierter Verstelleinrichtung die Verbindungselemente über die Verstelleinrichtung und deren entsprechend angeordnete Magnetelemente grundsätzlich in beide möglichen Drehrichtungen mit einem Rückstellmoment beaufschlagt werden können und damit durch die Verstelleinrichtung an sich eine Drehbarkeit in beide Drehrichtungen nicht eingeschränkt ist. So ist beispielsweise bei diametral magnetisierten Magnetelementen, die einerseits dem ersten und andererseits dem zweiten Verbindungselemente zugeordnet sind, in Abhängigkeit von der Relativlage der Magnetelemente und damit in Abhängigkeit von der Relativlage der Verbindungselemente ein Rückstellmoment in die eine oder die andere Drehrichtung erzeugbar. Dies gestattet im Vergleich mit einer federkraftbasierten Verstelleinrichtung eine deutlich größere Flexibilität bei der Ausgestaltung und Funktionsweise der Verschlussvorrichtung sowie auch einen deutlich geringeren Bauraumbedarf. Die Verschlussvorrichtung kann somit sehr kompakt und einer anwendungsabhängigen Wahl der Drehrichtungen für das Schließen und Öffnen ausgeführt werden.

In einem Ausführungsbeispiel sind in der Schließstellung der Führungsabschnitt des ersten Verbindungselements und der Verschlussabschnitt des zweiten Verbindungselements bezogen auf die Verbindungsachse der ersten und zweite Verschlussteile axial relativ zueinander um ein definiertes Spiel verlagerbar. Der Führungsabschnitt und der Verschlussabschnitt sind hierbei dann aber gleichzeitig - nach Überbrückung eines Spiels - gegen eine Drehung relativ zueinander um die Verbindungsachse und gegen die Entnahme entgegen der Schließrichtung gesperrt, wenn in der Schließstellung des zweiten Verbindungselements an dem ersten und/oder zweiten Verschlussteil eine entlang der Verbindungsachse wirkende Kraft angreift, die das jeweilige Verschlussteil in eine von dem anderen Verschlussteile weg weisende Richtung belastet. Zur Sperrung sind Sperrteile der ersten und zweiten Verbindungselemente vorgesehen, die nach Überbrückung eines Spiels miteinander zusammenwirken. Durch das vorgesehene Spiel ist somit der Verschlussabschnitt (oder sind mehrere Verschlussabschnitte) in - bezogen auf die Verbindungsachse - axialer Richtung zu dem (jeweiligen) zumindest teilweise hintergriffen Führungsabschnitt beabstandet. Um zu verhindern, dass dann beispielsweise durch Ziehen an dem zweiten Verschlussteil die Verschlussvorrichtung unerwünscht geöffnet wird, ohne dass das zweite Verbindungselement zuvor gedreht wurde, sind die miteinander zusammenwirkenden Sperrteile an den beiden Verbindungselementen vorgesehen. Wird beispielsweise das zweite Verschlussteil relativ zu dem ersten Verschlussteil in eine zur Schließrichtung entgegengesetzte Öffnungsrichtung gezogen, verhaken oder verrasten die Sperrteile der ersten und zweiten Verbindungselemente miteinander und verhindern eine Drehung der beiden Verbindungselemente relativ zueinander. Bei entsprechend anliegender Belastung kann somit die Verschlussvorrichtung nicht geöffnet werden. Dies ist nur in einem (weitgehend) unbelasteten Zustand der Verschlussvorrichtung möglich, wenn der Führungsabschnitt und der Verschlussabschnitt oder die Führungsabschnitte und die Verschlussabschnitte in axialer Richtung voneinander beabstandet sind und somit eine Drehung des zweiten Verbindungselements in die erste Drehrichtung relativ zu dem ersten Verbindungselement erfolgen kann.

In einer Weiterbildung ist ein erstes Sperrteil an dem Führungsabschnitt und ein zweites Sperrteil an dem Verschlussabschnitt vorgesehen. Beispielsweise ist ein erstes Sperrteil durch einen hakenförmig ausgestalteten Bereich an dem Führungsabschnitt und ein zweiter Sperrteil durch einen komplementär ausgestalteten hakenförmigen Bereich an dem Verschlussabschnitt ausgebildet.

Wie bereits zuvor erläutert, können an dem ersten Verbindungselement mehrere zueinander beabstandete Führungsabschnitte vorgesehen, insbesondere hieran ausgebildet sein, ebenso wie an dem zweiten Verbindungselement mehrere zueinander beabstandete Verschlussabschnitte vorgesehen, insbesondere hieran ausgebildet sein können. Über mehrere Führungsabschnitte und Verschlussabschnitte kann einerseits die Verbindung der beiden Verschlussteile in unterschiedlichen Relativlagen zueinander bezüglich der Verbindungsachse erleichtert werden. Andererseits ist hierüber eine erhöhte mechanische Stabilität der geschlossenen Verschlussvorrichtung erreicht.

Mit einer erfindungsgemäß ausgestatteten Verschlussvorrichtung kann eine Halterung für die Befestigung eines Gegenstands an einem Zwei- oder Dreirad, insbesondere in Form einer Lenkerhalterung bereitgestellt werden.

Weiterhin ist eine Halterung für ein elektronisches Gerät mit einer erfindungsgemäßen Verschlussvorrichtung vorgeschlagen. Das elektronische Gerät kann beispielsweise ein Mobiltelefon, insbesondere ein Smartphone, ein Navigationsgerät oder ein anderes Kommunikationsgerät oder mobiles Endgerät sein. Über die Halterung ist dieses elektronische Gerät dann an einem Trägerelement mittels der Verschlussvorrichtung befestigbar.

Ferner ist eine Halterung für die Befestigung eines Gegenstands an einem Zwei- oder Dreirad, insbesondere in Form einer Lenkerhalterung mit einer erfindungsgemäßen Verschlussvorrichtung vorgeschlagen. Bei dem zu befestigenden Gegenstand kann es sich beispielsweise um ein elektronisches Gerät oder eine Tasche handeln.

Darüber hinaus wird eine erfindungsgemäß ausgestalteten Verschlussvorrichtung aufgrund seiner leichten und schnellen Schließbarkeit, der zuverlässigen Verriegelung der Verschlussteile aneinander und der Möglichkeit, die Verschlussvorrichtung auch leicht und schnell zu öffnen, insbesondere für einen Kleidungs-, Schuh- oder Prothesenverschluss als vorteilhaft erachtet. So kann beispielsweise das erste Verschlussteil einem ersten Teil eines Kleidungsstücks, eines Schuhs oder einer Prothese zugeordnet und hieran fixiert sein. Das zweite Verschlussteil ist dann an einem weiteren Teil des Kleidungsstücks, des Schuhs oder der Prothese festgelegt, zum Beispiel an einer Lasche.

Selbstverständlich können hierbei auch mehrere erfindungsgemäß ausgestaltete Verschlussvorrichtungen an einem Kleidungsstück, einem Schuh oder einer Prothese vorgesehen sein und dementsprechend hieran mehrere Kleidungs-, Schuh- oder Prothesenverschlüsse mit jeweils einer erfindungsgemäß ausgestatteten Verschlussvorrichtung vorgesehen sein.

Vor diesem Hintergrund ist auch die Verwendung einer erfindungsgemäß ausgestatteten Verschlussvorrichtung als Teil eines Kleidungs-, Schuh- oder Prothesenverschlusses vorgeschlagen.

Weitere Vorteile und Merkmale der Erfindung werden bei der nachfolgenden Beschreibung von Ausführungsbeispielen anhand der Figuren deutlich werden.

Es zeigen:
- Figuren 1A bis 1C: eine erste Ausführungsvariante einer erfindungsgemäßen Verschlussvorrichtung in verschiedenen Ansichten in geschlossenem Zustand;
- Figuren 2A bis 2C: die Verschlussvorrichtung in verschiedenen Ansichten jeweils in einer Explosionsdarstellung;
- Figur 3: eine Draufsicht auf die Verschlussvorrichtung mit Darstellung einer Schnittlinie H-H;
- Figuren 4A bis 4G: die Verschlussvorrichtung in unterschiedlichen Phasen während des Schließens und Öffnens jeweils in geschnittener Darstellung entsprechend der Schnittlinie H-H der Figur 3;
- Figur 4H: eine Abwandlung der Verschlussvorrichtung nach der ersten Ausführungsvariante, bei der ein erstes Verschlusselement Führungs- und Halteabschnitte aufweist;
- Figuren 5A bis 5C: eine zweite Ausführungsvariante einer erfindungsgemäßen Verschlussvorrichtung in verschiedenen Ansichten in geschlossenem Zustand;
- Figuren 6A bis 6C: die Verschlussvorrichtung der Figuren 5A bis 5C in verschiedenen Ansichten jeweils in einer Explosionsdarstellung;
- Figur 7: eine Draufsicht auf die Verschlussvorrichtung der Figuren 5A bis 6C mit Darstellung einer Schnittlinie H-H;
- Figuren 8A bis 14D: in jeweils übereinstimmenden Ansichten die Verschlussvorrichtung der Figuren 5A bis 6C in unterschiedlichen Phasen während des Schließen und Öffnens, wobei zur Veranschaulichung der unterschiedlichen Phasen jeweils vier Ansichten gewählt sind;
- Figuren 15A bis 15C: ein drittes Ausführungsbeispiel einer erfindungsgemäßen Verschlussvorrichtung in verschiedenen Ansichten in geschlossenem Zustand;
- Figuren 16A bis 16C: die Verschlussvorrichtung der Figuren 15A bis 15C in verschiedenen Ansichten jeweils in einer Explosionsdarstellung;
- Figur 17: eine Draufsicht auf die Verschlussvorrichtung der Figuren 15A bis 16C mit Darstellung einer Schnittlinie H-H;
- Figuren 18A bis 25D: in jeweils übereinstimmenden Ansichten die Verschlussvorrichtung der Figuren 15A bis 16C in unterschiedlichen Phasen während des Schließen und Öffnens, wobei zur Veranschaulichung der unterschiedlichen Phasen jeweils vier Ansichten gewählt sind;
- Figur 26A: eine Ansicht der Unterseite eines zweiten Verschlussteils der Verschlussvorrichtung der Figuren 15A bis 16C in einem geschlossenen Zustand der Verschlussvorrichtung;
- Figur 26B: eine Ansicht von unten auf das zweite Verschlussteil, bei dem zum Öffnen der Verschlussvorrichtung ein außen liegendes Betätigungselement in eine erste Drehrichtung (hier im Uhrzeigersinn) gedreht wurde;
- Figuren 27A bis 27C: eine vierte Ausführungsvariante einer erfindungsgemäßen Verschlussvorrichtung in unterschiedlichen Ansichten in geschlossenem Zustand;
- Figur 27D: eine Draufsicht auf die Verschlussvorrichtung der Figuren 27A bis 27C mit Darstellung einer Schnittlinie A, A;
- Figur 27E: eine Schnittdarstellung der Verschlussvorrichtung der Figur 27D entlang der Schnittlinie A-A;
- Figuren 28A bis 28C: die Verschlussvorrichtung der Figuren 27A bis 27E in verschiedenen Ansichten jeweils in einer Explosionsdarstellung;
- Figuren 29A bis 36D: in jeweils übereinstimmenden Ansichten die Verschlussvorrichtung der Figuren 27A bis 28C in unterschiedlichen Phasen während des Schließen und Öffnens, wobei zur Veranschaulichung der unterschiedlichen Phasen jeweils fünf Ansichten gewählt sind.

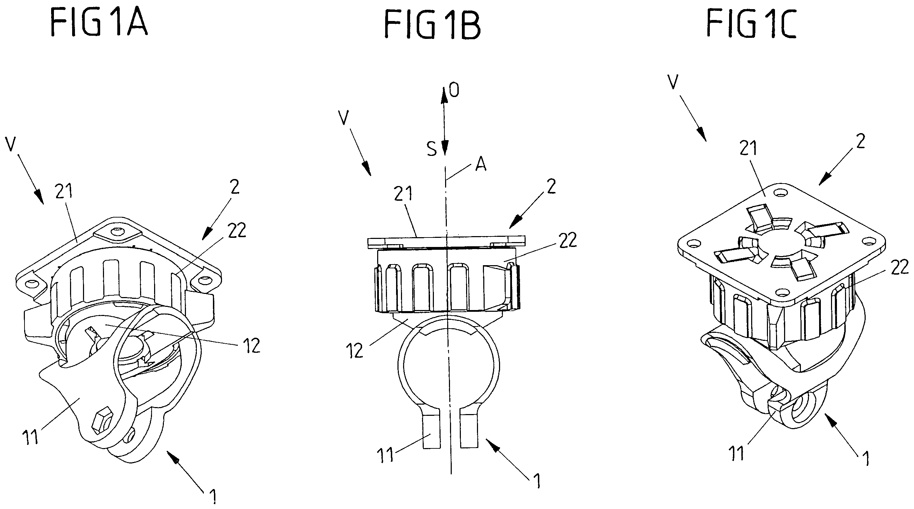

Die Figuren 1A bis 4G zeigen in verschiedenen Ansichten eine Verschlussvorrichtung V, die ein erstes Verschlussteil 1 und ein zweites Verschlussteil 2 umfasst. Die Verschlussteile 1 und 2 sind miteinander verbindbar und aneinander arretierbar, um die Verschlussvorrichtung V zu schließen, und sind voneinander lösbar, um die Verschlussvorrichtung V zu öffnen. Teil bei der Verschlussteile 1 und 2 ist jeweils ein Befestigungselement 11 oder 21 über die das jeweilige Verschlussteil 1 oder 2 an einem von zwei Elementen festlegbar ist, die über die Verschlussvorrichtung V miteinander lösbar gekoppelt werden sollen. Vorliegend weist das erste Verschlussteil 1 ein Befestigungselement 11 zum Befestigen des ersten Verschlussteils 1 an einem Trägerelement zum Beispiel einem Fahrrad- oder Motorradlenker auf. Hierfür ist das Befestigungselement 11 vorliegend als Schelle ausgeführt. Das zweite Befestigungselement 21 des zweiten Verschlussteils 2 wiederum ist vorliegend zum Befestigen eines elektronischen Geräts, beispielsweise eines Smartphones, vorgesehen und hierfür mit einer ebenen - vorliegend rechteckigen - Oberseite ausgebildet.

Das erste Verbindungselement 12 ist hierbei über zwei radial vorstehende Fixierungslaschen 122a und 122b an dem ersten Befestigungselement 11 fixiert. Die Fixierungslaschen 122a und 122b erstrecken sich in diametral zueinander und hintergreifen Randabschnitte des ersten Befestigungselements 11, die eine Lageröffnung 110 des ersten Befestigungselements 11 beranden, um das erste Verbindungselement 12 formschlüssig an dem ersten Befestigungselement 11 festzulegen.

Die beiden Verschlussteile 1, 2 sind in dem in den Figuren 1A, 1B und 1C dargestelltem verschlossenen Zustand der Verschlussvorrichtung V nach Art einer Bajonettverbindung aneinander gehalten und voneinander entlang einer Verbindungsachse A voneinander lösbar, nachdem eine Verriegelung zwischen zwei Verbindungselementen 12 und 22 der ersten und zweiten Verschlussteile 1, 2 gelöst wurde.

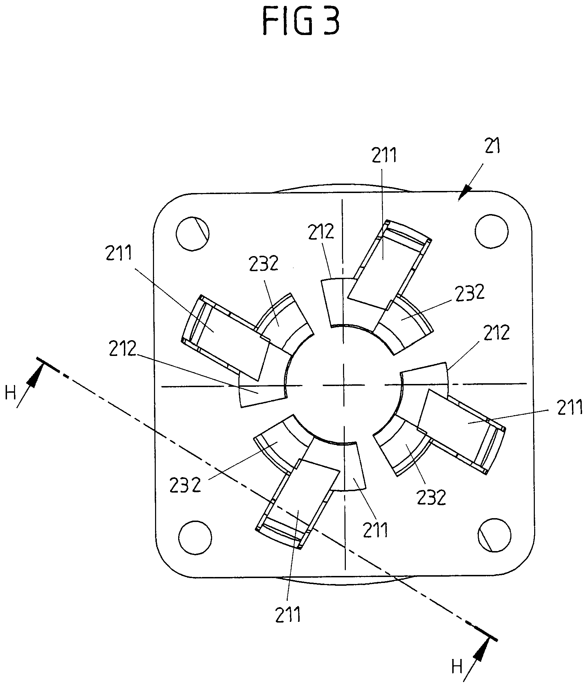

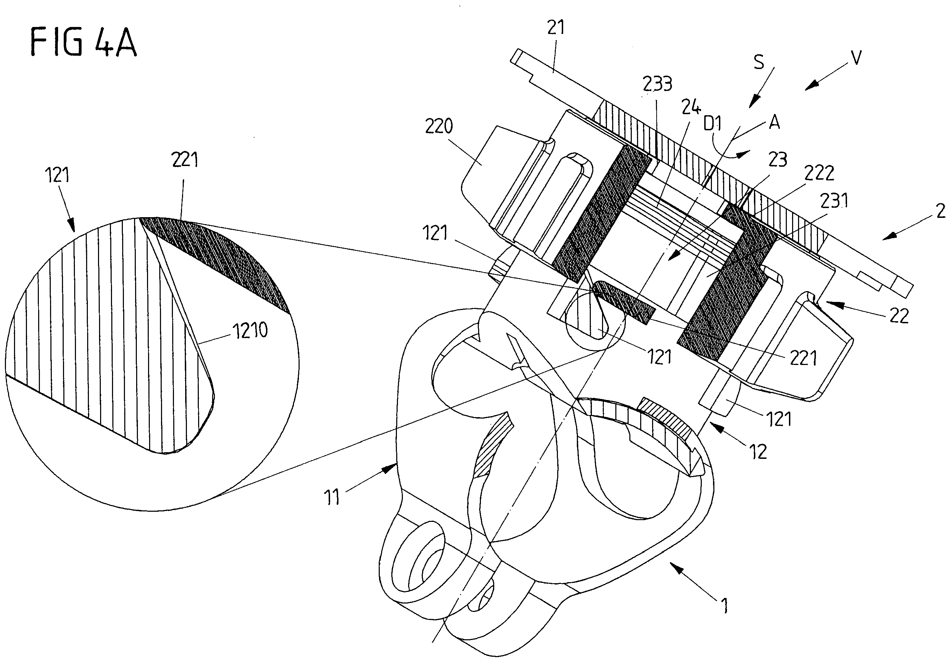

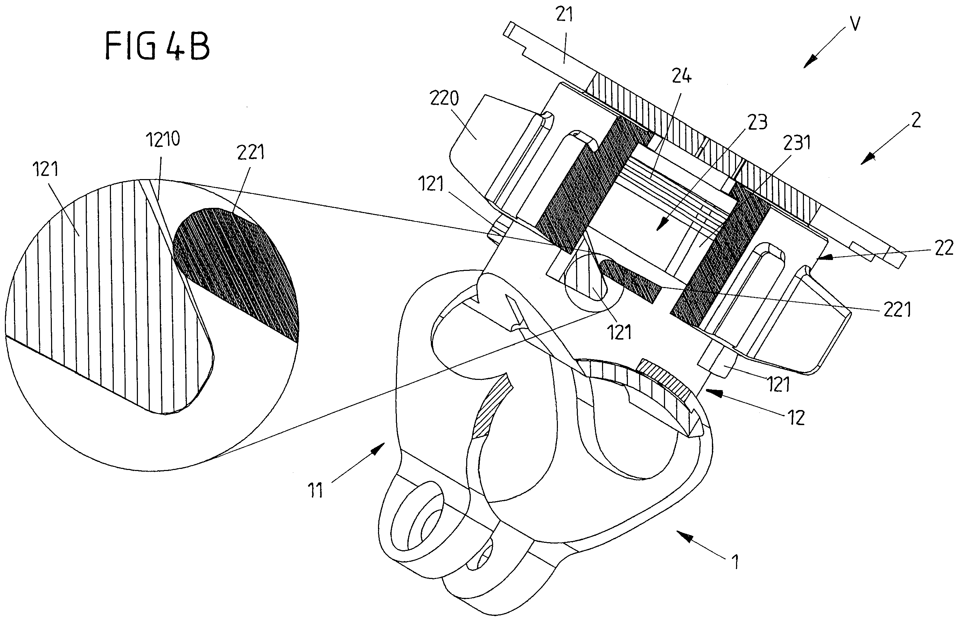

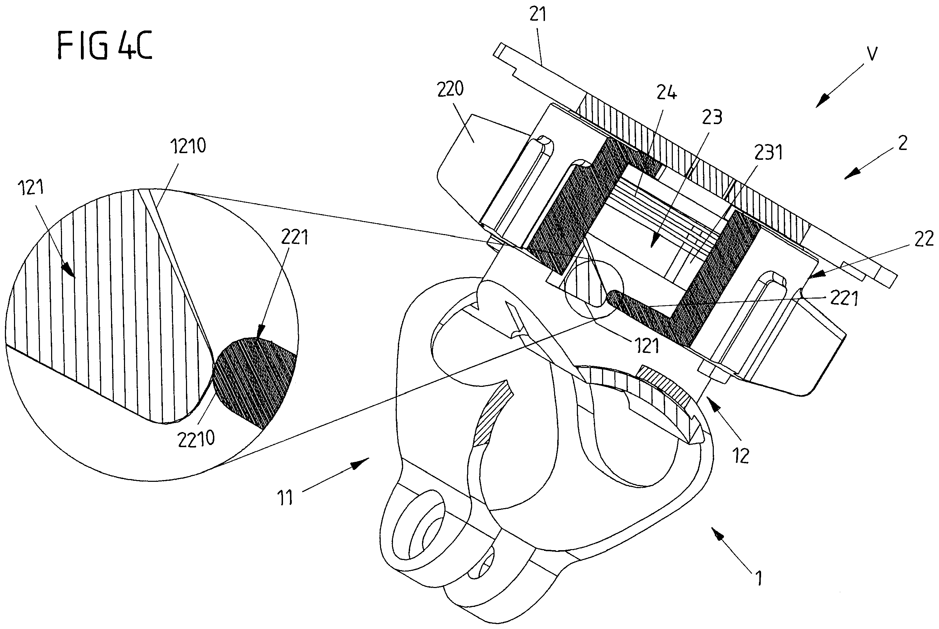

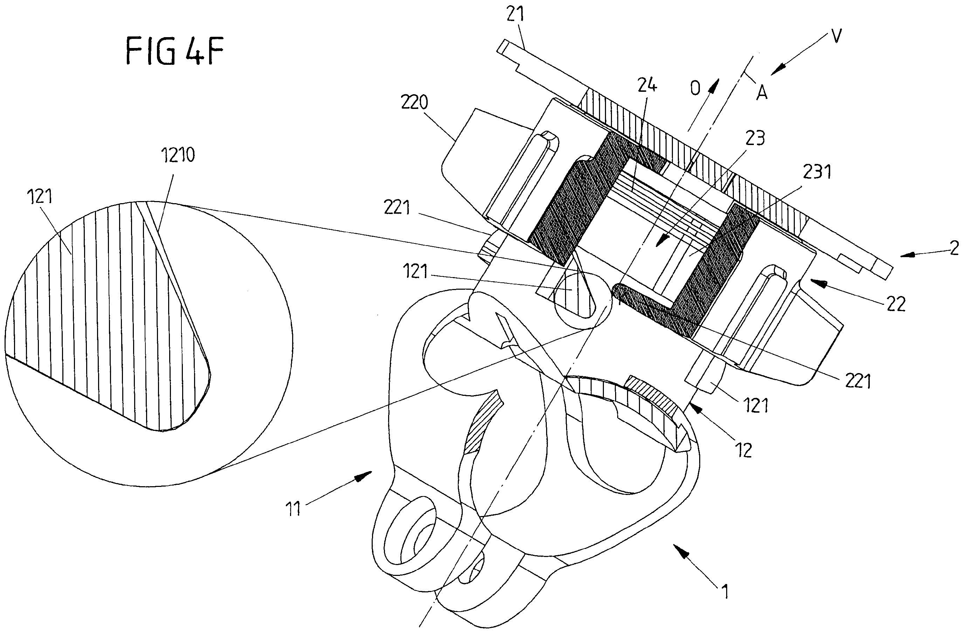

Zum Verschließen der Verschlussvorrichtung V wird beispielsweise das zweite Verschlussteil 2 entlang einer Verbindungsachse A in eine Schließrichtung S an das erste Verschlussteil 1 angesetzt und mit diesem verriegelt. Hierbei wird das (zweite) Verbindungselement 22 des zweiten Verschlussteils 2 in aufeinanderfolgenden Phasen eines Schließvorgangs in zueinander entgegengesetzte Drehrichtungen D1 und D2 um die Verbindungsachse A relativ zu dem (ersten) Verbindungselement des ersten Verschlussteils 1 gedreht, wie anhand der nachfolgend noch zu erläuternden Figuren 3 und 4A bis 4G im Detail veranschaulicht ist.

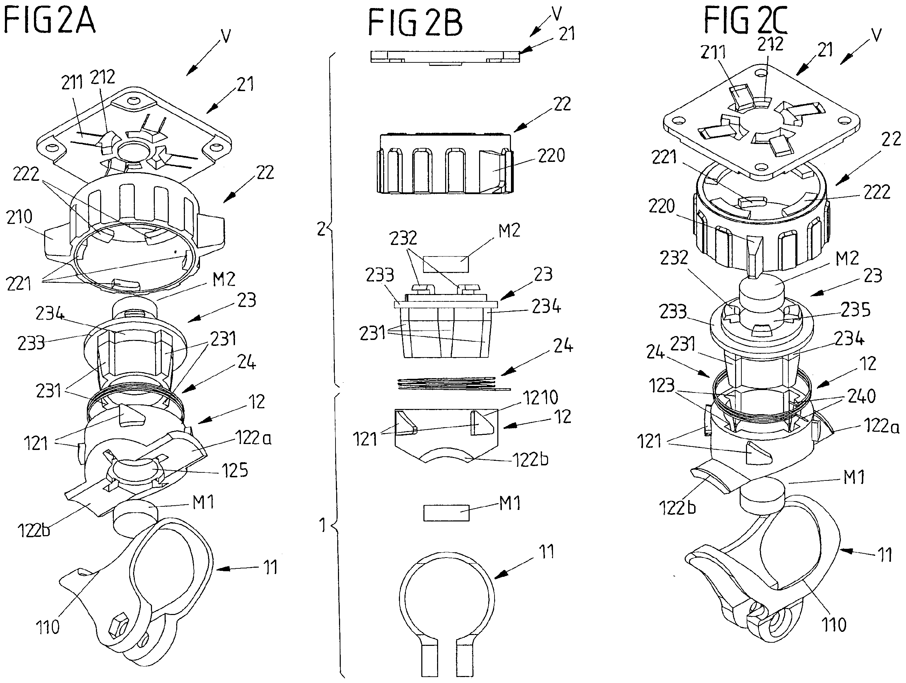

Zunächst soll der Aufbau der Verschlussteile 1 und 2 anhand der Explosionsansichten der Figuren 2A, 2B und 2C näher erläutert werden.

Aus diesen Explosionsansichten ist insbesondere ersichtlich, dass jedes der Verschlussteile 1, 2 mehrteilig aufgebaut ist und unter anderem jeweils ein Magnetelement M1 oder M2 umfasst, wobei die Magnetelemente M1 und M2 beim Ansetzen der Verschlussteile 1 und 2 aneinander magnetischen anziehend zwischen den Verschlussteilen 1 und 2 wirken. Das erste Magnetelement M1 des ersten Verschlussteils 1 ist vorliegend scheibenförmig ausgebildet. Das erste Magnetelement M1 ist dabei in einem Lagerabschnitt 125 des ersten Verbindungselements 12 mittig untergebracht.

Das erste Verbindungselement 12 bildet einen hohlzylindrischen Verbindungskörper aus, an dessen unteren Ende der Lagerabschnitt 125 vorgesehen ist und an dessen äußerer Mantelfläche mehrere zueinander beabstandete Führungsabschnitte 121 ausgebildet sind. Die einzelnen entlang einer Umfangslinie um die Verbindungsachse A zueinander beabstandeten Führungsabschnitte 121 des ersten Verbindungselements 12 sind im Querschnitt jeweils dreiecksförmig und stehen bezogen auf die Verbindungsachse A radial nach Außen an dem hohlzylindrischen Verbindungskörper des ersten Verbindungselements 12 hervor. Die einzelnen Führungsabschnitte 121 sind hierbei derart ausgestaltet, dass sie jeweils eine zu der Verbindungsachse A in Schließrichtung S geneigte Führungsfläche 1210 ausbilden, über die dem zweiten Verbindungselement 22 des zweiten Verschlussteils 2 unter der anziehenden Wirkung der Magnetelemente M1 und M2 nach dem Ansetzen des zweiten Verschlussteils 2 an das erste Verschlussteil 1 eine Drehung um die Verbindungsachse A bei weiterer Annäherung an das erste Verschlussteil 1 vorgegeben wird.

An seiner inneren Mantelfläche bildet der hohlzylindrischen Verbindungskörper des ersten Verbindungselements 12 erste Verdrehsicherungsabschnitte in Form von mehreren längserstreckten Ausnehmungen 123 aus. Diese Ausnehmungen 123 verlaufen längs der Verbindungsachse A und verjüngen sich entlang der Schließrichtung S, in der das zweite Verschlussteil 2 an das erste Verschlussteile 1 zum Schließen der Verschlussvorrichtung V angesetzt wird. Die vorliegend vier Ausnehmungen 123 sind jeweils um die Verbindungsachse A um 90° zueinander versetzt und dienen der Aufnahme von zweiten Verdrehsicherungsabschnitten in Form von längserstreckten, konischen Formschlusselementen 231 eines Verdrehsicherungselements 23 des zweiten Verschlussteils 2. Das Verdrehsicherungselement 23 des zweiten Verschlussteils 2 wird beim Ansetzen des zweiten Verschlussteils 2 an das erste Verschlussteil 1 in formschlüssigen Eingriff mit den Ausnehmungen 123 des ersten Verbindungselements 12 gebracht. Durch den Eingriff der Formschlusselemente 231 in die hierzu komplementär ausgebildeten Ausnehmungen 123 ist das Verdrehsicherungselement 23 des zweiten Verschlussteils während des Schließvorgangs drehfest mit dem ersten Verbindungselement 12 des ersten Verschlussteils 1 verbunden. Indem das zweite Befestigungselement 21 des zweiten Verschlussteils 2 wiederum drehfest mit dem Verdrehsicherungselement 23 verbunden ist, kann das Befestigungselement 21 und ein hieran befestigter Gegenstand in einer bestimmten Ausrichtung zu dem ersten Verschlussteil 1 verbleiben und muss nicht relativ zur Verbindungsachse A gedreht werden, um die beiden Verschlussteile 1 und 2 bestimmungsgemäß miteinander zu verbinden.

Über die Konusform der stegartig ausgebildeten und radial vorspringenden, längserstreckten Formschlusselemente 231 des Verdrehsicherungselements 23 sowie die Konusform der damit korrespondierenden längserstreckten Ausnehmungen 123 des ersten Verbindungselements 12 kann das Verdrehsicherungselement 23 nahezu toleranzfrei in das erste Verbindungselement 12 eingreifen, das bei geschlossener Verschlussvorrichtung V den eingesteckten Teil des Verdrehsicherungselements 23 rings um die Verbindungsachse A umlaufend umgibt.

Zur Fixierung des Verdrehsicherungselements 23 an dem Befestigungselement 21 sind an dem Verdrehsicherungselement 23 mehrere (vorliegend vier) Verschlusshaken 232 und an dem Befestigungselement 21 diesen Verschlusshaken 232 zugeordnete Bajonettöffnungen 212 mit Rastlaschen 211 vorgesehen. Die Verschlusshaken 232 des Verdrehsicherungselements 23 sind in die Bajonettöffnungen 212 des zweiten Befestigungselements 21 eingesteckt und hierin formschlüssig arretiert. Die einzelnen Rastlaschen 211 der Bajonettöffnung 212 sind jeweils elastisch gelagert und gestatten nach dem Einstecken eines Verschlusshaken 232 in die jeweilige Bajonettöffnung 212, dass der zugehörige Verschlusshaken 232 an einem radial innen liegenden Ende der jeweiligen Rastlasche 211 vorbei geführt wird, wenn das Verdrehsicherungselement 23 und das zweite Befestigungselement 21 beim Zusammenbau des zweiten Verschlussteils 2 relativ zueinander gedreht werden. Werden hierbei die Verschlusshaken 232 innerhalb der jeweils zugeordneten Bajonettöffnung 212 in einen Bereich mit kleineren Abmessungen verschoben, verrasten die Verschlusshaken 232 mit den Rastlaschen 211 und werden von diesen hintergriffen. Derart ist das Verdrehsicherungselement 23 an dem zweiten Befestigungselement 21 über eine Bajonettverbindung verriegelt und hiermit drehfest verbunden.

Die Verschlusshaken 232 stehen bezogen auf die Verbindungsachse A in axialer Richtung an dem Verdrehsicherungselement 23 hervor und sind oberhalb eines Befestigungskragen 233 des Verdrehsicherungselements 23 angeordnet. Nach der bestimmungsgemäßen Fixierung des Verdrehsicherungselements 23 an dem zweiten Befestigungselement 21 verbleibt zwischen dem Befestigungskragen 233 und einer Unterseite des zweiten Befestigungselements 21 ein kreisringförmiger Spalt. In diesen kreisringförmigen Spalt greifen radial nach innen vorstehende Lagerstege 222 des ebenfalls hohlzylindrischen zweiten Verbindungselements 22 ein, sodass diese Lagerstege 222 formschlüssig zwischen dem Befestigungskragen 233 und dem zweiten Befestigungselement 21 aufgenommen sind. Hierüber ist das zweite Verbindungselement 22 um die Schließrichtung S und die Verbindungsachse A drehbar an dem zweiten Verschlussteil 2 gelagert.

Das Verdrehsicherungselement 23 bildet ferner einen Lagerabschnitt 235 zur Aufnahme des zweiten Magnetelements M2 aus. Darüber hinaus ist an dem Verdrehsicherungselement 23 ein Federlagerbereich 234 vorgesehen, der in axialer Richtung zwischen den Formschlusselementen 231 und der Unterseite des Befestigungskragens 233 angeordnet ist. An diesem Federlagerbereich 234 lagert ein Federelement 24, das vorliegend als Spiralfeder ausgebildet ist. Dieses Federelement 24 stützt sich einerseits an dem zweiten Verbindungselement 22 des zweiten Verschlussteils 2 und andererseits an dem Verdrehsicherungselement 23 ab. Hierfür greift beispielsweise jeweils ein Federende 240 des Federelements 24 in eine hierfür vorgesehene Halteöffnung an dem zweiten Verbindungselement 22 oder dem Verdrehsicherungselement 23. Über das Federelement 24 wird derart das drehbar gelagerte zweite Verbindungselement 22 in eine Drehrichtung bezüglich des Verdrehsicherungselements 23 (und des damit drehfest verbundenen zweiten Befestigungselements 21) vorgespannt. Das Federelement 24 bildet damit vorliegend eine Belastungseinrichtung 24, über die das zweite Verbindungselement 22 beim Schließen der Verschlussvorrichtung V selbsttätig in eine Schließstellung überführt wird.

Für das Halten der beiden Verbindungselemente 12 und 22 und damit auch der Verschlussteile 1 und 2 aneinander weist das hohlzylindrische zweite Verbindungselement 22 an seiner inneren Mantelfläche mehrere (vorliegend vier) entlang einer Umfangslinie um die Verbindungsachse A zueinander äquidistant beabstandete Verschlussabschnitte 221 auf, die im geschlossenen Zustand der Verschlussvorrichtung V jeweils einen der Führungsabschnitte 121 zumindest teilweise hintergreifen. Die Verschlussabschnitte 221 sind hierbei jeweils als radial vorspringende und entlang einer Umfangslinie um die Verbindungsachse A längserstreckte Stege ausgebildet. Beim Schließen der Verschlussvorrichtung V treten diese Verschlussabschnitte 221 jeweils zunächst mit der geneigten Führungsfläche 1210 jeweils eines Führungsabschnitts 121 des ersten Verbindungselements 12 in Kontakt, wobei über die geneigte Führungsfläche 1210 dem zweiten Verbindungselement 22 bei weiterer Annäherung an das erste Verbindungselement 12 eine Drehbewegung um die Verbindungsachse A und relativ zu dem ersten Verbindungselement 12 in eine erste Schließrichtung D1 aufgezwungen wird. Dies ist insbesondere anhand der Figuren 4A bis 4C näher veranschaulicht.

Beim Ansetzen des zweiten Verschlussteils 2 an das erste Verschlussteil 1 wirken die Magnetelemente M1 und M2 magnetisch anziehend zwischen dem ersten Verschlussteil 1 und dem zweiten. Verschlussteile 2. Hierfür weisen die als Permanentmagnete ausgebildeten Magnetelemente M1 und M2 der beiden Verschlussteile 1 und 2 mit ungleichnamigen Magnetpolen aufeinander zu, wenn das zweite Verschlussteil 2 an das erste Verschlussteil 1 bestimmungsgemäß angesetzt wird, sodass eine magnetische Anziehungskraft in die Schließrichtung S zwischen den Magnetelementen M1 und M2 wirkt. Unter Wirkung der Magnetelemente M1 und M2 werden somit die beiden Verschlussteile 1 und 2 einander angenähert. Durch die in Schließrichtung S wirkende resultierende Magnetkraft wird jeder Verschlussabschnitt 221 des drehbar gelagerten 2. Verbindungselements 22 gegen eine Führungsfläche 1210 eines Führungsabschnitts 121 des ersten Verbindungselements 12 gedrückt. Eine Gleitfläche 2210 des jeweiligen Verschlussabschnitts 221 gleitet hier dann an der in Schließrichtung S abfallenden Führungsfläche 1210 des Führungsabschnitts 121 entlang, sodass dem zweiten Verbindungselement 22 hierdurch eine Drehung um die Verbindungsachse A relativ zu dem ersten Verbindungselement 12 entlang einer ersten Drehrichtung D1 aufgezwungen wird, wenn die beiden Verschlussteile 1 und 2 unter Wirkung der Magnetelemente M1 und M2 einander weiter angenähert werden.

Da der Verdrehsicherungsabschnitt 23 des zweiten Verschlussteils 2 hierbei mit dem ersten Verbindungselement 12 in Eingriff steht und starr mit diesem verbunden ist, wird hierbei das zweite Verbindungselement 22 auch relativ zu dem Verdrehsicherungselement 23 gedreht, wodurch das an dem zweiten Verbindungselement 22 und dem Verdrehsicherungselement 23 angreifende Federelement 24 (stärker) gespannt wird und eine Rückstellkraft auf das zweite. Verbindungselement 22 in eine entgegengesetzte zweite Drehrichtung D2 ausgeübt.

Bei weiterer Annäherung der beiden Verschlussteile 1 und 2 wird dann das zweite Verbindungselement 22 soweit in die erste Drehrichtung D1 gedreht, bis es ein in dieser Drehrichtung D1 liegendes Ende des jeweiligen Führungsabschnitts 121 erreicht und somit in axialer Richtung, d.h., in Schließrichtung S an diesem Führungsabschnitt 121 jeweils vorbeiführbar ist. Durch weitere Annäherung des zweiten Verschlussteils 2 an das erste Verschlussteil 1 erreicht das zweite Verbindungselement 22 derart eine Zwischenstellung, in der dessen Verschlussabschnitte 221 jeweils vollständig an den Führungsabschnitten 121 des ersten Verbindungsabschnitts 12 vorbeigeführt sind und die einzelnen Führungsabschnitte 121 einer Drehung des zweiten Verbindungselements 22 in die entgegengesetzte Drehrichtung D2 nicht mehr entgegenwirken.

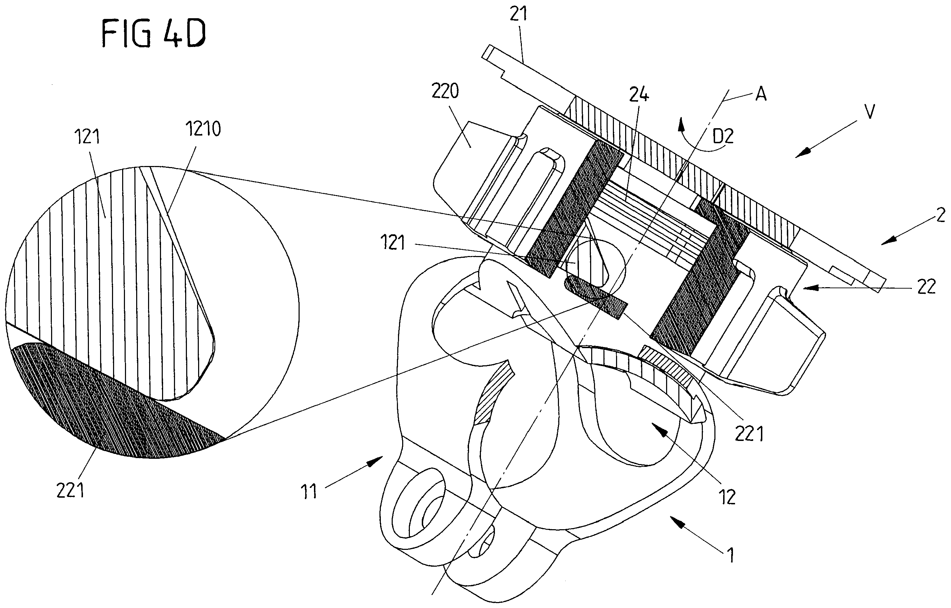

Unter Wirkung des Federelements 24 wird dann in einer anschließenden zweiten Phase eines Schließvorgangs das zweite Verbindungselement 22 des zweiten Verschlussteils 2 selbsttätig in eine Schließstellung entsprechend der Figur 4D gedreht. In dieser Schließstellung hintergreift jeder radial nach innen vorspringende Verschlussabschnitt 221 des zweiten Verbindungselements 22 zumindest teilweise einen radial nach außen vorspringenden Führungsabschnitt 121 des ersten Verbindungselements 12. Auf diese Weise sind die beiden Verbindungselemente 121 und 221 miteinander verriegelt und aneinander gehalten, sodass diese entlang der Verbindungsachse A aneinander arretiert und nicht ohne weiteres voneinander lösbar sind.

Jeder Verschlussabschnitt 221 weist an seinem in der zweiten Drehrichtung D2 liegenden Ende eine konvexe Wölbung auf. Über diese Wölbung und die hieran bereitgestellte Gleitfläche 2210 gleitet der Verschlussabschnitt 221 unter Wirkung der Magnetkraft der beiden Magnetelemente M1 und M2 an der Fläche 1210 des Führungsabschnitts 121 entlang. Ferner ist über die Wölbung das Vorbeiführen des Verschlussabschnitts 221 in der zweiten Drehrichtung D2 an dem Führungsabschnitt 121 erleichtert, wenn das zweite Verbindungselement 22 in seine Schließstellung überführt wird.

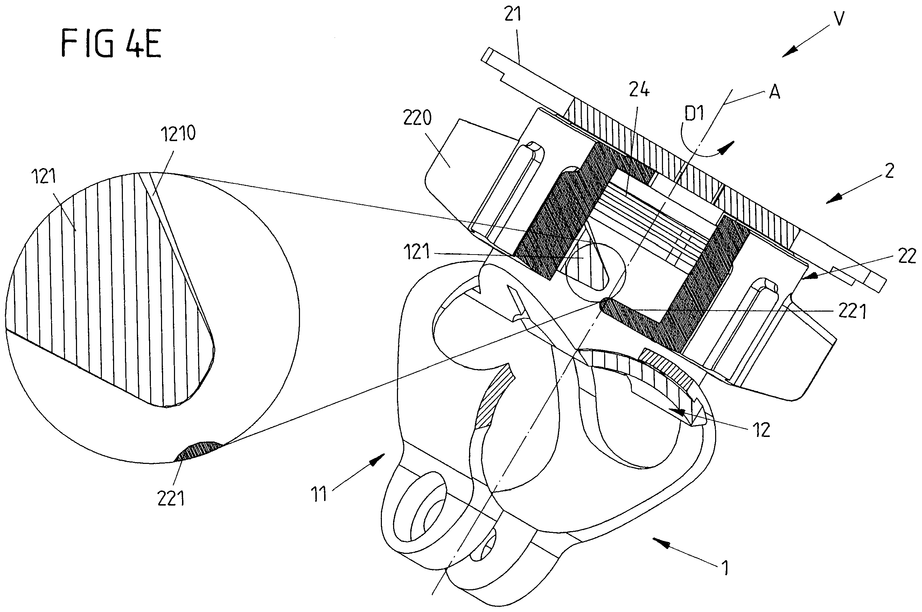

Für ein Öffnen der Verschlussvorrichtung V muss vielmehr entsprechend den Figuren 4E bis 4G das zweite Verbindungselement 2 relativ zu dem ersten Verbindungselement 12 in die erste Drehrichtung D1 um die Verbindungsachse A gedreht werden. Hierfür ist an dem zweiten Verbindungselement ein Griffbereich 220 vorgesehen. Dieser Griffbereich 220 kann von einem Nutzer bequem mit zwei Fingern ergriffen und gedreht werden. Wird das zweite Verbindungselement 22 in die erste Drehrichtung D1 - gegen die Rückstellkraft des Federelements 24 - so weit gedreht, dass die einzelnen Verschlussabschnitte 221 die Führungsabschnitte 121 des ersten Verbindungselements 12 nicht mehr hintergreifen, kann das zweite Verschlussteil 2 entlang der Verbindungsachse A in eine Öffnungsrichtung O von dem ersten Verschlussteil 1 abgezogen werden.

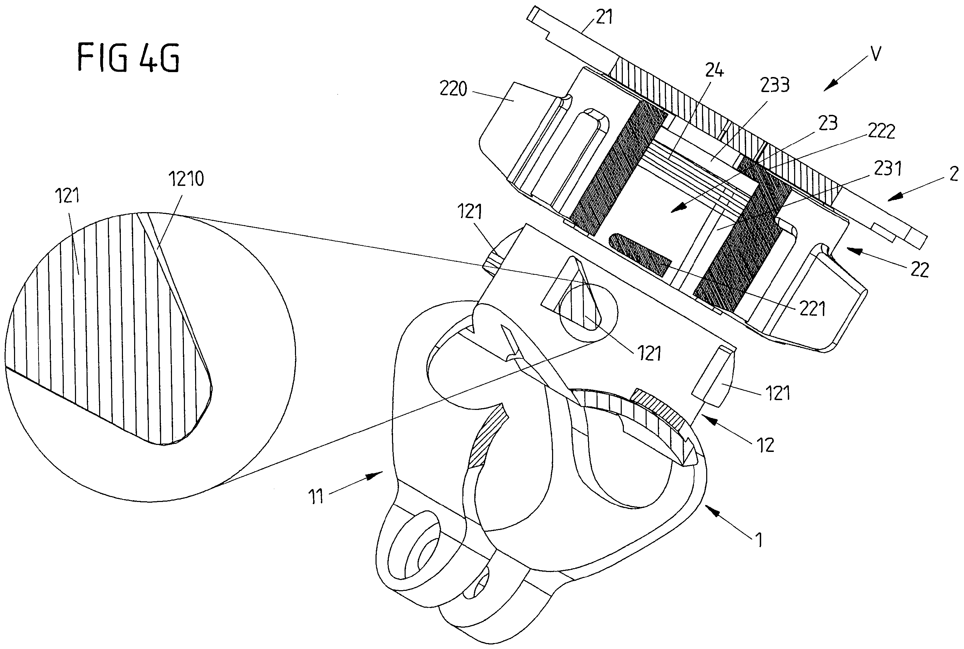

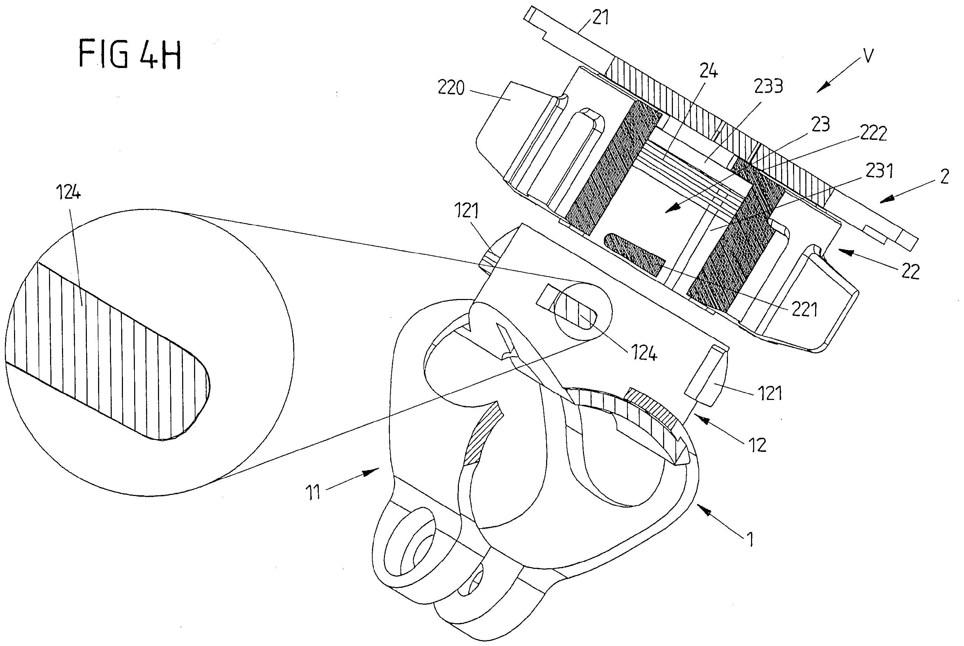

In der Figur 4H ist noch in mit der Figur 4G übereinstimmender Ansicht eine abgewandelte Verschlussvorrichtung V veranschaulicht, bei der derjenige Abschnitt, der eine Führungsfläche 1210 bereitstellt und derjenige Abschnitt, der von einem Verschlussabschnitt 221 hintergriffen wird, um die beiden Verschlussteile 1, 2 aneinander zu halten, auseinanderfallen. Sind in der Ausführungsvariante der Figuren 1A bis 4G an einem ersten Verbindungselement 12 entlang seines Umfang lediglich zueinander äquidistant beabstandete Führungsabschnitte 121 für die Generierung der Drehung des zweiten Verbindungselementes 22 vorgesehen, wechseln sich bei der Verschlussvorrichtung V der Figur 4G entlang des Umfang des ersten Verbindungselements 12 Führungsabschnitte 121 und Halteabschnitte 124 einander ab. Die Halteabschnitte 124 bilden dabei jeweils keine Führungsfläche 1210 für die Generierung einer Drehbewegung durch das zweite Verbindungselement 22 aus, werden aber in der Schließstellung jeweils von einem Verschlussabschnitt 221 des zweiten Verbindungselements 22 hintergriffen. In der dargestellten Ausführungsvariante sind an dem ersten Verbindungselement 12 zwei Führungsabschnitte 121 und zwei Halteabschnitte 124 entlang der im Querschnitt kreisförmigen Mantelfläche alle 90° einander abwechselnd vorgesehen, d.h., bei 90° (3 Uhr) und 270° (9 Uhr) jeweils ein Führungsabschnitt 121 und bei 180° (6 Uhr) und 360° (12 Uhr) jeweils ein Halteabschnitt 124. Im Übrigen bleiben aber die zuvor beschrieben Funktionen und Merkmale identisch.

Es sei ferner noch darauf hingewiesen, dass selbstverständlich auch bei den nachfolgend erläuterten Ausführungsvarianten wenigstens ein Führungsabschnitt durch einen Halteabschnitt ersetzt sein kann, um über unterschiedlich ausgestaltete Abschnitte des ersten Verbindungselements 12 eine funktionale Trennung zwischen Führen und Halten des zweiten Verbindungselements 22 an dem ersten Verbindungselement 12 vorzunehmen. Beispielsweise können sich auch hier dann in einer Weiterbildung Führungsabschnitte mit Führungsfläche(n) und Halteabschnitte paarweise entlang des Umfangs abwechseln.

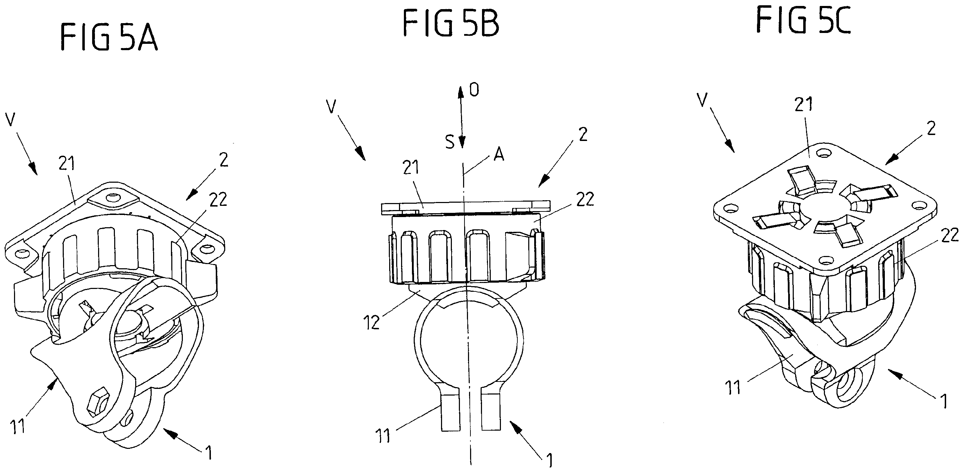

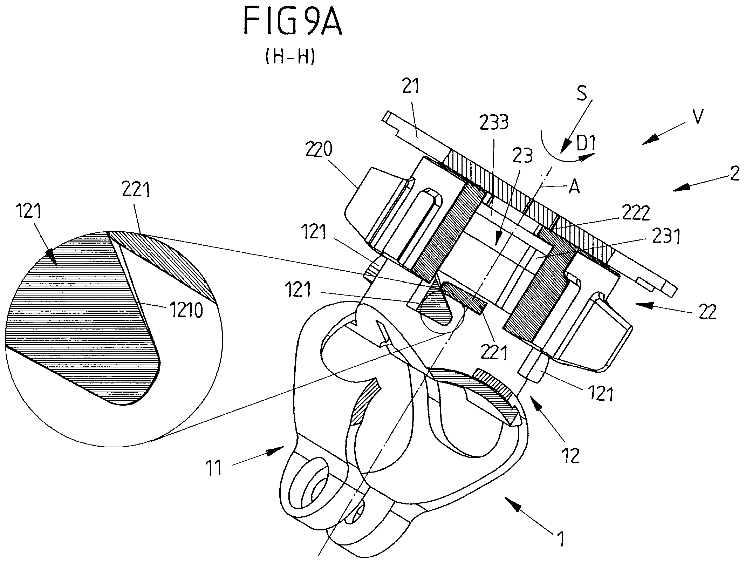

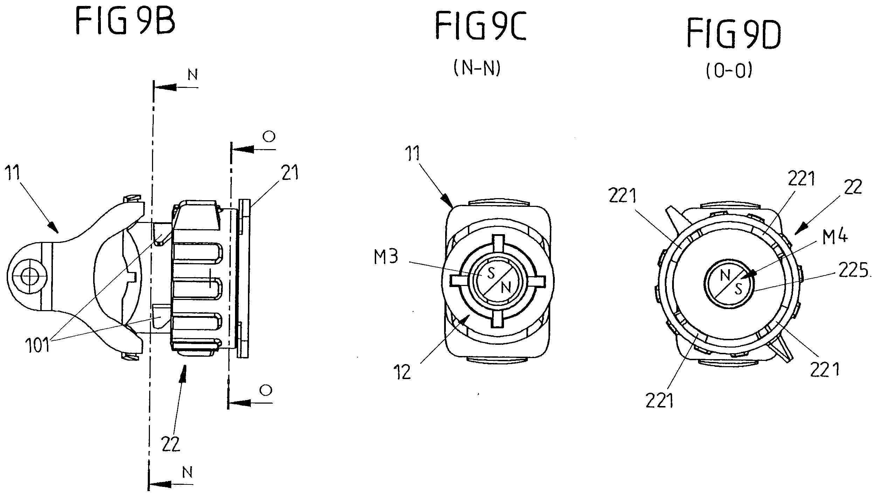

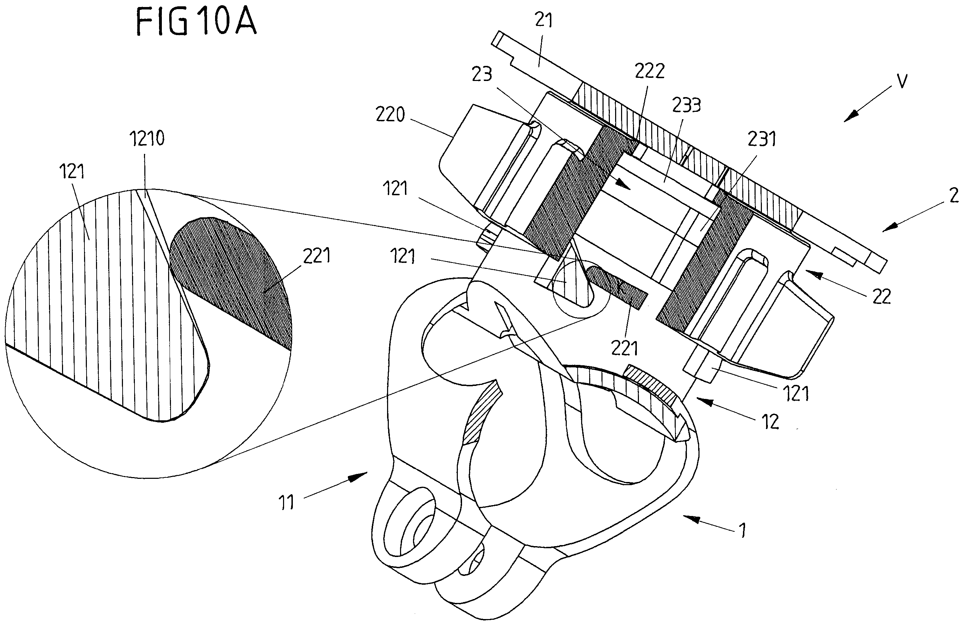

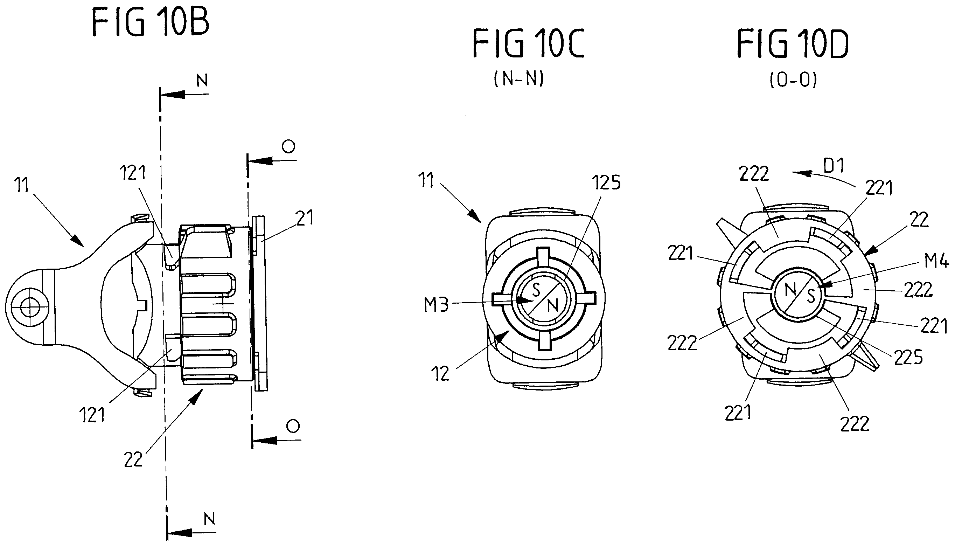

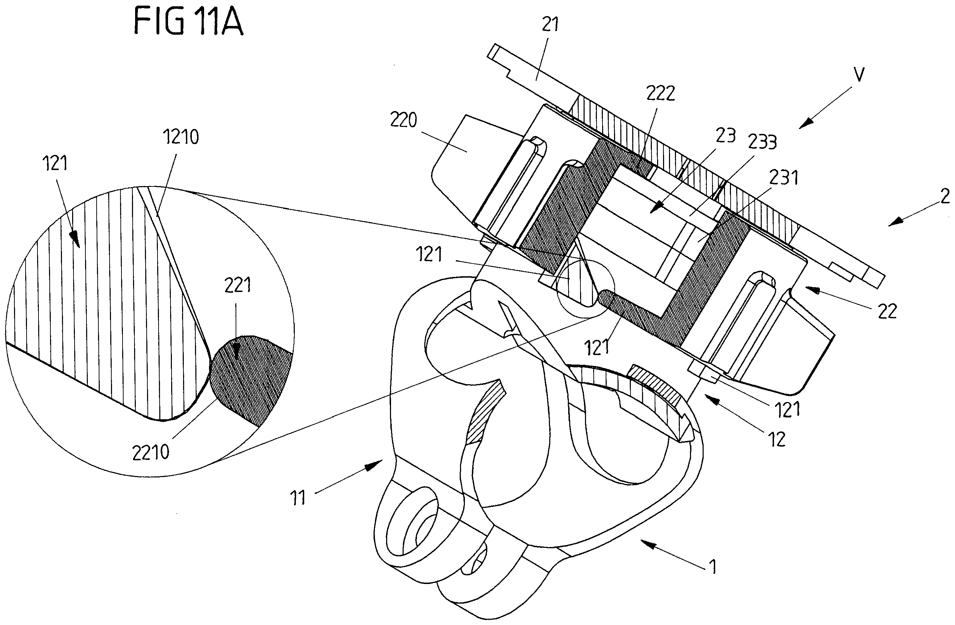

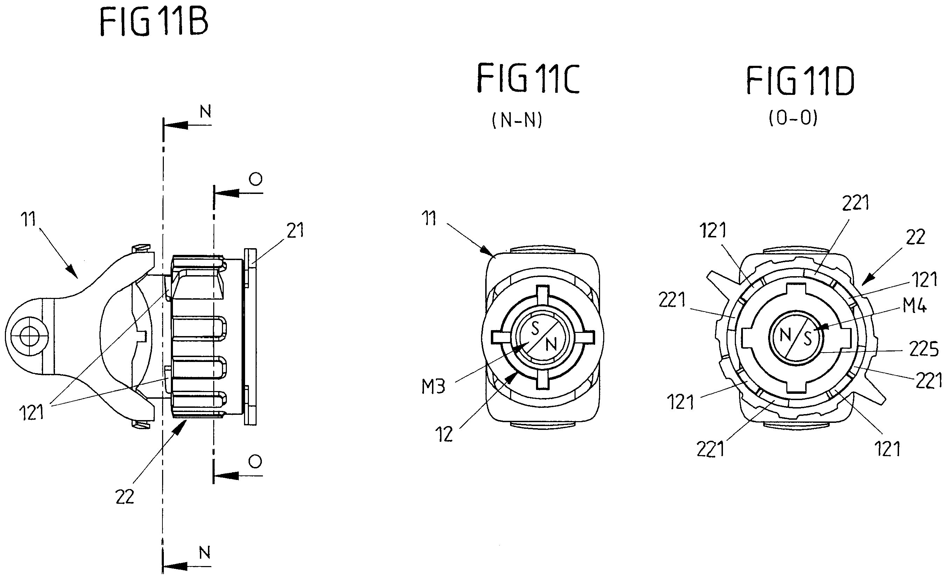

Die Figuren 5A bis 14D zeigen in unterschiedlichen Ansichten eine zweite Ausführungsvariante einer erfindungsgemäßen Verschlussvorrichtung V, bei der identische Komponenten mit übereinstimmenden Bezugszeichen gekennzeichnet sind.

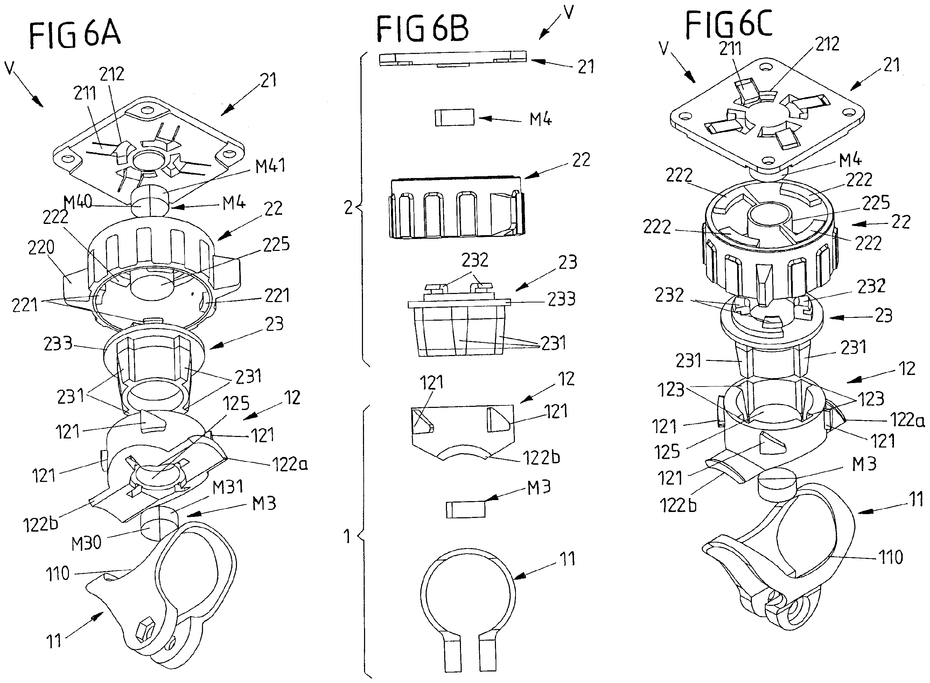

So weist eine Verschlussvorrichtung V der Figuren 6A bis 14D ebenfalls zwei jeweils mehrteilig aufgebaute Verschlussteile 1 und 2 auf. Das erste Verschlussteil 1 umfasst hierbei unter anderem in Übereinstimmung mit der ersten Ausführungsvariante der Figuren 1A bis 4G ein erstes Befestigungselement 11 sowie ein erstes Verbindungselement 12 mit hieran ausgebildeten Führungsabschnitten 121. Das zweite Verschlussteil 2 weist ferner ein zweites Befestigungselement 21 und ein drehfest hiermit verbundenes Verdrehsicherungselement 23 auf sowie ein relativ zu dem zweiten Befestigungselement 21 und dem Verdrehsicherungselement 23 drehbar gelagertes zweites Verbindungselement 22.

Im Unterschied zu der ersten Ausführungsvariante der Figuren 1A bis 4G ist vorliegend anstelle einer federkraftbasierten Verstelleinrichtung mit einem Federelement 24 eine magnetkraftbasierte Verstelleinrichtung mit zwei Magnetelementen im M3 und M4 zur Vorspannung des zweiten Verbindungselements 22 vorgesehen. Die Magnetelemente M3 und M4, die hier durch scheibenförmige und diametral, d.h., entlang ihres Durchmessers und mithin quer zu Verbindungsachse A magnetisierte Permanentmagnete gebildet sind, sind hierbei einerseits an dem Lagerabschnitt 125 des ersten Verbindungselements 12 und andererseits an einem Lagerabschnitt 225 des zweiten Verbindungselements 22 angeordnet.

Die beiden Magnetelemente M3 und M4 weisen jeweils zwei Magnetsegmente M30, M31 oder M40, M41 auf und sind hierbei derart an den jeweiligen Verschlussteilen 1 und 2 angeordnet, dass verschiedene, ungleichnamige Magnetpole jeweils in einer quer zu Verbindungsachse A und mithin zur Schließrichtung S verlaufenden Ebenen nebeneinander vorliegen. Es hängt somit von einer relativen Drehlage der beiden Verschlussteile 1 und 2 zueinander bezüglich der Verbindungsachse A ab, ob sich die beiden Magnete M3 und M4 anziehen oder abstoßen. Liegen sich beispielsweise der Nordpol eines Magnetsegments M40 des zweiten Verschlussteils 2 und ein Südpol des Magnetsegments M31 des ersten Verschlussteils 1 gegenüber, liegen sich auch der Südpol des Magnetsegment M41 des zweiten Verschlussteils 2 und der Nordpol M31 des ersten Verschlussteils 1 gegenüber und die beiden Magnetelemente M3 und M4 wirken einander anziehend. Bei nicht vollkommen deckungsgleicher Ausrichtung der beiden Magnetelemente M3 und M4 zueinander bezüglich der Verbindungsachse A, d.h., bei nicht paralleler Ausrichtung der zwischen den Magnetsegmenten M30 und M31 sowie M40 und M41 verlaufenden Trennlinien zueinander, sind die Magnetelement M3 und M4 stets bestrebt, sich entsprechend zueinander auszurichten, sodass hierbei dann ein Drehmoment in die eine oder andere Drehrichtung um die Verbindungsachse A erzeugt wird.

Dieser Umstand wird sich vorliegend zunutze gemacht, um die Magnetelemente M3 und M4 als Teil einer Verstelleinrichtung zu nutzen, mittels der beim Schließen der Verschlussvorrichtung V das zweite Verbindungselement 22 in seiner Zwischenstellung mit einer Magnetkraft beaufschlagt wird. Über diese Magnetkraft wird das zweite Verschlusselement 22 in eine Schließstellung relativ zu dem erste Verbindungselement 12 gedreht, in der die Verschlussabschnitte 221 des zweiten Verbindungselements 22 die Führungsabschnitte 121 des ersten Verbindungselements 12 hintergreifen.

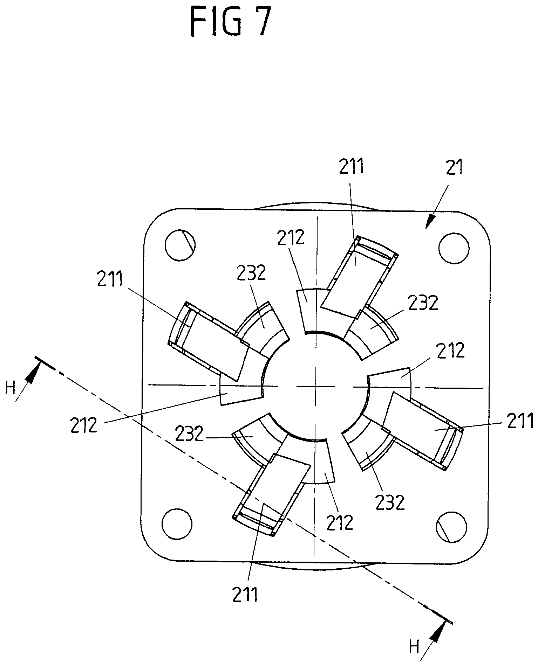

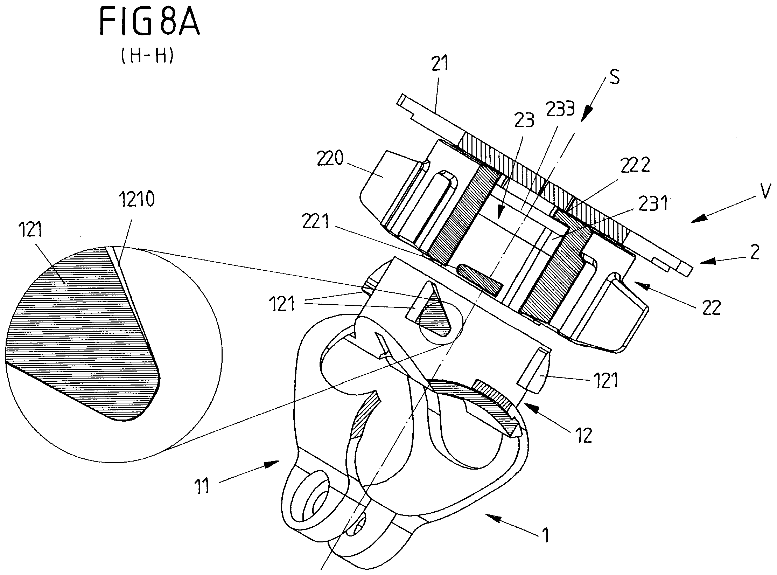

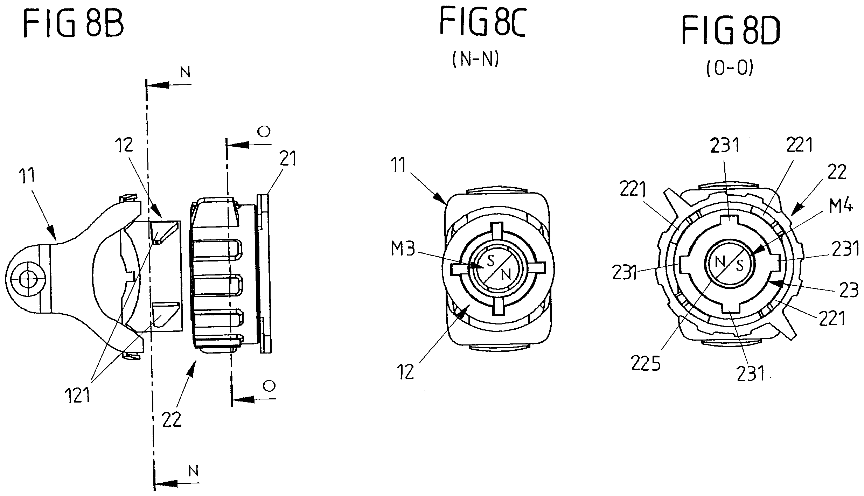

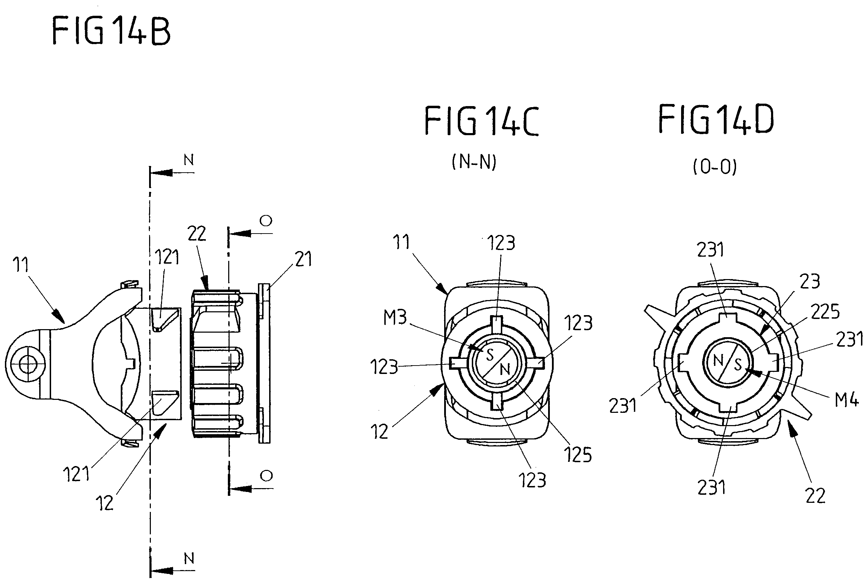

Während mit den Figuren 5A bis 5C und 6A bis 6C jeweils der Aufbau der Verschlussvorrichtung V gemäß der zweiten Ausführungsvariante veranschaulicht ist, verdeutlichen die Figuren 7 und 8A bis 14D die Funktion der Verschlussvorrichtung V beim Schließen und Öffnen. Dabei zeigt die Figur 7 in Draufsicht eine Schnittlinie H-H, entlang der anhand der jeweils mit "A" gekennzeichneten nachfolgenden Figuren 8A bis 14A die Verschlussvorrichtung V in unterschiedlichen Phasen beim Schließen und Öffnen dargestellt ist. In den jeweils mit "C" und "D" bezeichneten Figuren 8C/8D bis 14C/14D sind jeweils Querschnittsdarstellungen gezeigt, die sich aus Schnitten entlang von Schnittlinien N-N und O-O der mit "B" gekennzeichneten Figuren 8B bis 14B ergeben. Anhand der Querschnittsdarstellungen ist hierbei insbesondere die sich ändernde Ausrichtung der diametral magnetisierten Magnetelementen M3 und M4 des ersten und zweiten Verschlussteils 1, 2 veranschaulicht.

Anhand der Figuren 8A bis 11D ist eine erste Phase eines Schließvorgangs beim Schließen der Verschlussvorrichtung V veranschaulicht. In dieser ersten Phase wird das an das erste Verschlussteil 1 entlang der Schließrichtung S angesetzte zweite Verschlussteil 2 unter der anziehenden Wirkung der Magnetelemente M3 und M4 dem ersten Verschlussteil 1 angenährt. Hierbei wird dem drehbar an dem Verdrehsicherungsabschnitt 23 gelagerten zweiten Verbindungselement 22 des zweiten Verschlussteils 2 eine Drehbewegung um die Verbindungsachse A in die erste Drehrichtung D1 aufgezwungen, indem die einzelnen Verschlussabschnitte 221 des zweiten Verbindungselements 22 jeweils an den schräg verlaufenden Führungsflächen 1210 der einzelnen Führungsabschnitte 121 des ersten Verbindungselements 12 hinab gleiten.

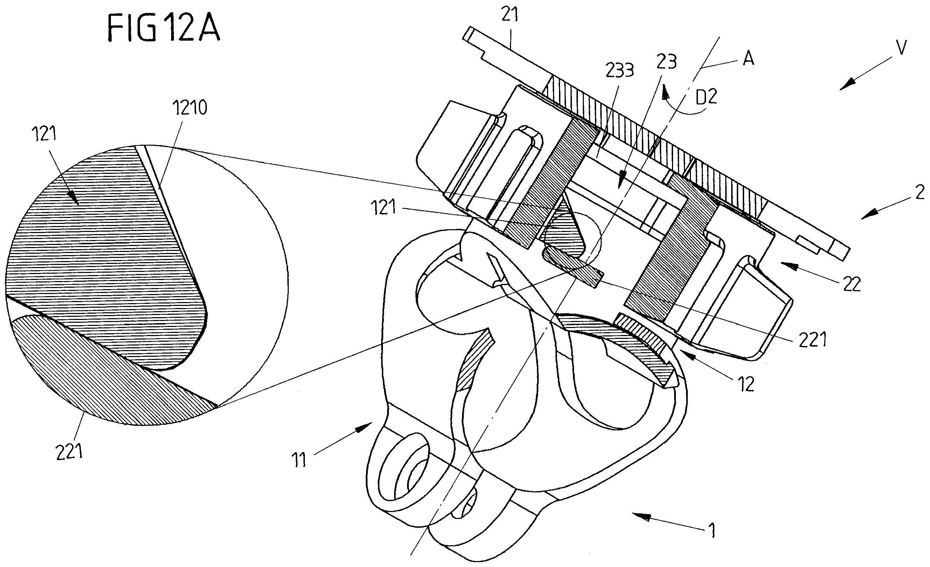

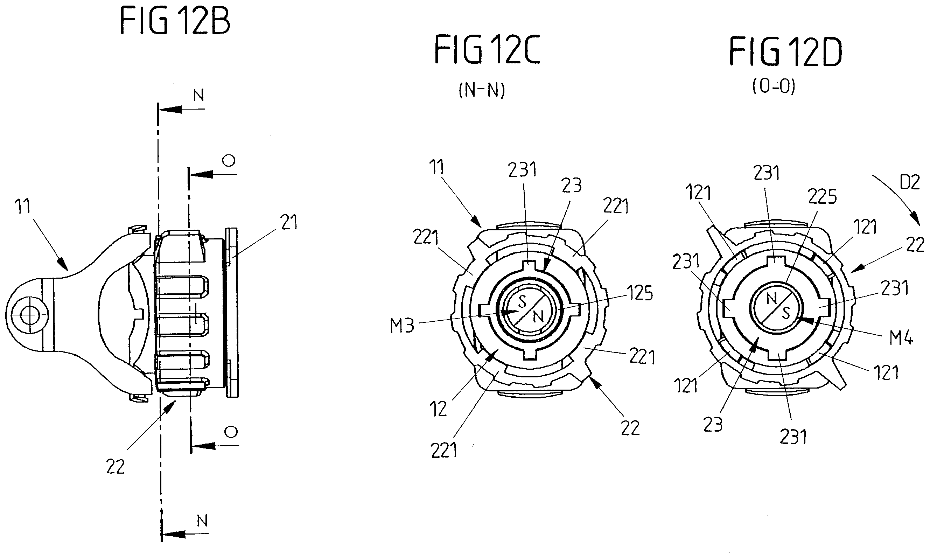

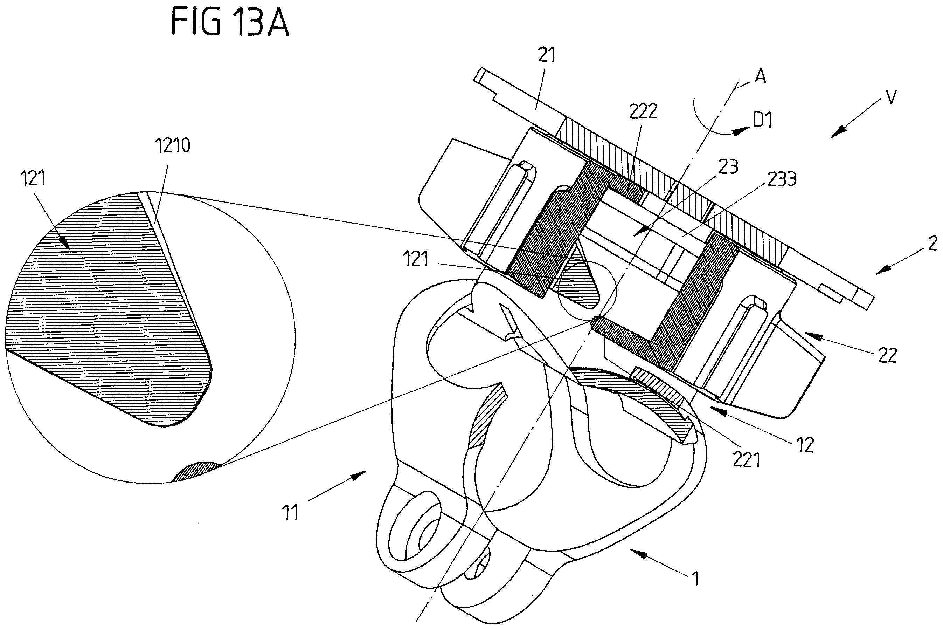

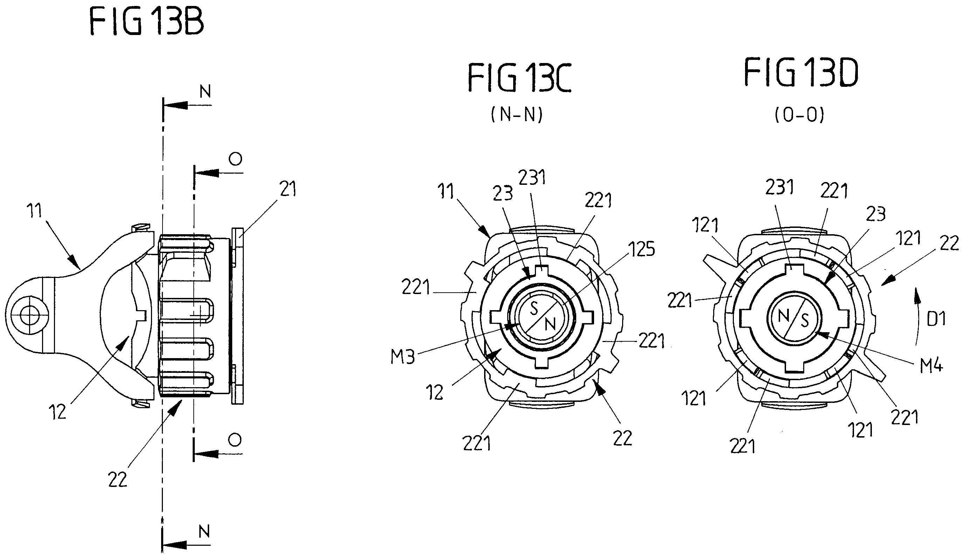

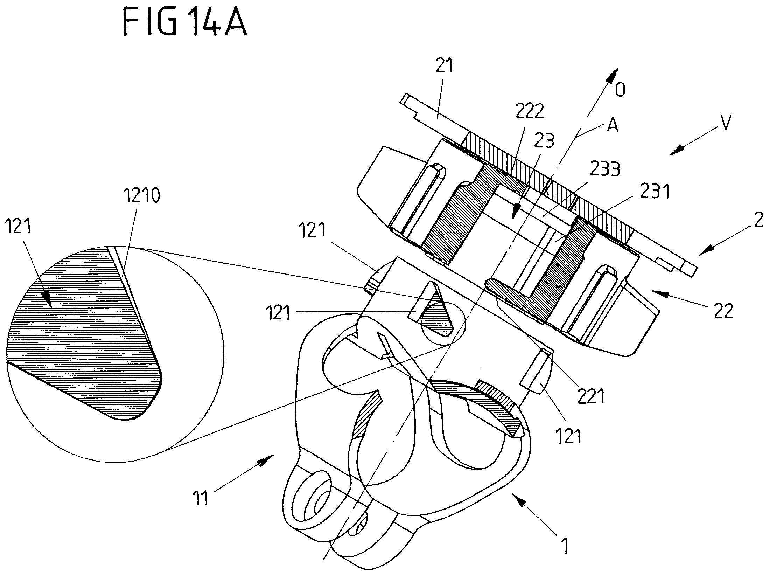

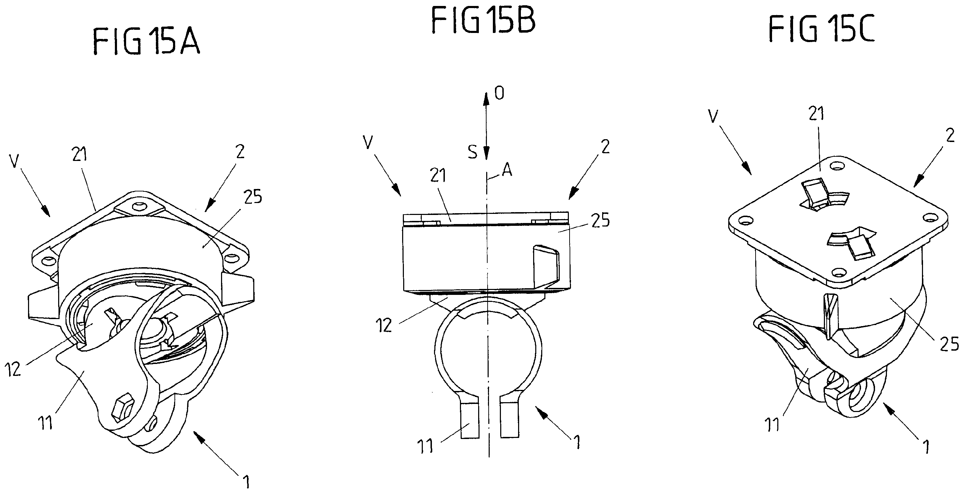

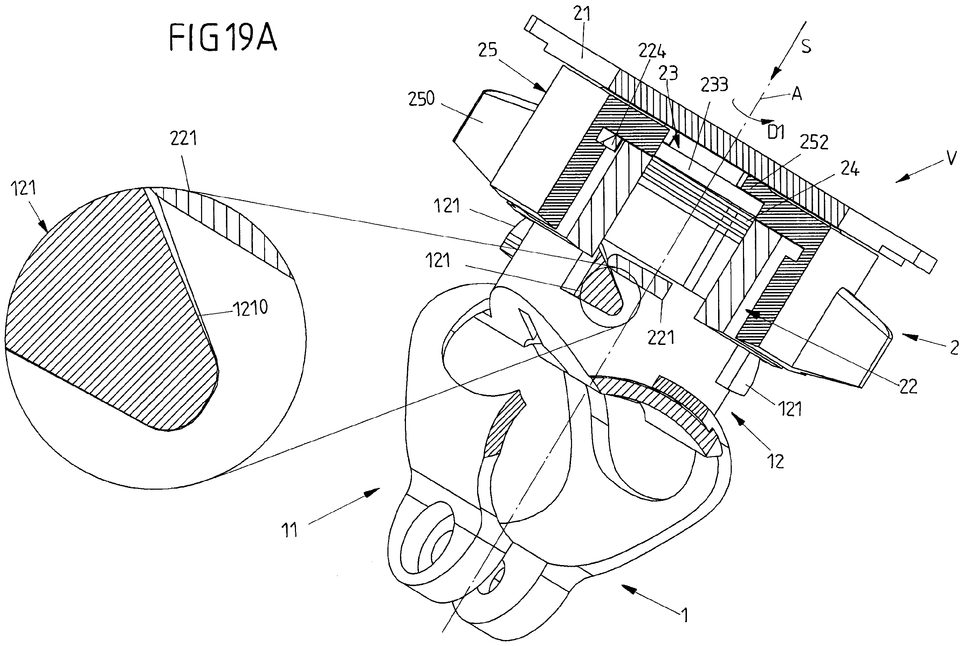

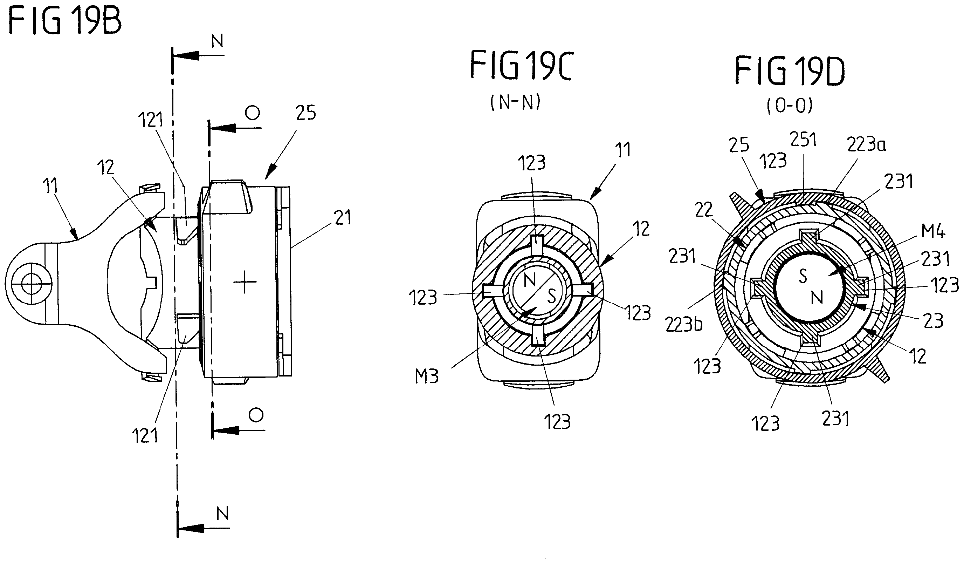

Wurde das zweite Verschlussteil 2 dem ersten Verschlussteil 1 ausreichend angenährt, sodass die sich jeweils in Umfangsrichtung erstreckenden Verschlussabschnitte 221 jeweils an ihren Führungsabschnitten 121 in Schließrichtung S vorbei geführt sind und damit das zweite Verbindungselement 22 in einer hierdurch definierten Zwischenstellung vorliegt, liegen die beiden Magnetelemente M3 und M4 bezogen auf ihre ungleichnamigen Magnetpole der beiden Magnetsegmente M30, M31 und M40, M41 nicht exakt übereinander. Eine diametral verlaufende Trennlinie des einen Magnetelements M3 verläuft somit nicht parallel zur Trennlinie des anderen Magnetelements M4. Durch das Bestreben der beiden Magnetelemente M3 und M4 sich so zueinander auszurichten, dass ein Nordpol des Magnetsegment des einen Magnetelements M4 einem Südpol des Magnetsegment des anderen Magnetpol M3 axial direkt gegenüber liegt, wird in der Zwischenstellung des zweiten Verbindungselements 22 ein Drehmoment erzeugt, über das das zweite Verbindungselement 22 in seine Schließstellung in die zweite Drehrichtung D2 gedreht wird.
In der Schließstellung entsprechend den Figuren 12A bis 12D hintergreift jeder Verschlussabschnitt 221 des zweiten Verbindungselements 22 einen zugeordneten Führungsabschnitt 121 des ersten Verbindungselements 12 und die beiden Magnetelemente M3 und M4 sind exakt zueinander ausgerichtet.
Wird aus dem hiermit definierten geschlossenen Zustand der Verschlussvorrichtung V das zweite Verbindungselement 22 wieder in die erste Drehrichtung D1 entgegen der aufgebrachten Magnetkraft gedreht, wie dies anhand der Figuren 13A bis 13D veranschaulicht ist, kann ein Hintergriff der Verschlussabschnitte 221 gelöst werden. Hiernach sind dann die Verschlussabschnitte 221 in die zu der Schließrichtung S entgegengesetzte Öffnungsrichtung O an den Führungsabschnitten 121 vorbeiführbar und das zweite Verschlussteil 2 kann somit von dem ersten Verschlussteil 1 abgezogen werden, wie dies in den Figuren 14A bis 14D veranschaulicht ist.
Bei einer Verschlussvorrichtung V gemäß einer dritten Ausführungsvariante entsprechend den Figuren 15A bis 26B ist an dem zweiten Verschlussteil 2 zusätzlich zu einem zweiten Verbindungselement 22, einem Verdrehsicherungselement 23 und einem zweiten Befestigungselement 21 noch ein Betätigungselement 25 vorgesehen.
Das Betätigungselement 25 ist hier hohlzylindrisch ausgeführt und weist einen zentralen Lagerabschnitt 255 auf, in dem das zweite diametral magnetisierte Magnetelement M4 angeordnet ist. Über radial nach innen vorspringende Lagerstege 252 ist das Betätigungselement 25 an dem Befestigungskragen 233 des Verdrehsicherungselement 23 drehbar gelagert. Das zweite Verbindungselement 22 des zweiten Verschlussteils 2 ist hierbei wiederum an dem Betätigungselement 25 drehbar gehalten. Hierfür bildet das zweite Verbindungselement 22 radial vorstehende Vorsprünge 224 aus, über die das zweite Verbindungselement 22 an dem Betätigungselement 25 drehbar gehalten ist. Im zusammengebauten Zustand des zweiten Verschlussteils 2 ist das zweite Verbindungselement 2 dann um die Verbindungsachse A umlaufend von dem Betätigungselement 25 umgeben und zwischen dem Betätigungselement 25 und dem Verdrehsicherungselement 23 angeordnet.

An dem Verdrehsicherungselement 23 lagert an einem Federlagerbereich 234 ein Federelement 24, das hier erneut als Schenkelfeder ausgebildet ist. Das Federelement 24 stützt sich einerseits an dem Verdrehsicherungselement 23 und andererseits an dem zweiten Verbindungselement 22 ab, sodass das zweite Verbindungselement 22 über das Federelement 24 relativ zu dem Verdrehsicherungselement 23 elastisch vorgespannt ist. Das Federelement 24 bildet hier somit einen Teil einer Verstelleinrichtung an dem zweiten Verschlussteil 2, über die das zweite Verbindungselement 22 beim Schließen der Verschlussvorrichtung V mit einer Kraft in die Schließstellung beaufschlagt wird, um über seine Verschlussabschnitte 221 die Führungsabschnitte 121 des ersten Verbindungselements 12 des ersten Verschlussteils 1 zu hintergreifen. Die Verschlussabschnitte 221 sind hierbei in einem bezogen auf die Schließrichtung S unteren Bereich an einer inneren Mantelfläche des zweiten Verbindungselements 22 ausgebildet. Die mehreren, hier vier, zueinander äquidistant angeordneten Verschlussabschnitte 221 liegen somit auf einer Innenseite des zweiten Verbindungselements 22, während an einer äußeren Mantelfläche des zweiten Verbindungselements und damit an dessen Außenseite die Vorsprünge 224 vorgesehen sind. Diese Vorsprünge befinden sich im Übrigen an einem oberen Bereich des zweiten Verbindungsabschnitts 22.

An der äußeren Mantelfläche des zweiten Verbindungselements 22 sind vorliegend ferner noch sich parallel zur Schließrichtung S verlaufende, längserstreckte Gegenanschläge 223a und 223b ausgebildet. Über diese Gegenanschläge 223a und 223b wird im Zusammenspiel mit einem radial nach innen vorstehenden Anschlag 251 des Betätigungselements 25 eine Drehbarkeit des Betätigungselements 25 relativ zu dem zweiten Verbindungselement 22 begrenzt. Hierfür ist der Anschlag 251 des Betätigungselements 25 zwischen den zwei Gegenanschlägen 223a und 223b angeordnet, sodass das Betätigungselement 25 in beide mögliche Drehrichtungen D1 und D2 um die Verbindungsachse A oder die Schließrichtung S über einen der Gegenanschläge 223a, 223b nicht hinweg verstellt werden kann. Beispielsweise wird über die Gegenanschläge 223a und 223b die Drehbarkeit des Betätigungselements 25 relativ zu dem zweiten Verbindungselement 22 auf einen Drehwinkelbereich von weniger als 90°, insbesondere weniger als 80°, hier beispielsweise 70° begrenzt.

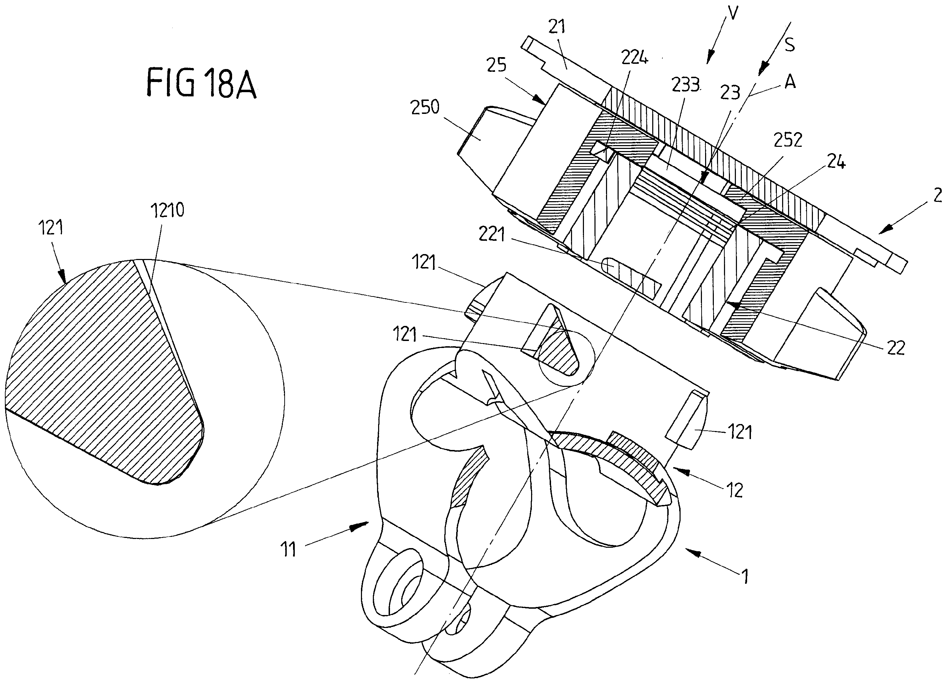

Durch Zusammenwirken des einen Gegenanschlag 223a des zweiten Verbindungselements 22 mit dem Anschlag 251 des Betätigungselements 25 wird das Betätigungselement 25 beim Schließen der Verschlussvorrichtung V in die erste Drehrichtung D1 mitgenommen. Für das Öffnen der Verschlussvorrichtung V wirkt ferner der Anschlag 251 mit dem anderen Gegenanschlag 223b zusammen, um beim Drehen des Betätigungselements 25 in die erste Drehrichtung D1 das zweite Verbindungselement 22 mitzunehmen. Dies wird nachfolgend noch anhand der Figuren 18A bis 25D näher erläutert werden.

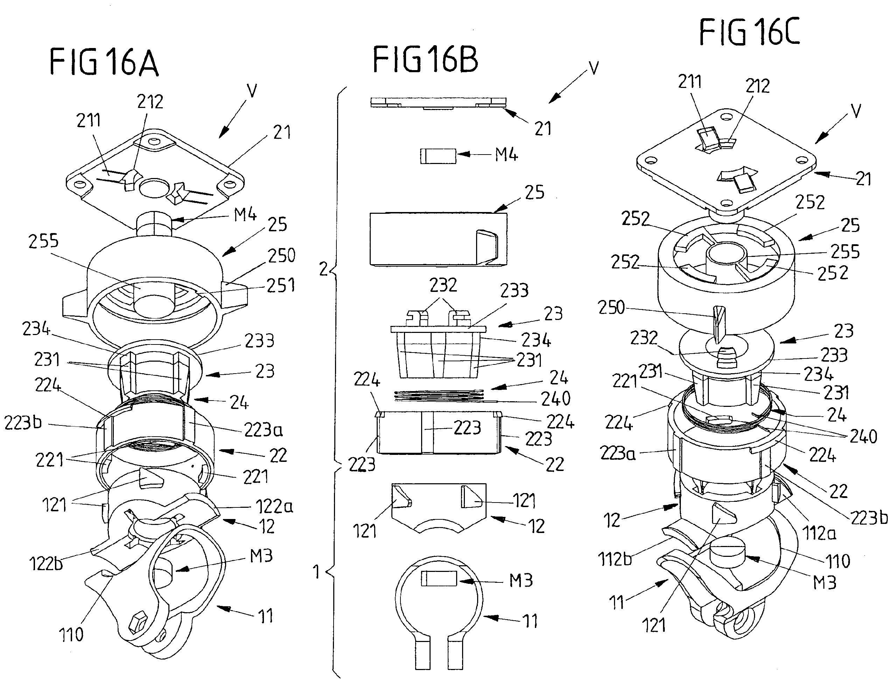

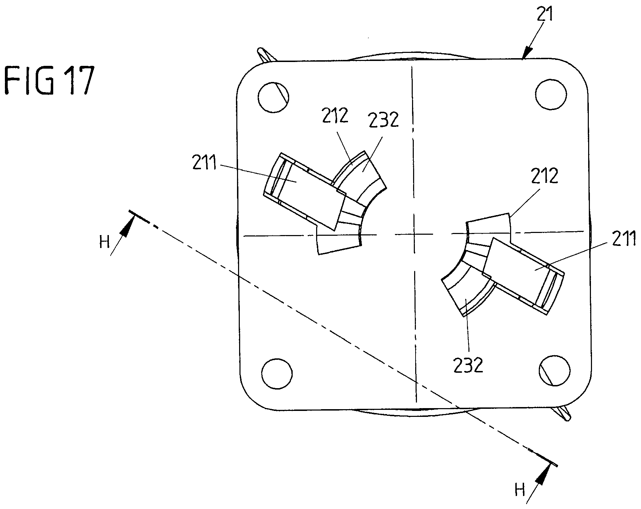

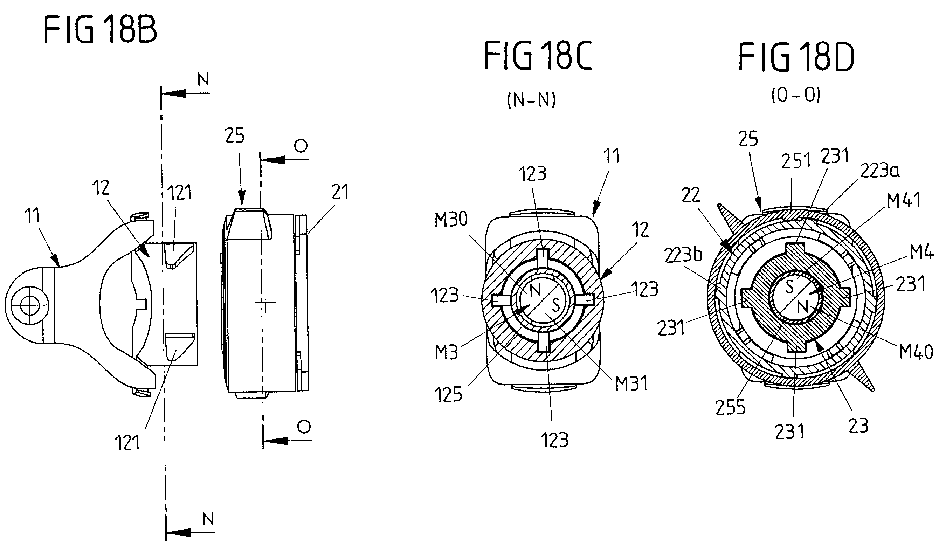

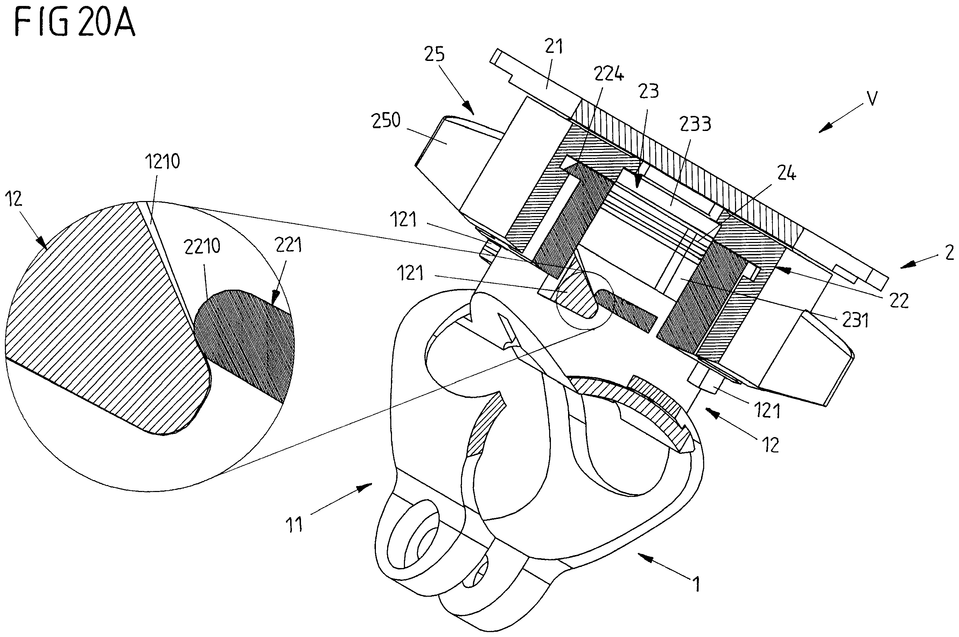

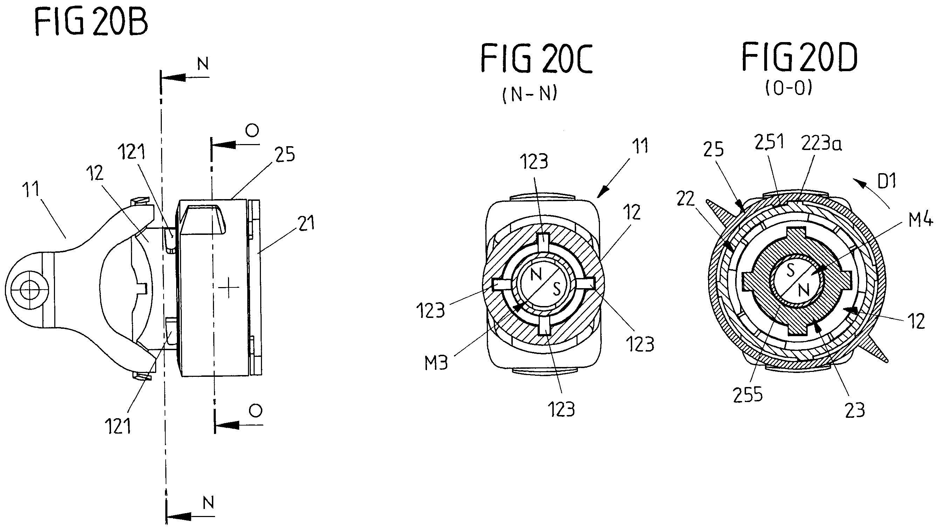

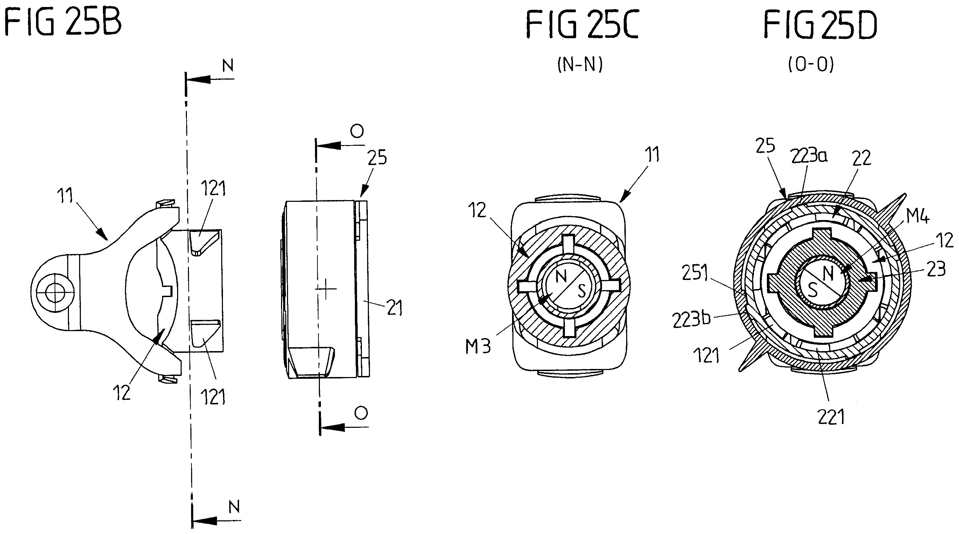

Während mit den Figuren 15A bis 15C und 16A bis 16C jeweils der Aufbau der Verschlussvorrichtung V gemäß der dritten Ausführungsvariante veranschaulicht ist, verdeutlichen die Figuren 17 und 18A bis 18D die Funktion der Verschlussvorrichtung V beim Schließen und Öffnen. Dabei zeigt die Figur 17 in Draufsicht eine Schnittlinie H-H, entlang der anhand der jeweils mit "A" gekennzeichneten nachfolgenden Figuren 18A bis 25A die Verschlussvorrichtung V in unterschiedlichen Phasen beim Schließen und Öffnen dargestellt ist. In den jeweils mit "C" und "D" bezeichneten Figuren 18C/18D bis 25C/25D sind jeweils Querschnittsdarstellungen gezeigt, die sich aus Schnitten entlang von Schnittlinien N-N und O-O der mit "B" gekennzeichneten Figuren 18B bis 25B ergeben. Anhand der Querschnittsdarstellungen ist hierbei insbesondere die sich ändernde Ausrichtung der diametral magnetisierten Magnetelementen M3 und M4 des ersten und zweiten Verschlussteils 1, 2 veranschaulicht sowie die Wechselwirkung zwischen dem zweiten Verbindungselement 22 und dem Betätigungselement 25.
Beim Schließen der Verschlussvorrichtung V, veranschaulicht anhand der Figuren 18A bis 21D, wirken analog zu der Ausführungsvariante der Figuren 5A bis 14D die beiden diametral magnetisierten, scheibenförmigen Magnetelemente M3 und M4 einander anziehend, um die Verschlussteile 1 und 2 einander weiter anzunähern, nachdem das zweite Verschlussteil 2 an das erste Verschlussteil 1 entlang der Verbindungsachse A in Schließrichtung S angesetzt wurde. Die Wirkung der Magnetelemente M3 und M4 ist insoweit funktional identisch zu der zweiten Ausführungsvariante, gleichwohl das Magnetelement M4 des zweiten Verschlussteils 2 nicht in dem zweiten Verbindungselement 22, sondern in dem Betätigungselement 25 angeordnet ist.
Durch die mittels der Magnetelemente M3 und M4 unterstützte oder sogar vollständig selbsttätig ablaufende Annäherung der beiden Verschlussteile 1 und 2 und das Entlanggleiten der Verschlussabschnitte 221 an den Führungsabschnitten 121 wird auch hier dem zweiten Verbindungselement 22 eine Drehbewegung in die erste Drehrichtung D1 um die Verbindungsachse V und die Schließrichtung S aufgezwungen. Hierbei drückt das zweite Verbindungselement 22 über seinen einen Gegenanschlag 223a gegen den Anschlag 251 des Betätigungselements 25 und nimmt auf diese Weise das Betätigungselement 25 in die erste Drehrichtung D1 mit, sodass dieses auch in der ersten Phase des Schließvorgangs in die erste Drehrichtung D1 gedreht wird (vergleiche beispielsweise Figuren 20A bis 20D).

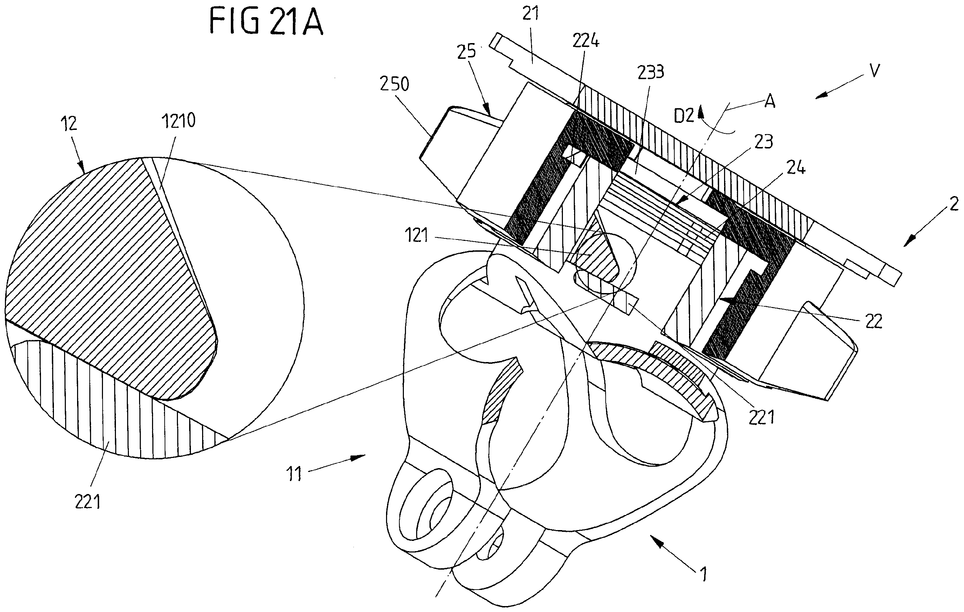

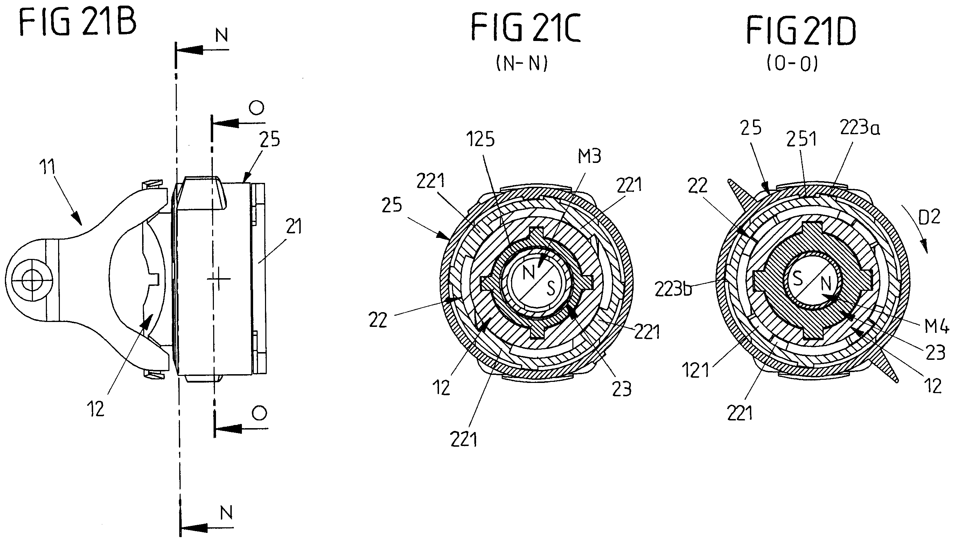

Hat das zweite Verbindungselement 22 relativ zu dem ersten Verbindungselement 12 seine Zwischenstellung erreicht und werden die Verschlussabschnitte 221 an einem Drehen in die entgegengesetzte Drehrichtung D2 durch die Führungsabschnitte 121 nicht mehr gehindert, wird das zweite Verbindungselement 22 unter Wirkung der Federkraft des Federelements 24 selbsttätig in die Schließstellung gedreht. Diese Schließstellung ist in den Figuren 21A bis 21D näher veranschaulicht. Das Federelement 24 wurde hierbei in der vorangegangenen ersten Phase des Schließvorgangs durch das Drehen des zweiten Verbindungselements 22 relativ zu dem drehfest mit dem ersten Verbindungselement 12 verbundenen Verdrehsicherungselement 23 (stärker) gespannt, sodass in der Zwischenstellung des zweiten Verbindungselements 22 eine ausreichend hohe Rückstellkraft für das Drehen des zweiten Verbindungselements 22 in seine Schließstellung zur Verfügung steht.

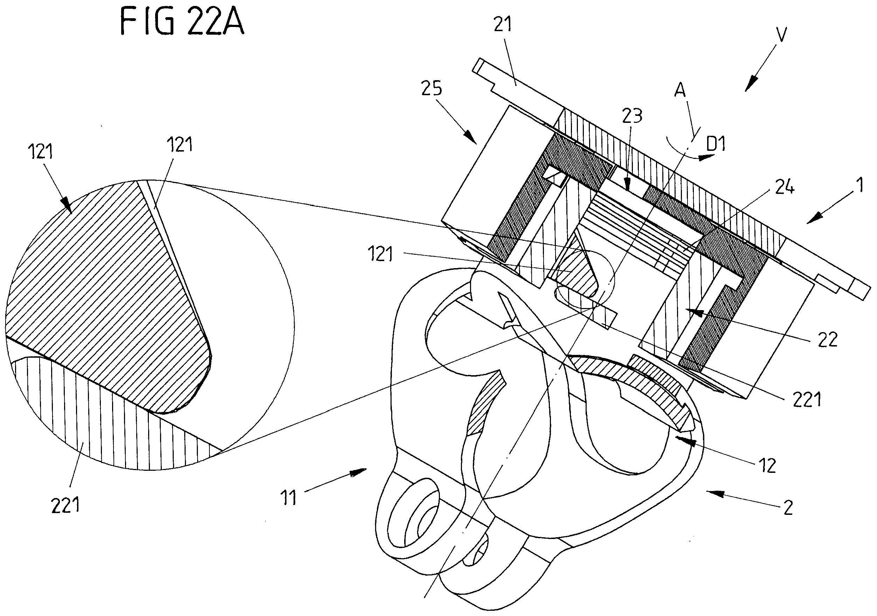

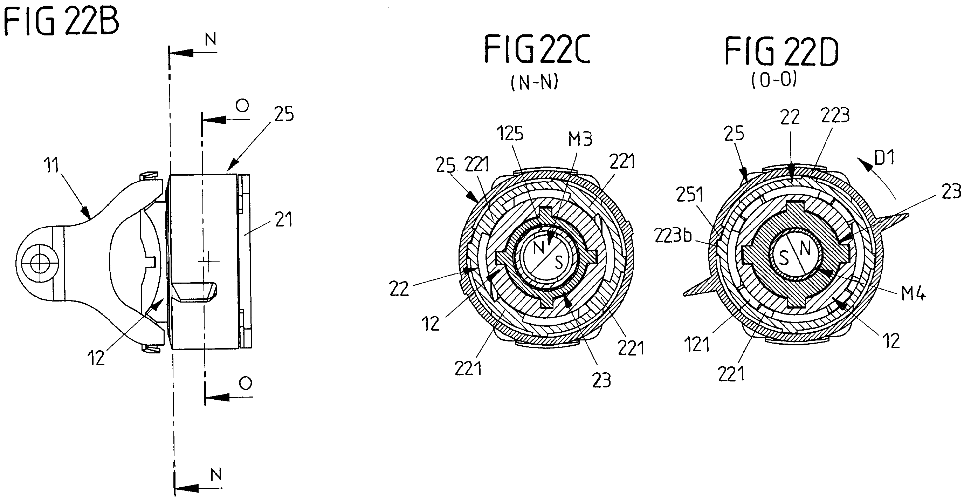

Zum Öffnen der Verschlussvorrichtung V wird zunächst entsprechend den Figuren 22A bis 22D das Betätigungselement 25 in die erste Drehrichtung D1 um die Verbindungsachse A gedreht. Hierbei muss das Betätigungselement 25 zunächst einen über den Abstand der beiden Gegenanschläge 223a und 223b definierten Leerdrehweg überbrücken, in dessen Verlauf das Betätigungselement 25 noch nicht auf das zweite Verbindungselement 22 einwirkt. Erst nachdem das Betätigungselement 25 so weit gedreht wurde - vorliegend um ca. 70° - wirkt es über seinen Anschlag 251 mit dem zweiten Verbindungselement 22 zusammen, indem der Anschlag 251 mit dem Gegenanschlag 223b in Kontakt tritt. Das Betätigungselement 25, das von einem Benutzer zu diesem Zweck an einem Griffbereich 250 ergriffen werden kann, nimmt somit das zweite Verbindungselement 22 erst nach dem Überbrücken des Leerdrehwegs in die erste Drehrichtung D1 mit.

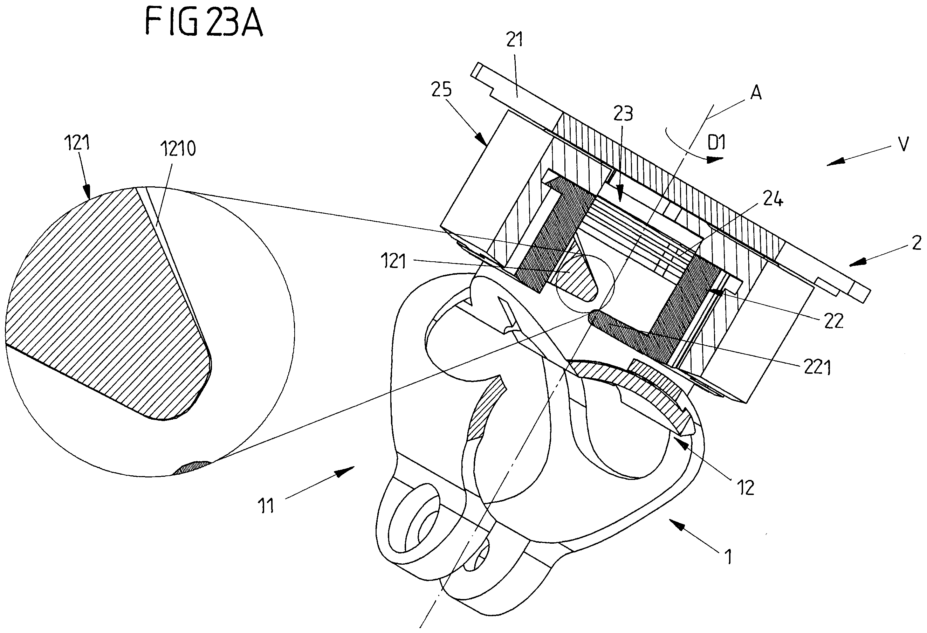

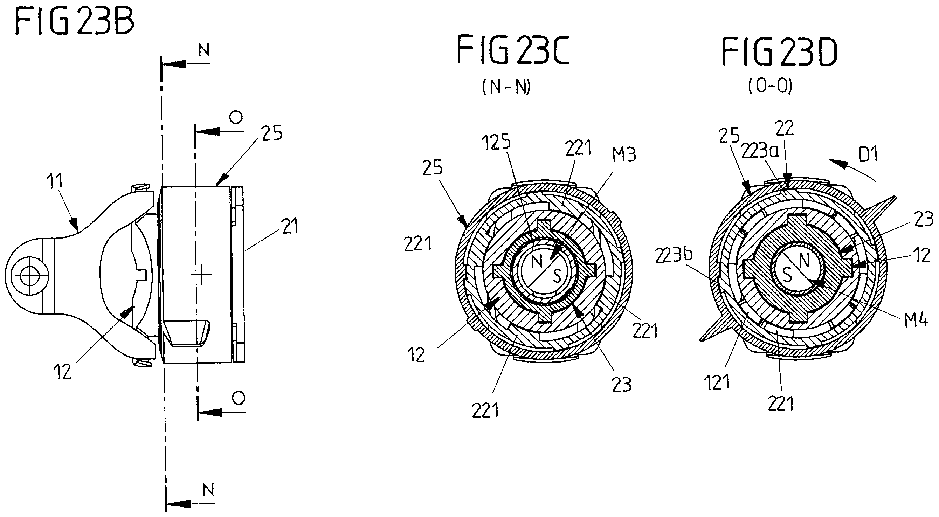

Wird das Betätigungselement 25 in die erste Drehrichtung D1 dann weiter gedreht, erfolgt diese Drehung nicht nur gegen ein mittels der Magnetelemente M3 und M4 aufgebrachtes Drehmoment, sondern auch gegen die Rückstellkraft des Federelements 24, wenn das zweite Verbindungselement 22 mitgedreht wird (vergleiche insbesondere Figuren 23A bis 23D).

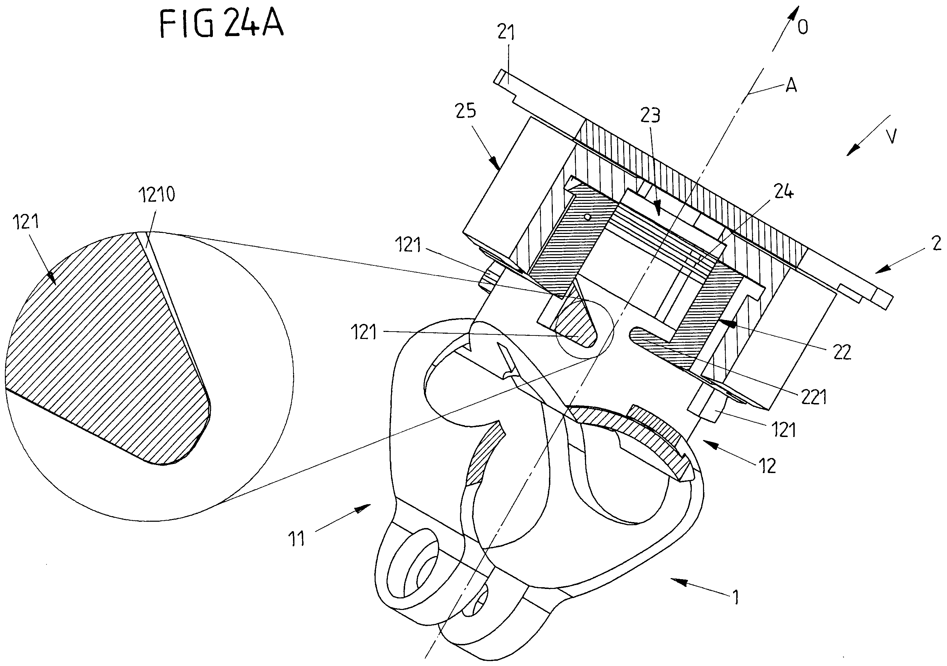

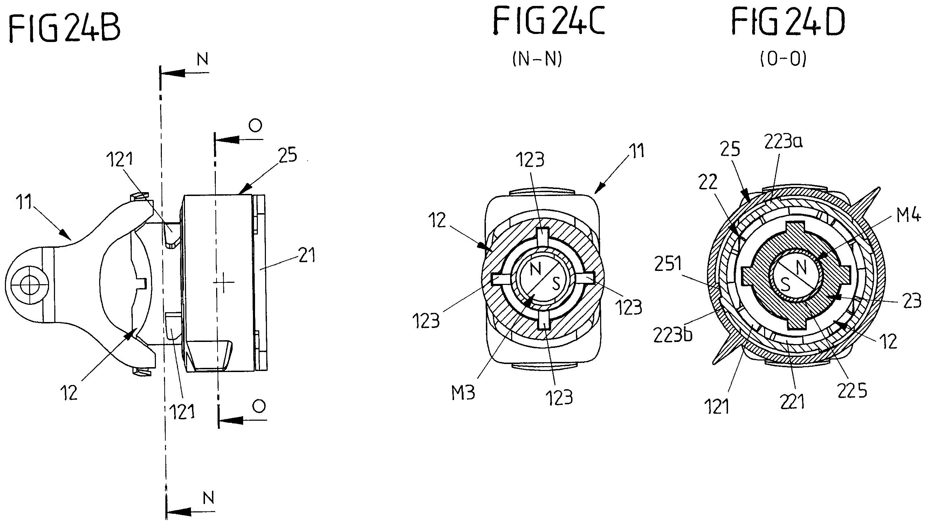

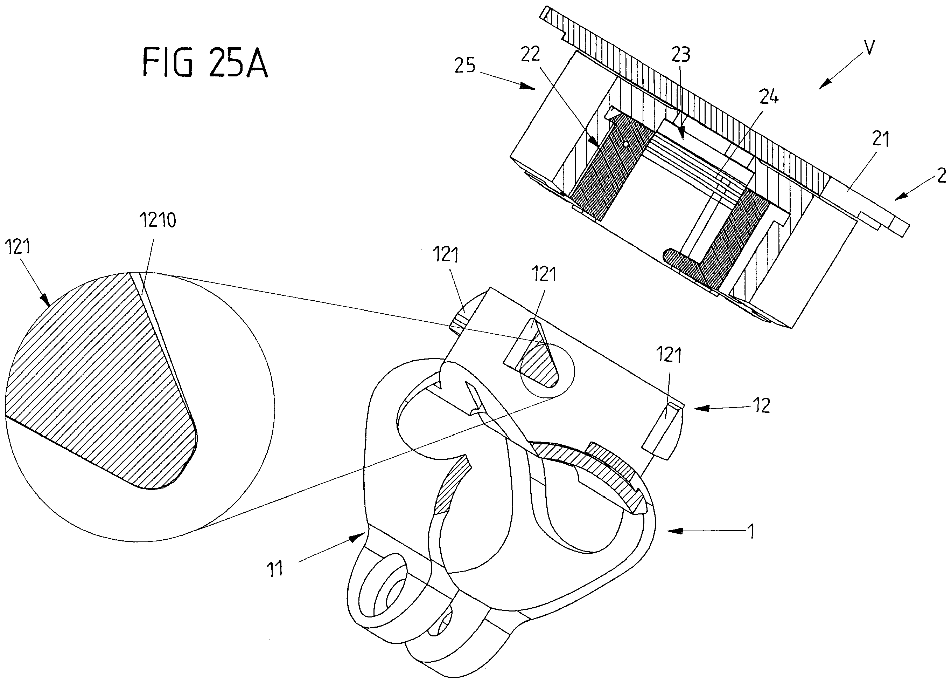

Das erste und zweite Verschlussteil 1 und 2 sind vorliegend so aufgebaut und aufeinander abgestimmt, dass beim Öffnen der Verschlussvorrichtung V ein Drehen des zweiten Verbindungselements 22 relativ zu dem ersten Verbindungselement 12 über die Zwischenstellung hinaus zugelassen ist. Derart kann das Betätigungselement 25 bezüglich des ersten Verbindungselements 12 so weit in die erste Drehrichtung D1 um die Verbindungsachse A gedreht werden, dass die Magnetelemente M3 und M4 der beiden Verschlussteile 1 und 2 in einer solchen Drehlage relativ zueinander vorliegen, dass gleichnamige Magnetpole der beiden Magnetelemente M3 und M4 einander mit größerer Überdeckung derart gegenüberliegen, dass die beiden Magnetelemente M3 und M4 relativ zueinander abstoßend wirken. Durch die Magnetelemente M3 und M4 wird somit das Lösen der beiden Verschlussteile 1 und 2 unterstützt. So wird hierdurch das zweite Verschlusselement 2 von dem ersten Verschlusselement 1 beim Öffnen der Verschlussvorrichtung V in Öffnungsrichtung O abgestoßen, wie dies anhand der Figuren 24A bis 24D und 25A bis 25D veranschaulicht ist.

Die beiden Magnetelemente M3 und M4, die einerseits an dem ersten Verbindungselement 12 und andererseits an dem Betätigungselement 25 angeordnet sind, unterstützen somit sowohl beim Schließen der Verschlussvorrichtung V das Annähern der beiden Verschlussteile 1 und 2 aneinander als auch beim Öffnen der Verschlussvorrichtung V deren Lösen voneinander. Die zur Erzeugung einer Rückstellkraft auf das zweite Verbindungselement 22 in Richtung seiner Schließstellung vorgesehene Verstelleinrichtung ist demgegenüber nicht magnetkraftbasiert, sondern wirkt rein mechanisch federkraftbasiert über das Federelement 24.

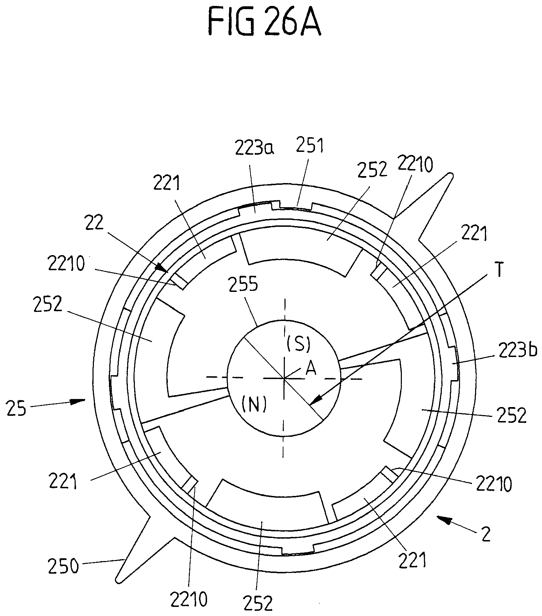

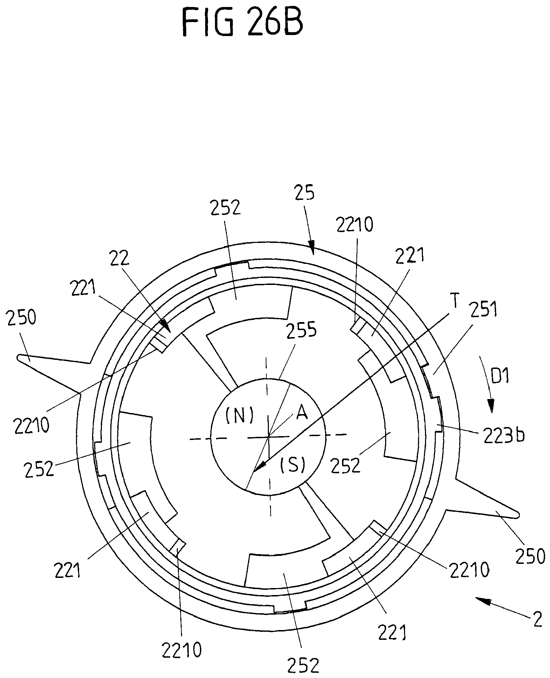

Anhand der Figuren 26A und 26B wird mit Blick auf eine Unterseite des zweiten Verschlussteils 2 im Detail die Relativbewegung des Betätigungselements 25 bezüglich des zweiten Verbindungselements 22 beim Öffnen der Verschlussvorrichtung V veranschaulicht. Hierbei zeigt die Figur 26A die Relativlage des Betätigungselements 25 zu dem zweiten Verbindungselement 22 bei bestimmungsgemäß geschlossener Verschlussvorrichtung V, während die Figur 26B den Zustand gezeigt, bei dem das Betätigungselement 25 bereits um 70° in die erste Drehrichtung D1 gedreht wurde, um den Leerdrehweg zu überbrücken. Aus den beiden genannten Figuren ist insbesondere ersichtlich, wie der zunächst im Bereich des einen Gegenanschlag 223a vorliegende Anschlag 251 des Betätigungselements 25 durch Drehung des Betätigungselements 25 in die erste Drehrichtung D1 in Kontakt mit dem anderen Gegenanschlag 223b gebracht wird, um beim weiteren Drehen des Betätigungselements 25 in diese Drehrichtung D1 das zweite Verbindungselement 22 mitzunehmen. Ferner ist hieraus auch die in der ersten Drehrichtung D1 (hier im Uhrzeigersinn) wandernde Trennlinie T zwischen den Magnetsegmenten M40 und M41 des drehfest an dem Betätigungselement 25 gelagerten Magnetelements M4 in vergrößertem Maßstab veranschaulicht.

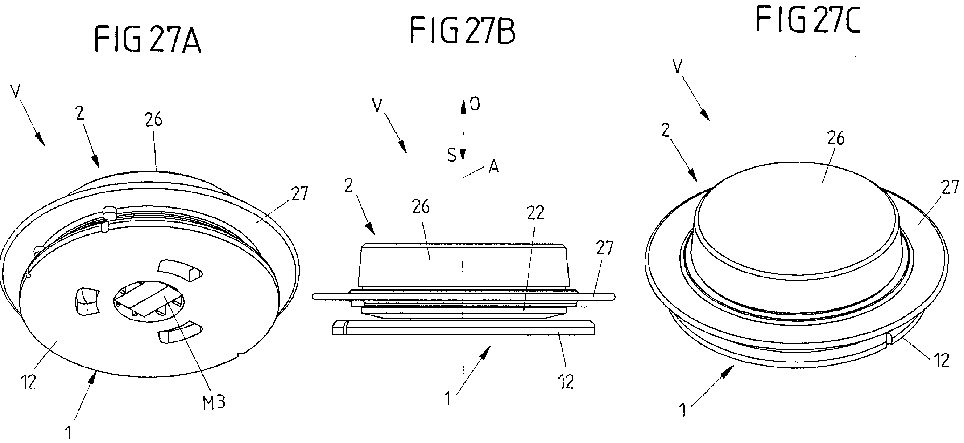

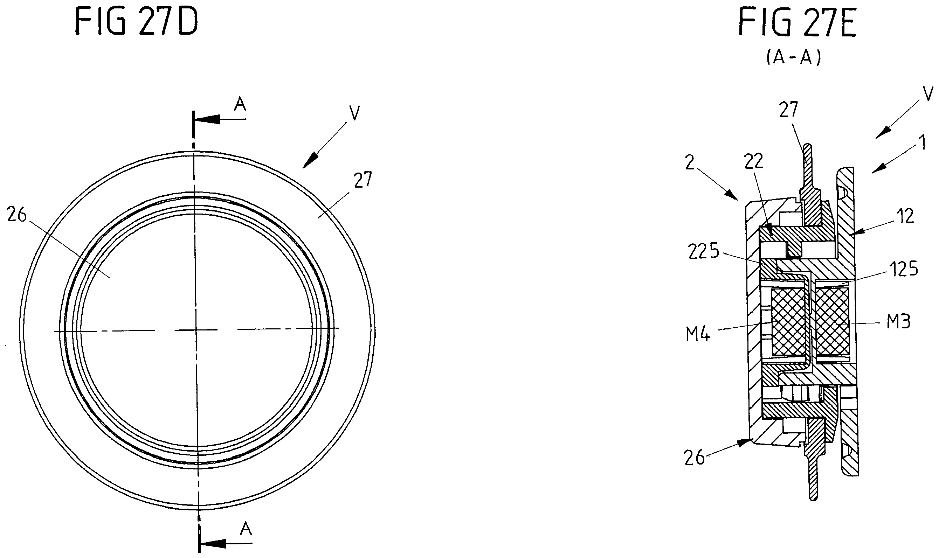

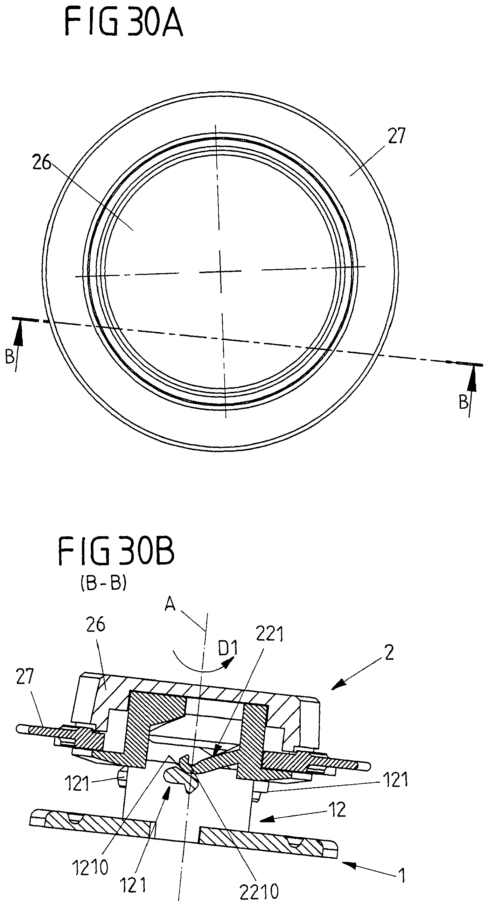

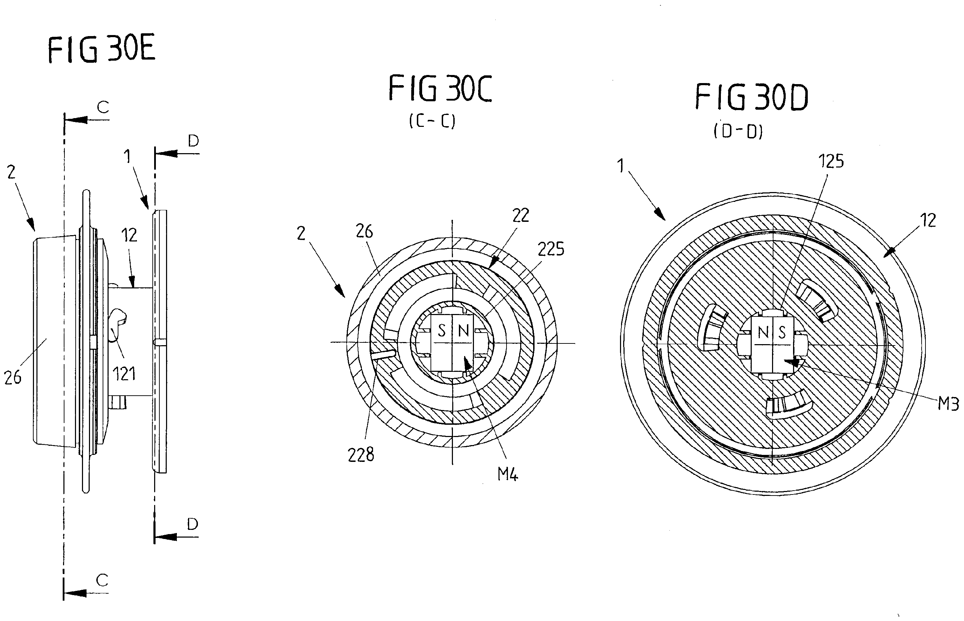

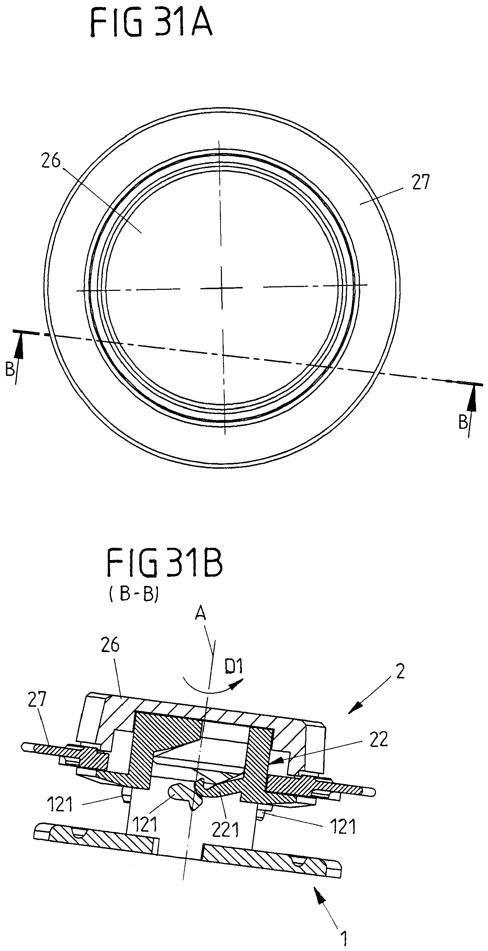

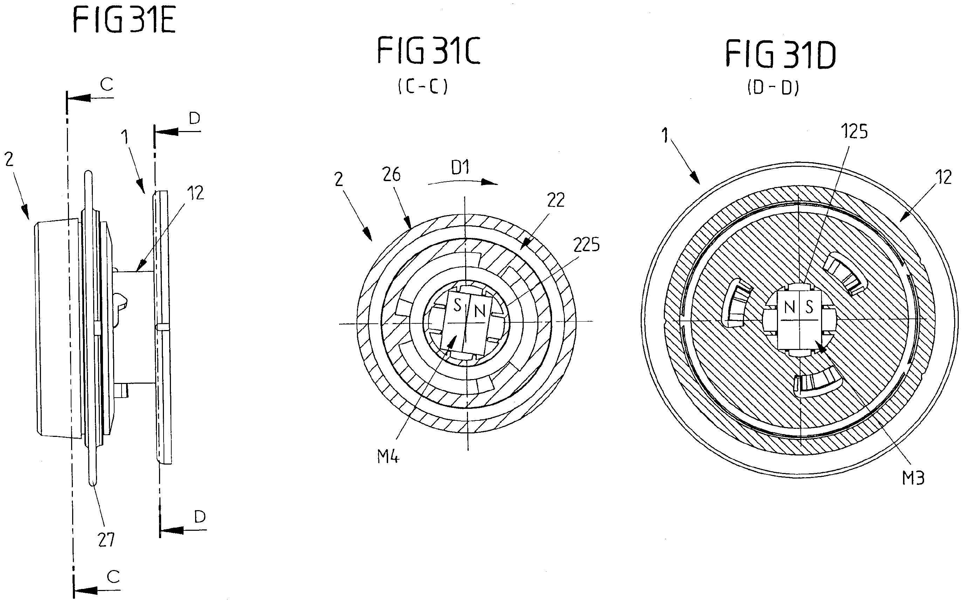

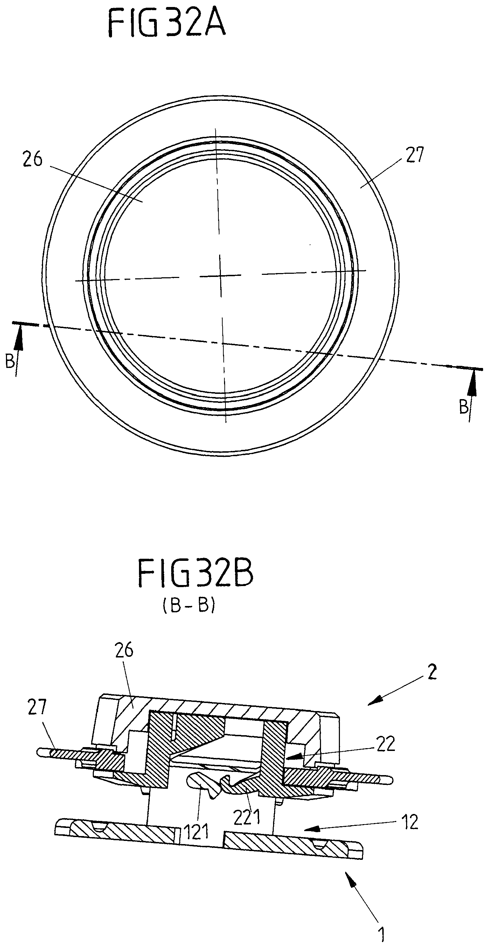

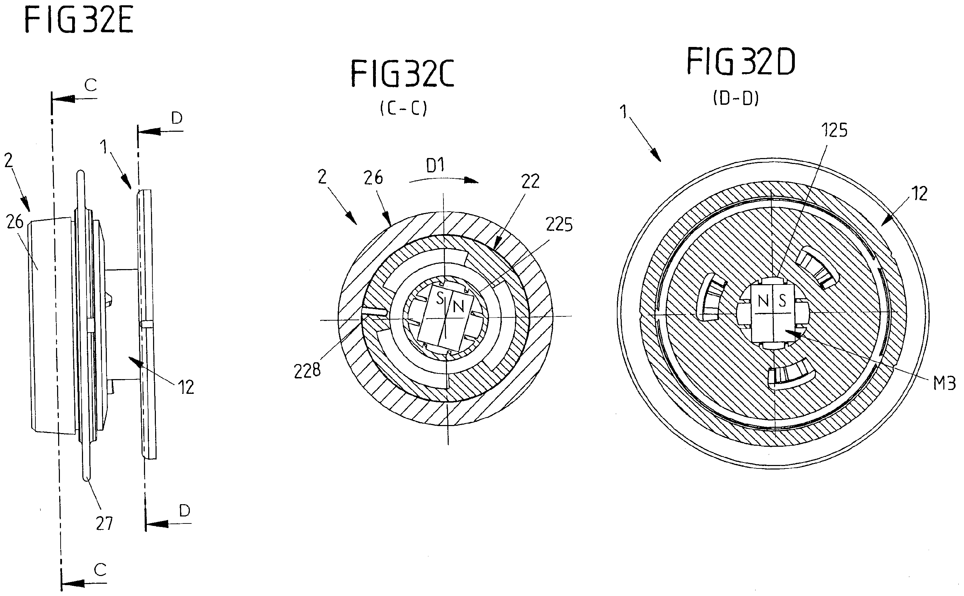

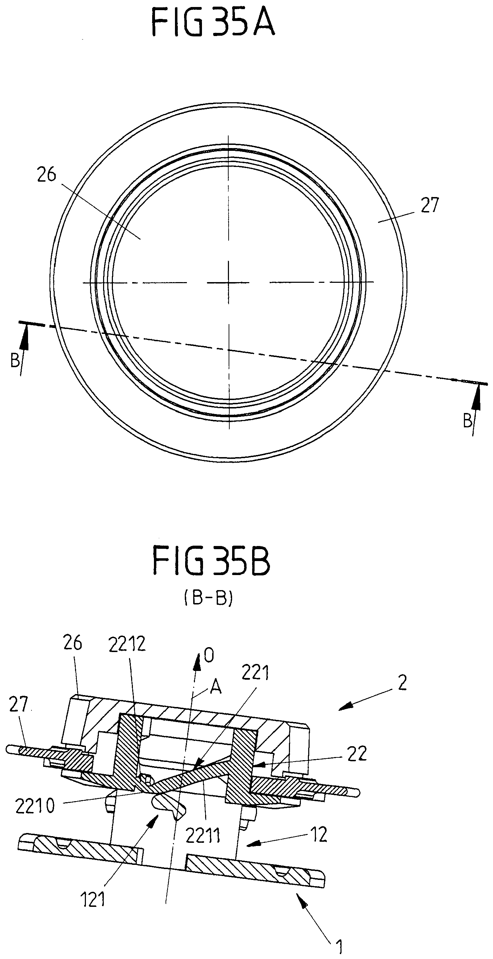

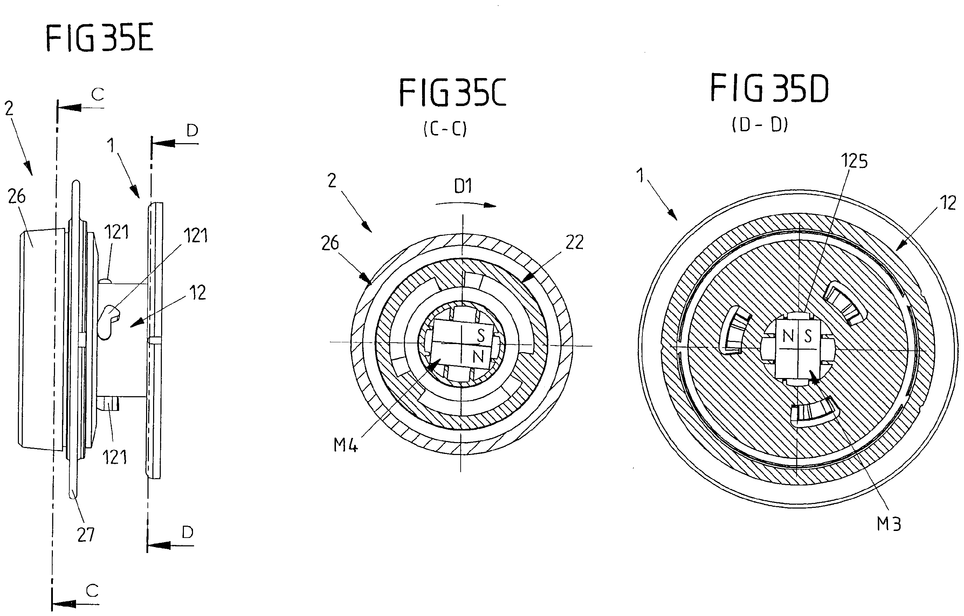

Während sich die zuvor beschriebenen Verschlussvorrichtungen V gemäß den Figuren 1A bis 4G, 5A bis 14D und 15A bis 26B auf Ausführungsvarianten konzentrierten, bei denen die Verschlussteile 1 und 2 mit Befestigungselementen 11 und 21 versehen sind, über die eine Verbindung eines Gegenstandes mit einem rohrförmigen Trägerelement, zum Beispiel einem elektronischen Gerät mit einem Lenker, möglich ist, ist mit den Figuren 27 bis 36D eine Variante einer Verschlussvorrichtung V veranschaulicht, die sich insbesondere für einen Verschluss für ein Kleidungsstück, einen Schuh und/oder eine Prothese Schuh eignet. Diese Verschlussvorrichtung V weist dabei ebenfalls zwei aneinander ansetzbare und miteinander lösbar verbindbare Verschlussteile 1 und 2 auf, die mittels zweier Verbindungselemente 12 und 22 in einem geschlossenen Zustand der Verschlussvorrichtung V aneinander gehalten sind, indem an einem (zweiten) Verbindungselement 22 des zweiten Verschlussteils 2 ausgebildete Verschlussabschnitte 221 an dem anderen (ersten) Verbindungselement 12 des ersten Verschlussteils 1 ausgebildete Führungsabschnitte 121 hintergreifen.

Das erste Verbindungselement 12 des ersten Verschlussteils 1 weist auch in dieser Variante einen hohlzylindrischen Verbindungskörper auf, der im geschlossenen Zustand von dem hohlzylindrischen zweiten Verbindungselement 22 umgeben ist. An einer äußeren Mantelfläche des Verbindungskörpers des ersten Verbindungselements 12 sind die radial nach außen vorstehenden Führungsabschnitte 121 vorgesehen. Diese weisen vorliegend neben einer schräg zur Verbindungsachse A und der Schließrichtung S verlaufenden (ersten) Führungsfläche 1210 eine zusätzliche (zweite) Führungsfläche 1211 auf. Die zusätzliche Führungsfläche 1211 verläuft unter einem Winkel von mehr als 90° - vorliegend etwa 120° - zu der anderen Führungsfläche 1210 ebenfalls geneigt zur Verbindungsachse V und in Schließrichtung S abfallend. Je nachdem, ob die Verschlussvorrichtung V geschlossen oder geöffnet wird, stützt sich das zweite Verbindungselement 22 an der einen oder der anderen Führungsfläche 1210, 1211 beim ab und ist hierüber geführt. Dies wird im Folgenden noch näher erläutert werden.

Zur Erzeugung einer beim Schließen der Verschlussvorrichtung V die beiden Verschlussteile 1 und 2 einander anziehenden Magnetkraft sind erneut Magnetelemente M3 und M4 mit zwei Magnetsegmenten vorgesehen, die durch eine quer zur Schließrichtung S verlaufende Trennlinie zueinander getrennte, ungleichnamige Pole aufweisen. Die Magnetelemente M3 und M4 sind hierbei jeweils quaderförmig ausgebildet und einerseits in einem Lagerabschnitt 125 des ersten Verbindungselements 12 und andererseits in einem Lagerabschnitt 225 des zweiten Verbindungselements 22 formschlüssig aufgenommen. Der mittig an dem zweiten Verbindungselement 22 ausgebildete Lagerabschnitt 225 für das Magnetelement M4 ist dabei ringsum von einer inneren Mantelfläche des zweiten Verbindungselements 22 umgeben, an der die sich jeweils zumindest teilweise entlang einer Schraubenlinie längs erstreckten Verschlussabschnitte 221 ausgebildet sind.

An einer äußeren Mantelfläche des zweiten Verbindungselements 22 ist ein Anschlag 227 ausgebildet. Dieser radial nach außen vorstehende Anschlag 227 ist im zusammengebauten Zustand des zweiten Verschlussteils 2 in einer zwischen zwei Gegenanschlägen 271a und 271b kreisbogenförmig verlaufenden Aussparung eines Anschlagrings 27 des zweiten Verschlussteils 2 aufgenommen. Der Anschlagring 27 dient dabei als Befestigungselement, über das das zweite Verschlussteil 2 an einem Gegenstand, beispielsweise an einer Verschlusslasche eines Kleidungsstücks, eines Schuhs oder einer Prothese, fixiert ist. Das zweite Verbindungselement 22 ist an diesem Anschlagring 27 um die Verbindungsachse A drehbar gelagert.

Über die Gegenanschläge 271A und 271B ist dabei an dem Anschlagring 27 eine Stellwegbegrenzung realisiert, sodass das zweite Verbindungselement 22 lediglich in einem definierten Drehwinkelbereich von unter 180°, vorliegend unter 150°, z.B. im Bereich von etwa 120°, relativ zu dem Anschlagring 27 drehbar ist. Hierdurch wird sichergestellt, dass die beiden Magnetelemente M3 und M4 der beiden Verschlussteile 1 und 2 nicht in einer unerwünschten Relativlage zueinander ausgerichtet werden können, in der beispielsweise die beiden Magnetelemente M3 und M4 beim Schließen der Verschlussvorrichtung V einander abstoßend wirken. Durch die Stellwegbegrenzung an dem Anschlagring 27 ist demgegenüber sichergestellt, dass das zweite Verbindungselement 22 und damit dessen Magnetelement M4 beim Schließen der Verschlussvorrichtung V in einer Relativlage bezüglich der Verbindungsachse A zu dem ersten Verbindungselement 12 und dessen Magnetelement M3 vorliegt, in der die beiden Magnetelemente M3 und M4 einander anziehend wirken. So können insbesondere bei diesem Ausführungsbeispiel mit einer magnetkraftbasierten Verstelleinrichtung auf Basis der jeweils diametral magnetisierten Magnetelemente M3 und M4 die Verbindungselemente 12 und 22 grundsätzlich in beide möglichen Drehrichtungen D1 und D2 mit einem Rückstellmoment in Richtung einer Schließstellung beaufschlagt werden, und zwar in Abhängigkeit von der Relativlage der Magnetelemente M3 und M4 und damit in Abhängigkeit von der Relativlage der Verbindungselemente 12 und 22 zueinander. Dies gestattet, z.B. im Vergleich mit einer federkraftbasierten Verstelleinrichtung, eine sehr kompakte Gestaltung der Verschlussvorrichtung V sowie einer anwendungsabhängigen Wahl der Drehrichtungen für das Schließen und Öffnen, da durch die Verstelleinrichtung an sich eine Drehbarkeit in beide Drehrichtungen nicht eingeschränkt ist.

Das zweite Verschlussteil 2 umfasst ferner noch ein Drehelement 26, das zur Fixierung des zweiten Verbindungselements 22 an dem Anschlagring 27 vorgesehen ist und an dem ein Nutzer angreifen kann um durch Betätigen des Drehelements 26 die Verschlussvorrichtung V zu öffnen. Hierfür ist der Anschlagring 27 zwischen dem deckelartig ausgestalteten Drehelement 26 und dem zweiten Verbindungselement 22 angeordnet. Der hohlzylindrischen Verbindungskörper des zweiten Verbindungselements mit dem Lagerabschnitt 225 für das Magnetelement M3 erstreckt sich hierbei durch die zentrale Öffnung des Anschlagrings 27 hindurch und ist mit dem Drehelement 26 formschlüssig verbunden.

Für die formschlüssige Verbindung zwischen dem zweiten Verbindungselement 22 und dem Drehelement 26 ist an dem Verbindungskörper des zweiten Verbindungselements 22 ein Steckschlitz 228 vorgesehen. Dieser Steckschlitz liegt an einem radial äußeren Bereich des Verbindungskörpers vor und erstreckt sich in eine Außenwand des Verbindungskörpers sowohl in radialer als auch in axialer Richtung hinein. An dem Drehelement 26 ist wiederum ein radial nach innen vorstehender Verbindungssteg 260 vorgesehen, der in diesen Steckschlitz 228 des zweiten Verbindungselements formschlüssig eingesteckt ist, um das Drehelement 26 drehfest mit dem zweiten Verbindungselement 22 zu verbinden.

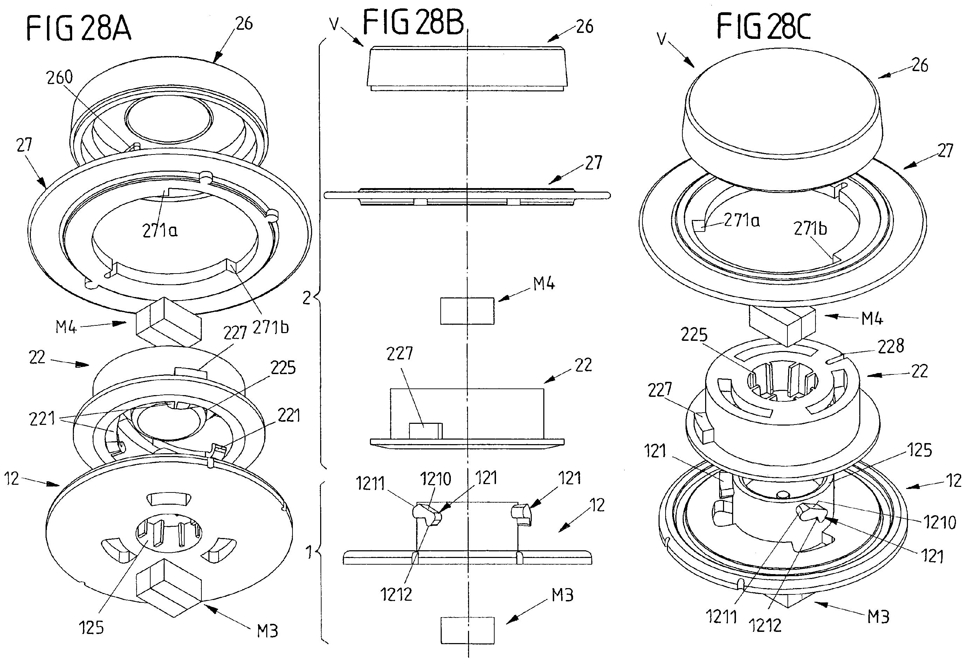

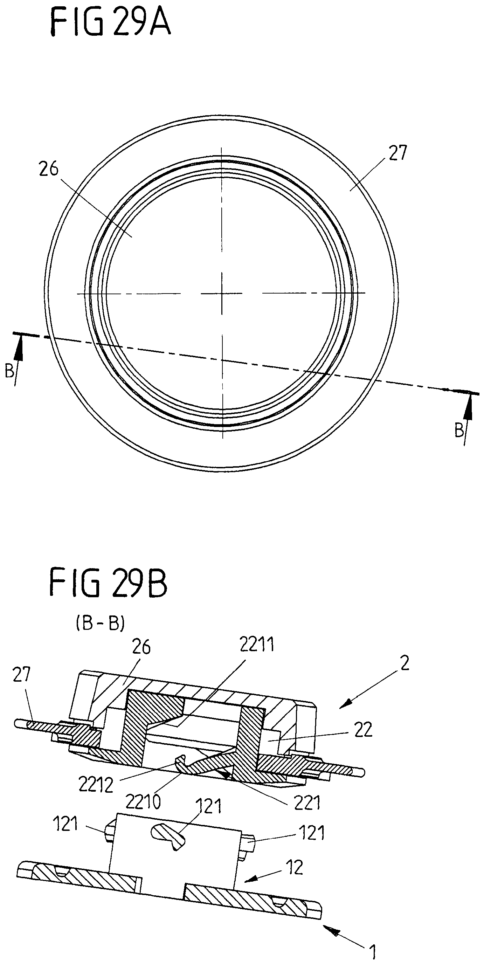

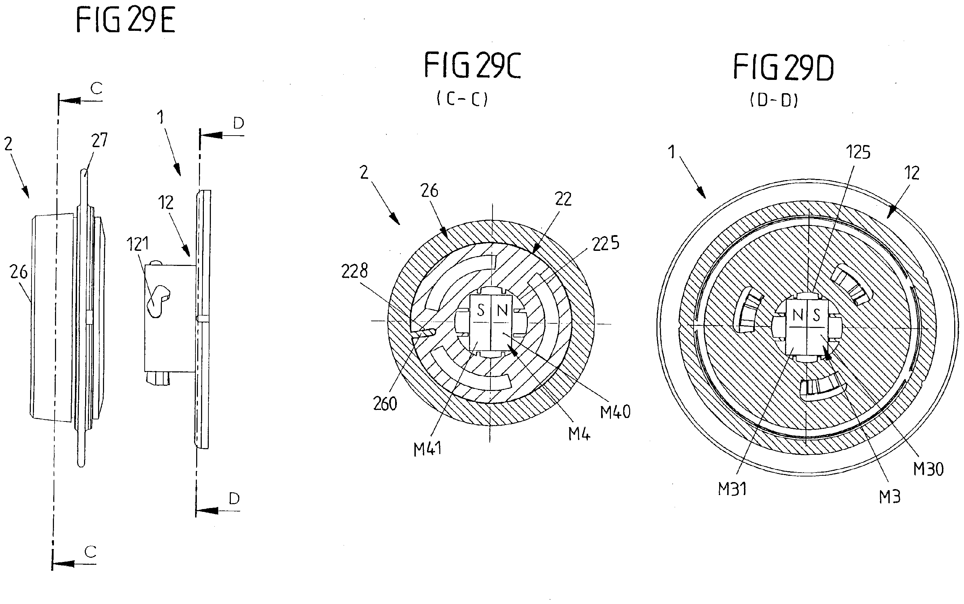

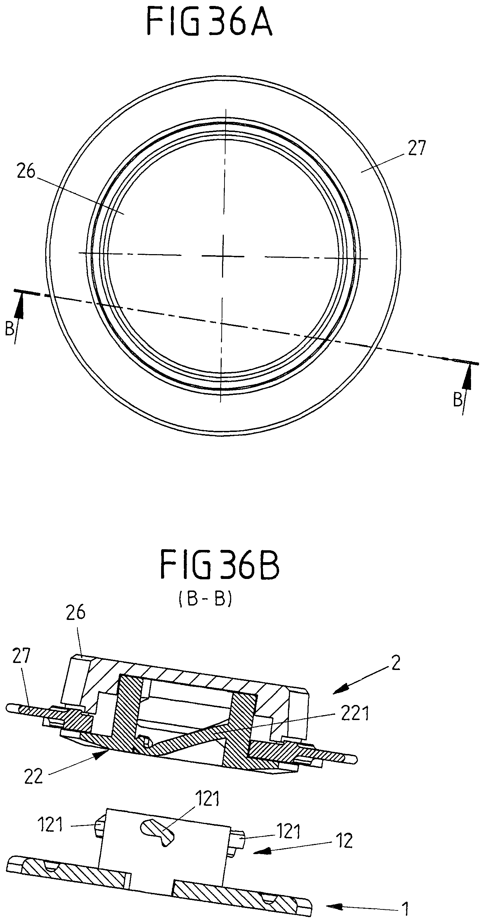

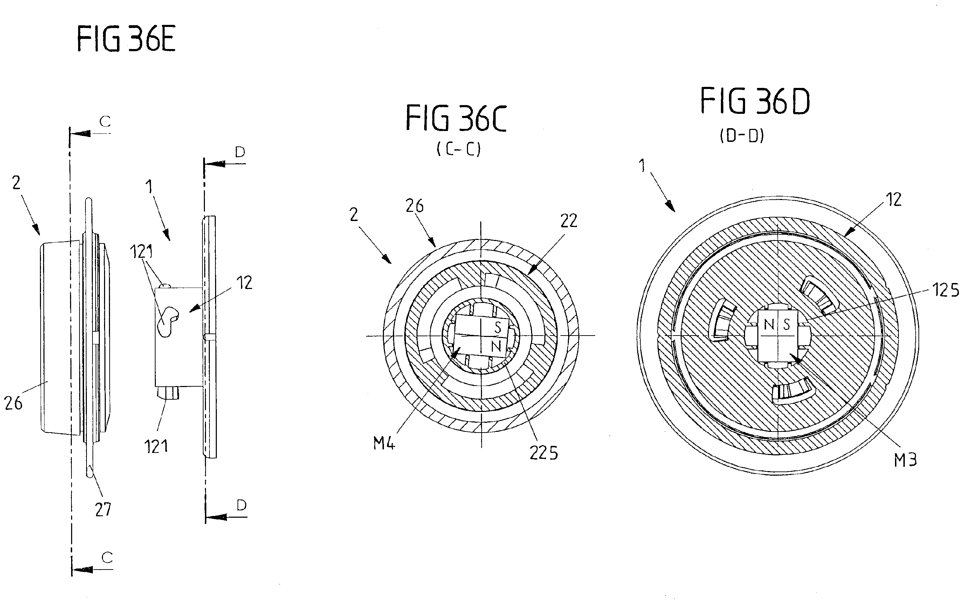

Während die unterschiedlichen Ansichten der Figuren 27A bis 27C und 28A bis 28C den zuvor geschilderten Aufbau der Verschlussvorrichtung V verdeutlichen, veranschaulichen die Figuren 29A bis 36E die Funktion der Verschlussvorrichtung V anhand von Darstellungen zu unterschiedlichen Phasen während des Schließens und Öffnens der Verschlussvorrichtung V. Hierbei zeigt eine jeweils mit "A" gekennzeichnete Figur 29A bis 36A eine Draufsicht auf die Verschlussvorrichtung V unter Darstellung einer Schnittlinie B-B. Entlang dieser Schnittlinie B-B ist die längsgeschnittene Schnittdarstellung der jeweils mit "B" gekennzeichneten Figuren 29B bis 36B gewonnen. Die mit "C" und "D" gekennzeichneten Figuren 29C/29D bis 36C/36D zeigen jeweils Querschnittsdarstellungen entlang der Schnittlinien C-C und D-D der jeweils zugehörigen, mit "E" gekennzeichneten Figur 29E bis 36E.

Wie anhand der Figuren 29A bis 32E veranschaulicht ist, wird dem zweiten Verbindungselement 22 nach dem Ansetzen des zweiten Verschlussteils 2 an das erste Verschlussteil 1 durch das Aufgleiten der Verschlussabschnitte 221 an jeweils einem Führungsabschnitt 121 eine Drehbewegung in die erste Drehrichtung D1 um die Verbindungsachse A aufgezwungen, wenn das zweite Verschlussteil 2 entlang der Schließrichtung S dem ersten Verschlussteil 1 weiter angenähert wird. Ein vorliegend hakenförmig ausgestaltetes Ende des jeweiligen Verschlussabschnitt 221 gleitet dabei mit einer an einer Unterseite ausgebildeten Gleitfläche 2210 an der geneigt verlaufenden (ersten) Führungsfläche 1210 eines Führungsabschnitts 121 entlang.

Wurde das Verbindungselement 22 relativ zu dem ersten Verbindungselement 12 in der ersten Drehrichtung D1 ausreichend weit verlagert, sodass ein Ende des jeweiligen Verschlussabschnitt 221 in Schließrichtung S an dem zugeordneten Führungsabschnitt 121 vorbeiführbar ist, nimmt das zweite Verbindungselement 22 eine Zwischenstellung relativ zu dem ersten Verbindungselement 12 ein. Aufgrund der nicht parallel zueinander ausgerichteten Trennlinien der beiden quer zur Schließrichtung S magnetisierten Magnetelemente M3 und M4 wirkt nun aber in dieser Zwischenstellung eine Magnetkraft, infolge derer das zweite Verbindungselement 22 bestrebt ist, eine Relativlage zu dem ersten Verbindungselement 12 einzunehmen, bei der jeder Führungsabschnitt 121 von einem Verschlussabschnitt 221 zumindest teilweise hintergriffen ist. Über die Magnetelemente M3 und M4 ist somit eine Verstelleinrichtung bereitgestellt, mittels der das zweite Verbindungselement 22 selbsttätig aus seiner Zwischenstellung in eine Schließstellung um die Verbindungsachse A in die zu der ersten Drehrichtung D1 entgegengesetzte zweite Drehrichtung D2 gedreht wird. In den zwei aufeinander folgenden Phasen eines Schließvorgangs beim Schließen der Verschlussvorrichtung V erfolgt somit ein Wechsel der Drehrichtungen, entlang derer das zweite Verbindungselement 22 eine Drehbewegung relativ zu dem ersten Verbindungselement 12 ausgeführt, um bestimmungsgemäß an dem ersten Verbindungselement 12 gehalten zu werden.

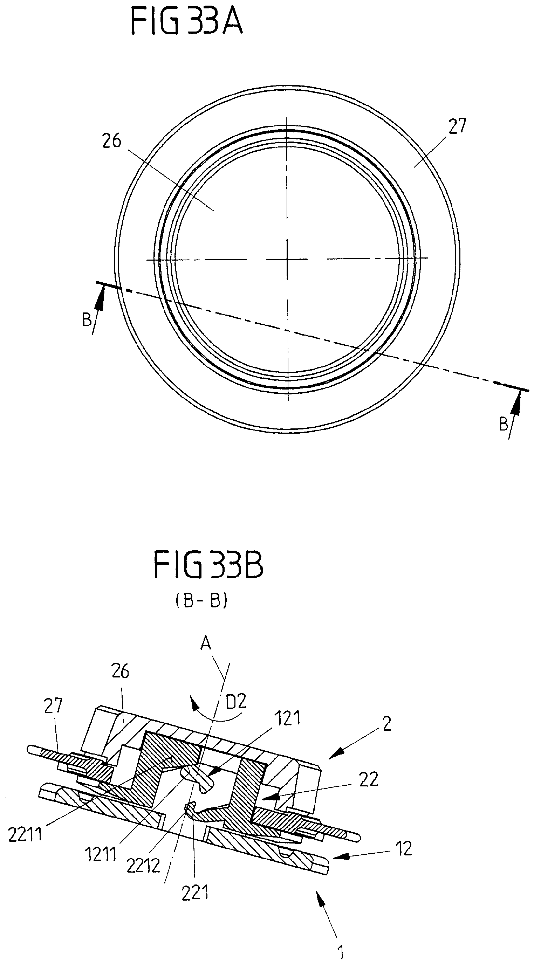

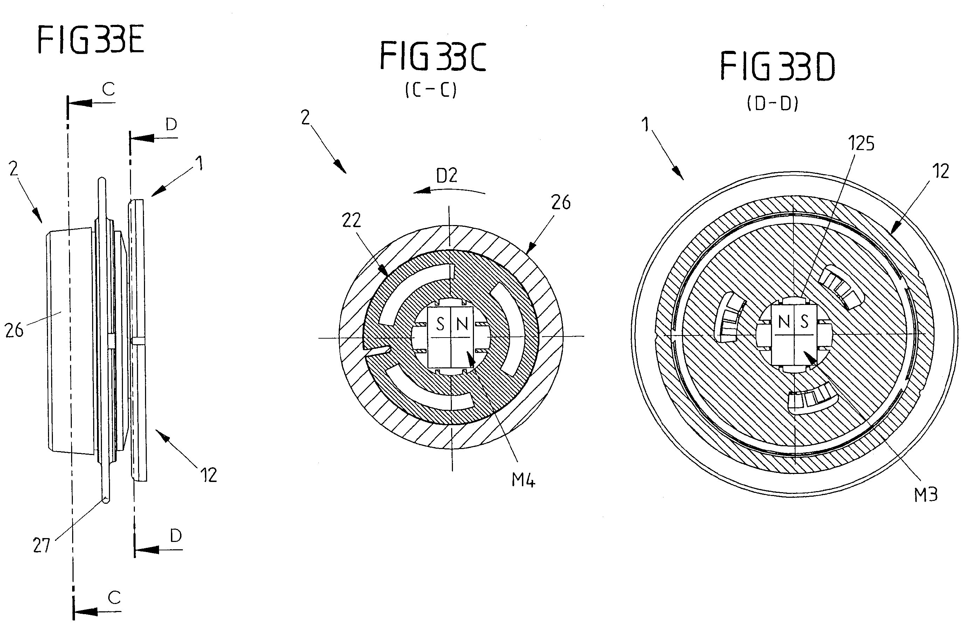

In einem bestimmungsgemäß geschlossenen Zustand der Verschlussvorrichtung V, der anhand der Figuren 33A bis 33D veranschaulicht ist, liegen die einzelnen Verschlussabschnitte 221 des zweiten Verbindungselements jeweils in axialer Richtung (bezogen auf die Verbindungsachse A) beabstandet zu einem Führungsabschnitt 121 vor. Derart sind die beiden Verbindungselemente 12 und 22 und damit die Verschlussteile 1 und 2 um ein definiertes axiales Spiel verlagerbar, das durch den axialen Abstand zwischen einem Führungsabschnitt 121 und einem Verschlussabschnitt 221 definiert ist.

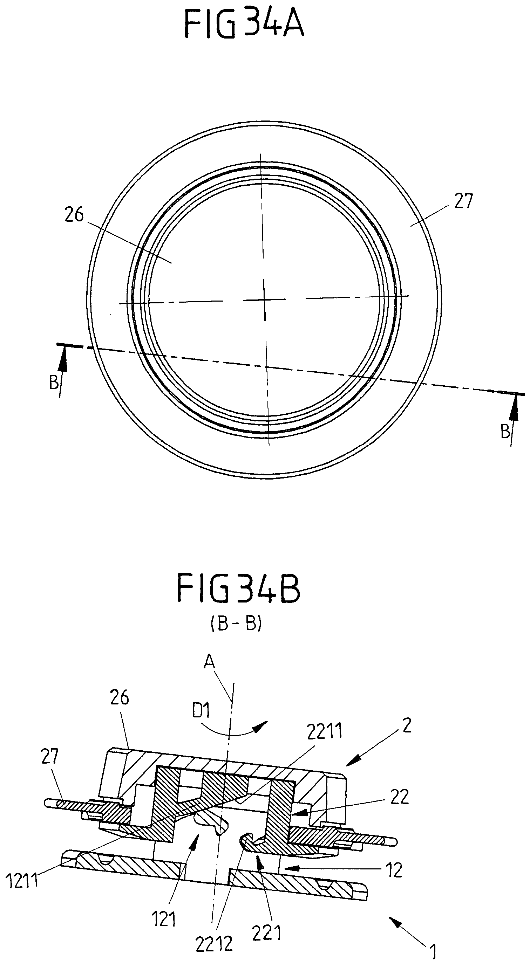

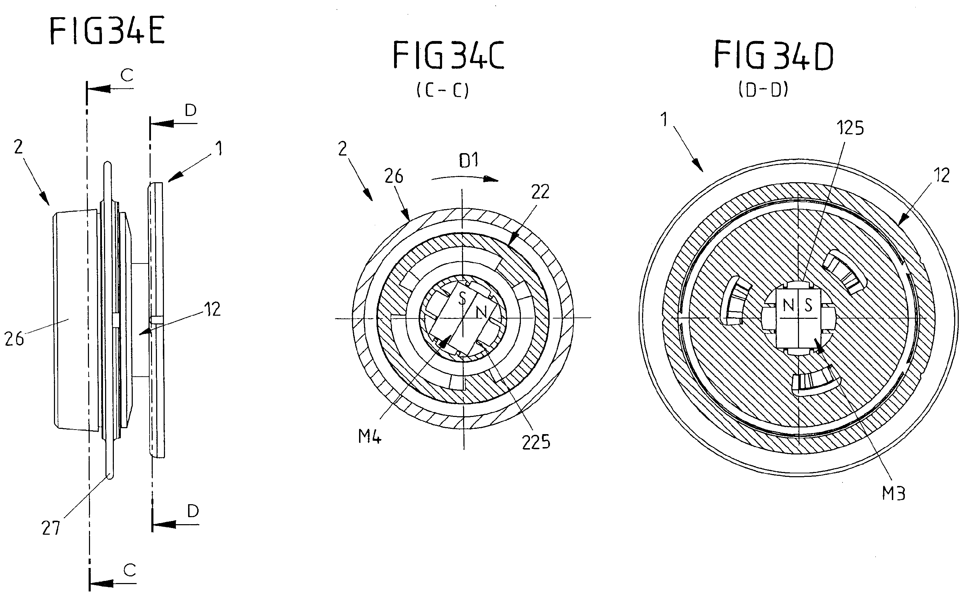

Um hierbei dennoch eine axiale Sicherung der beiden Verschlussteile 1, 2 aneinander sicherzustellen, sodass die beiden Verschlussteile 1, 2 entlang der Verbindungsachse A nicht ohne Weiteres voneinander getrennt werden können, weist jeder Führungsabschnitt 121 an einem in der Drehrichtung D1 liegenden Ende einen (ersten) hakenförmigen Sperrteil 1212 auf. Hierzu komplementär ist an einem in der zweiten Drehrichtung D2 liegenden Ende jedes Verschlussabschnitts 221 ein (zweiter) hakenförmigen Sperrteil 2212 ausgebildet. Wird die bestimmungsgemäß geschlossene Verschlussvorrichtung V entlang der Verbindungsachse A mit einer resultierenden Kraft belastet, über die die beiden Verschlussteile 1 und 2 voneinander entfernt werden sollen, können sich die beiden Verschlussteile 1 und 2 zwar um das definierte Spiel axial verlagern. Hierdurch kommen aber die beiden Sperrteile 1212 und 2212 der beiden Verbindungselemente 12 und 22 miteinander in formschlüssigen Eingriff. Durch diesen formschlüssigen Eingriff sind die beiden Verbindungselemente 12 und 22 nicht mehr relativ zueinander drehbar. Erst nach einer entsprechenden Entlastung und einer damit verbundenen erneuten Annäherung der beiden Verschlussteile 1 und 2 gelangen die beiden hakenförmigen Sperrteile 1212 und 2212 wieder außer Eingriff und das zweite Verschlussteil 2 kann in die erste Drehrichtung D1 um die Verbindungsachse A relativ zu dem ersten Verbindungselement 12 gedreht werden, sodass die Verschlussabschnitte 221 die Führungsabschnitte 121 nicht mehr hintergreifen und das zweite Verschlussteil 2 entlang der Verbindungsachse A in die Öffnungsrichtung O von dem ersten Verschlussteil 1 abgezogen werden kann. Dies ist anhand der Figuren 34A bis 36E näher veranschaulicht.

Wie hierbei insbesondere mit Blick auf die Figur 34B veranschaulicht ist, gleitet während des Öffnens der Verschlussvorrichtung V bei einer Drehung des zweiten Verbindungselements 22 in die erste Drehrichtung D1 ein Verschlussabschnitt 221 über eine Gleitfläche 2212 an der zusätzlichen (zweiten) Führungsfläche 1212 eines Führungsabschnitts 121 entlang. Der Führungsabschnitt 121 weist hier also zwei jeweils geneigt verlaufende Führungsflächen 1210 und 1211 auf, die zwar bei Drehung des zweiten Verbindungselements 22 in dieselbe Drehrichtung D1 bezüglich des ersten Verbindungselements 12 eine körperliche Führung für das zweite Verbindungselement 22 bereitstellen, wobei jedoch in Abhängigkeit davon, ob die Verschlussvorrichtung V geschlossen oder geöffnet wird, nur die eine oder die andere Führungsfläche 1210 oder 1211 die Führung bereitstellt. Dabei liegt beim Schließen der Verschlussvorrichtung derjenige Verschlussabschnitt 221 an der einen (ersten) Führungsfläche 1210 an, der im geschlossenen Zustand der Verschlussvorrichtung V den zugehörigen Führungsabschnitt 121 teilweise hintergreift. Beim Öffnen der Verschlussvorrichtung V liegt an der anderen (zweiten) Führungsfläche 1211 des Führungsabschnitts 121 nicht dieser Verschlussabschnitt 221, sondern ein benachbarter Verschlussabschnitt 221 mit einer Gleitfläche 2211 an.

Um beim Schließen der Verschlussvorrichtung V ein selbsttätiges Eindrehen des zweiten Verbindungselements 22 relativ zu dem ersten Verbindungselement 12 zu unterstützen und beim Öffnen der Verschlussvorrichtung V ein leichtgängiges Aufdrehen für einen Nutzer zu ermöglichen, ist jede Gleitfläche 2210, 2211 um mehr als 60° zur Verbindungsachse A geneigt ausgeführt, vorliegen um etwa 70°.

Bei der Verschlussvorrichtung V der Figuren 27A bis 36E ist ferner beim Drehen des zweiten Verbindungselements 22 aus seiner Schließstellung in der erste Drehrichtung D1 eine Verstellung über die Zwischenstellung hinaus zugelassen. Derart können die beiden Magnetelemente M3 und M4 beim Öffnen der Verschlussvorrichtung V in eine Relativlage zueinander gebracht werden, in der sie einander abstoßend wirken und damit das Trennen der beiden Verschlussteile 1 und 2 voneinander unterstützen.

### Bezugszeichenliste

- **1**: Erste Verschlussteil
- **11**: Erstes Befestigungselement
- 110: Lageröffnung
- **12**: Verbindungselement
- 121: Führungsabschnitt
- 1210: Führungsfläche
- 1211: Zusätzliche (zweite) Führungsfläche
- 1212: Erster Sperrteil
- 122a, 122b: Fixierungslasche
- 123: Ausnehmung (erster Verdrehsicherungsabschnitt)
- 124: Halteabschnitt
- 125: Lagerabschnitt
- **2**: Zweite Verschlussteil
- **21**: Zweites Befestigungselement
- 211: Rastlasche
- 212: Bajonettöffnung
- **22**: Zweites Verbindungselement
- 220: Griffbereich
- 221: Verschlussabschnitt
- 2210: Gleitfläche
- 2211: Gleitfläche
- 2212: Zweiter Sperrteil
- 222: Lagersteg
- 223a, 223b: Gegenanschlag
- 224: Vorsprung
- 225: Lagerabschnitt
- 227: Anschlag
- 228: Steckschlitz
- **23**: Verdrehsicherungselement
- 231: Formschlusselement (zweiter Verdrehsicherungsabschnitt)
- 232: Verschlusshaken
- 233: Befestigungskragen
- 234: Federlagerbereich
- 235: Lagerabschnitt
- **24**: Federelement
- 240: Federende
- **25**: Betätigungselement
- 250: Griffbereich
- 251: Anschlag
- 252: Lagersteg
- 255: Lagerabschnitt
- **26**: Drehelement
- 260: Verbindungssteg
- **27**: Anschlagring / Befestigungselement
- 271a, 271b: Gegenanschlag
- A: Verbindungsachse
- D1, D2: Drehrichtung
- M1, M2, M3, M4: Magnetelement
- M30, M31, M40, M41: Magnetsegment
- O: Öffnungsrichtung
- S: Schließrichtung
- T: Trennlinie
- V: Verschlussvorrichtung

## Patentansprüche

1. Verschlussvorrichtung, mit wenigstens einem ersten und einem zweiten Verschlussteil (1, 2), die miteinander verbindbar sind, um die Verschlussvorrichtung (V) zu schließen, und voneinander lösbar sind, um die Verschlussvorrichtung (V) zu öffnen, wobei
- das zweite Verschlussteil (2) zum Schließen der Verschlussvorrichtung (V) entlang einer Verbindungsachse (A) in einer Schließrichtung (S) an das erste Verschlussteil (1) ansetzbar ist,
- die Verschlussvorrichtung (V) wenigstens zwei Magnetelemente (M1, M2; M3, M4) aufweist, die beim Ansetzen der Verschlussteile (1, 2) aneinander magnetisch anziehend zwischen dem ersten Verschlussteil (1) und dem zweiten Verschlussteil (2) wirken, und
- das erste Verschlussteil (1) ein erstes Verbindungselement (12) und das zweite Verschlussteil (2) ein zweites Verbindungselement (22) aufweisen, wobei bei geschlossener Verschlussvorrichtung (V) das erste und zweite Verschlussteil (1, 2) über die ersten und zweiten Verbindungselemente (12, 22) aneinander gehalten sind,
**dadurch gekennzeichnet, dass**
das erste Verbindungselement (12) mindestens einen Führungsabschnitt (121) aufweist und das zweite Verbindungselement (22) mindestens einen Verschlussabschnitt (221) aufweist, wobei
- der Führungsabschnitt (121) eine zur Verbindungsachse (A) geneigte Führungsfläche (1210) aufweist, mit der der Verschlussabschnitt (221) beim Ansetzen des zweiten Verschlussteils (2) an das erste Verschlussteil (1) in Kontakt tritt und die dem zweiten Verbindungselement (22) eine Drehung um die Verbindungsachse (A) relativ zu dem ersten Verbindungselement (12) entlang einer ersten Drehrichtung (D1) aufzwingt, wenn die ersten und zweiten Verschlussteile (1, 2) einander weiter angenähert werden,
- der Führungsabschnitt (121) in der ersten Drehrichtung (D1) ein Ende aufweist, so dass der Verschlussabschnitt (221) in Schließrichtung (S) an dem Führungsabschnitt (121) vorbeiführbar ist, bevor das zweite Verbindungselement (22) eine Zwischenstellung relativ zu dem ersten Verbindungselement (12) erreicht,
- die Verschlussvorrichtung (V) eine Verstelleinrichtung (M3, M4; 24) aufweist, über die das zweite Verbindungselement (22) in der Zwischenstellung mit einer Kraft in eine zu der ersten Drehrichtung (D1) entgegengesetzte, zweite Drehrichtung (D2) beaufschlagt ist, so dass das zweite Verbindungselement (22) aus der Zwischenstellung selbsttätig relativ zu dem ersten Verbindungselement (12) und um die Verbindungsachse (A) entlang der zweiten Drehrichtung (D2) in eine Schließstellung gedreht wird, und
- in der Schließstellung der Verschlussabschnitt (221) den Führungsabschnitt (121) zumindest teilweise hintergreift, um die ersten und zweiten Verbindungselemente (12, 22) und damit die ersten und zweiten Verschlussteile (1, 2) aneinander zu halten, und für ein Lösen der ersten und zweiten Verschlussteile (1, 2) voneinander das zweite Verbindungselement (22) in die erste Drehrichtung (D1) um die Verbindungsachse (A) relativ zu dem ersten Verbindungselement (12) drehbar ist um die Verschlussvorrichtung (V) zu öffnen.

2. Verschlussvorrichtung, mit wenigstens einem ersten und einem zweiten Verschlussteil (1, 2), die miteinander verbindbar sind, um die Verschlussvorrichtung (V) zu schließen, und voneinander lösbar sind, um die Verschlussvorrichtung (V) zu öffnen, wobei
- das zweite Verschlussteil (2) zum Schließen der Verschlussvorrichtung (V) entlang einer Verbindungsachse (A) in einer Schließrichtung (S) an das erste Verschlussteil (1) ansetzbar ist,
- die Verschlussvorrichtung (V) wenigstens zwei Magnetelemente (M1, M2; M3, M4) aufweist, die beim Ansetzen der Verschlussteile (1, 2) aneinander magnetisch anziehend zwischen dem ersten Verschlussteil (1) und dem zweiten Verschlussteil (2) wirken, und
- das erste Verschlussteil (1) ein erstes Verbindungselement (12) und das zweite Verschlussteil (2) ein zweites Verbindungselement (22) aufweisen, wobei bei geschlossener Verschlussvorrichtung (V) das erste und zweite Verschlussteil (1, 2) über die ersten und zweiten Verbindungselemente (12, 22) aneinander gehalten sind,
**dadurch gekennzeichnet, dass**
das erste Verbindungselement (12) mindestens einen Führungsabschnitt (121) und einen Halteabschnitt (124) aufweist und das zweite Verbindungselement (22) mindestens einen ersten und einen zweiten Verschlussabschnitt (221) aufweist, wobei
- der Führungsabschnitt (121) eine zur Verbindungsachse (A) geneigte Führungsfläche (1210) aufweist, mit der der erste Verschlussabschnitt (221) beim Ansetzen des zweiten Verschlussteils (2) an das erste Verschlussteil (1) in Kontakt tritt und die dem zweiten Verbindungselement (22) eine Drehung um die Verbindungsachse (A) relativ zu dem ersten Verbindungselement (12) entlang einer ersten Drehrichtung (D1) aufzwingt, wenn die ersten und zweiten Verschlussteile (1, 2) einander weiter angenähert werden,
- der Führungsabschnitt (121) und der Halteabschnitt (124) in der ersten Drehrichtung (D1) jeweils ein Ende aufweisen, so dass der erste Verschlussabschnitt (221) in Schließrichtung (S) an dem Führungsabschnitt (121) und der andere, zweite Verschlussabschnitt an dem Halteabschnitt (124) vorbeiführbar sind, bevor das zweite Verbindungselement (22) eine Zwischenstellung relativ zu dem ersten Verbindungselement (12) erreicht,
- die Verschlussvorrichtung (V) eine Verstelleinrichtung (M3, M4; 24) aufweist, über die das zweite Verbindungselement (22) in der Zwischenstellung mit einer Kraft in eine zu der ersten Drehrichtung (D1) entgegengesetzte, zweite Drehrichtung (D2) beaufschlagt ist, so dass das zweite Verbindungselement (22) aus der Zwischenstellung selbsttätig relativ zu dem ersten Verbindungselement (12) und um die Verbindungsachse (A) entlang der zweiten Drehrichtung (D2) in eine Schließstellung gedreht wird, und
- in der Schließstellung der zweite Verschlussabschnitt (221) den Halteabschnitt (124) zumindest teilweise hintergreift, um die ersten und zweiten Verbindungselemente (12, 22) und damit die ersten und zweiten Verschlussteile (1, 2) aneinander zu halten, und für ein Lösen der ersten und zweiten Verschlussteile (1, 2) voneinander das zweite Verbindungselement (22) in die erste Drehrichtung (D1) um die Verbindungsachse (A) relativ zu dem ersten Verbindungselement (12) drehbar ist, um die Verschlussvorrichtung (V) zu öffnen.

3. Verschlussvorrichtung nach Anspruch 1 oder 2, **dadurch gekennzeichnet, dass** die Verstelleinrichtung (M3, M4; 24) federkraftbasiert und/oder magnetkraftbasiert ist, um das zweite Verbindungselement (22) in der Zwischenstellung mit einer Federkraft und/oder einer Magnetkraft in die zweite Drehrichtung (D2) zu beaufschlagen.

4. Verschlussvorrichtung nach einem der Ansprüche 1 bis 3, **dadurch gekennzeichnet, dass** wenigstens ein Magnetelement (M3, M4) zwei verschiedene Magnetpole in einer im Wesentlichen senkrecht zur Schließrichtung (S) verlaufenden Ebene aufweist oder an einem Verschlussteil (1, 2) wenigstens zwei Magnetelemente derart vorgesehen sind, dass hierüber zwei verschiedene Magnetpole in einer im Wesentlichen senkrecht zur Schließrichtung (S) verlaufenden Ebene vorliegen.

5. Verschlussvorrichtung nach Anspruch 4, **dadurch gekennzeichnet, dass**
- eine Drehung des zweiten Verbindungselements (22) relativ zu dem ersten Verbindungselement (12) aus der Schließstellung in die erste Drehrichtung (D1) über die Zwischenstellung hinaus zugelassen ist und
- jedes Verschlussteil (1, 2) wenigstens ein Magnetelement (M3, M4) mit zwei verschiedenen Magnetpolen oder wenigstens zwei Magnetelemente derart aufweist, dass an dem Verschlussteil (1; 2) zwei verschiedene Magnetpole in einer im Wesentlichen senkrecht zur Schließrichtung (S) verlaufenden Ebene vorliegen,
wobei die Magnetelemente (M3, M4) der ersten und zweiten Verschlussteile (1, 2) derart an den ersten und zweiten Verschlussteilen (1, 2) angeordnet sind, dass die Magnetelemente (M3, M4)
(a) beim Schließen der Verschlussvorrichtung (V) magnetisch anziehend wirken und in der Zwischenstellung des zweiten Verbindungselements (12, 22) derart zueinander angeordnet sind, dass die beiden Verbindungselemente (12, 22) aufgrund der wirkenden Magnetkräfte bestrebt sind, eine der Schließstellung entsprechende Relativlage zueinander einzunehmen, und
(b) beim Öffnen der Verschlussvorrichtung (V) magnetisch abstoßend wirken, wenn das zweite Verbindungselement (22) aus der Schließstellung relativ zu dem ersten Verbindungselement (12) in die erste Drehrichtung (D1) oder die zweite Drehrichtung (D2) über die Zwischenstellung hinaus gedreht wird.

6. Verschlussvorrichtung nach einem der vorhergehenden Ansprüche, **dadurch gekennzeichnet, dass** der Führungsabschnitt (121) zwei unterschiedliche Führungsflächen (1210, 1211) derart aufweist, dass beim Schließen der Verschlussvorrichtung (V) eine Führung des zweiten Verbindungselements (22) an der einen zur Verbindungsachse (A) geneigten Führungsfläche (1210) für die Drehung des zweiten Verbindungselements (22) in die erste Drehrichtung (D1) erfolgt und beim Öffnen der Verschlussvorrichtung (V) eine Führung an der anderen Führungsfläche (1211) für die Drehung in die erste Drehrichtung (D1), und zwar derart, dass beim Öffnen der Verschlussvorrichtung (V) die zwei Verschlussteile (1, 2) entgegen der Schließrichtung schraubend getrennt werden.

7. Verschlussvorrichtung nach einem der vorhergehenden Ansprüche, **dadurch gekennzeichnet, dass** das erste Verschlussteil (1) mindestens einen ersten Verdrehsicherungsabschnitt (123) umfasst und das zweite Verschlussteil (2) mindestens einen zweiten Verdrehsicherungsabschnitt (231) umfasst, wobei an dem zweiten Verschlussteil (2) das zweite Verbindungselement (22) relativ zu dem zweiten Verdrehsicherungsabschnitt (231) drehbar ist und der mindestens eine erste Verdrehsicherungsabschnitt (123) und der mindestens eine zweite Verdrehsicherungsabschnitt (231) beim Schließen der Verschlussvorrichtung (V) drehfest miteinander verbindbar sind.

8. Verschlussvorrichtung nach den Ansprüchen 3 und 7, **dadurch gekennzeichnet, dass** das zweite Verschlussteil (2) wenigstens ein Federelement (24) als Teil einer federkraftbasierten Verstelleinrichtung aufweist, wobei sich das Federelement (24) einerseits an dem Verbindungselement (22) und andererseits an einem den zweiten Verdrehsicherungsabschnitt (231) aufweisenden Verdrehsicherungselement (23) des zweiten Verschlussteils (2) abstützt.

9. Verschlussvorrichtung nach Anspruch 7 oder 8, **dadurch gekennzeichnet, dass** das zweite Verschlussteil (2) ein Betätigungselement (25) aufweist, das relativ zu dem zweiten Verdrehsicherungsabschnitt (231) und relativ zu dem zweiten Verbindungselement (22) drehbar ist und das bei geschlossener Verschlussvorrichtung (V) in die erste Drehrichtung (D1) um die Verbindungsachse (A) drehbar ist, um auf das zweite Verbindungselement (22) einzuwirken und das zweite Verbindungselement (22) relativ zu dem ersten Verbindungselement (12) zu drehen.

10. Verschlussvorrichtung nach einem der Ansprüche 1 bis 8, **dadurch gekennzeichnet, dass** die Verschlussvorrichtung (V) eine Stellwegbegrenzung mit wenigstens einem Anschlag (227) an dem zweiten Verbindungselement (22) und wenigstens einem Gegenanschlag (271a, 271b) derart aufweist, dass eine Drehung des zweiten Verbindungselements (22) beim Öffnen und/oder beim Schließen der Verschlussvorrichtung (V) durch Kontakt von Anschlag (227) und Gegenanschlag (271a, 271b) auf einen vorgegebenen maximalen Drehwinkel begrenzt ist.

11. Verschlussvorrichtung nach einem der vorhergehenden Ansprüche, **dadurch gekennzeichnet, dass**, in der Schließstellung, der Führungsabschnitt (121) und der Verschlussabschnitt (221) bezogen auf die Verbindungsachse (A) axial relativ zueinander um ein definiertes Spiel verlagerbar sind, der Führungsabschnitt (121) und der Verschlussabschnitt (221) aber gegen eine Drehung relativ zueinander um die Verbindungsachse (A) durch miteinander, nach Überbrückung des Spiels zusammenwirkende Sperrteile (1212, 2212) der ersten und zweiten Verbindungselemente (12, 22) gesperrt sind, wenn in der Schließstellung des zweiten Verbindungselements (22) an dem ersten und/oder zweiten Verschlussteil (1, 2) eine entlang der Verbindungsachse (A) wirkende Kraft angreift, die das jeweilige Verschlussteil (1; 2) in eine von dem anderen Verschlussteil (2; 1) weg weisende Richtung belastet.

12. Halterung für die Befestigung eines Gegenstands an einem Zwei- oder Dreirad, insbesondere in Form einer Lenkerhalterung, mit einer Verschlussvorrichtung nach einem der vorhergehenden Ansprüche.

13. Halterung für die Befestigung eines elektronischen Geräts, insbesondere eines Mobilgeräts, an einem Trägerelement, mit einer Verschlussvorrichtung nach einem der vorhergehenden Ansprüche 1 bis 11, wobei eines der Verschlussteile (1, 2) mit dem Trägerelement und das andere der Verschlussteile (1, 2) mit dem elektronischen Gerät zu verbinden ist.

14. Kleidungs-, Schuh- oder Prothesenverschluss mit einer Verschlussvorrichtung nach einem der vorhergehenden Ansprüche 1 bis 11.

15. Verwendung einer Verschlussvorrichtung nach einem der vorhergehenden Ansprüche 1 bis 11 als Teil eines Kleidungs-, Schuh- oder Prothesenverschluss.

## Claims

1. A closure device with at least one first and one second closure part (1, 2) which are connectable together in order to close the closure device (V), and are releasable from one another in order to open the closure device (V), wherein
- the second closure part (2) is movable toward the first closure part (1) in a closing direction (S) along a connection axis (A) for closing the closure device (V),
- the closure device (V) comprises at least two magnetic elements (M1, M2; M3, M4) which, when the closure parts (1, 2) are moved toward one another, cause magnetic attraction between the first closure part (1) and the second closure part (2), and
- the first closure part (1) comprises a first connection element (12) and the second closure part (2) a second connection element (22), wherein, with the closure device (V) closed, the first and second closure parts (1, 2) are held together by means of the first and second connection elements (12, 22),
**characterized in that**
the first connection element (12) comprises at least one guide portion (121) and the second connection element (22) at least one closure portion (221), wherein
- the guide portion (121) comprises a guide surface (1210) which is angled toward the connection axis (A), is contacted by the closure portion (221) when the second closure part (2) is moved toward the first closure part (1) and which forces the second connection element (22) to rotate about the connection axis (A) relative to the connection element (12) along a first direction of rotation (D1) when the first and second closure parts (1, 2) are moved closer together,
- the guide portion (121) comprises an end in the first direction of rotation (D1) such that the closure portion (221) is guidable past the guide portion (121) in the closing direction (S) before the second connection element (22) reaches an intermediate position relative to the first connection element (12),
- the closure device (V) comprises an adjusting device (M3, M4; 24) by means of which the second connection element (22) in the intermediate position is acted upon with a force in a second direction of rotation (D2) opposite the first direction of rotation (D1) such that the second connection element (22) is automatically rotated out of the intermediate position relative to the first connection element (12) and about the connection axis (A) along the second direction of rotation (D2) into a closed position, and
- in the closed position the closure portion (221) engages behind the guide portion (121) at least in part in order to hold the first and second connection elements (12, 22) and consequently the first and second closure parts (1, 2) together, and for releasing the first and second closure parts (1, 2) from one another, the second connection element (22) is rotatable in the first direction of rotation (D1) about the connection axis (A) relative to the first connection element (12) in order to open the closure device (V).

2. The closure device, with at least one first and one second closure part (1, 2) which are connectable together in order to close the closure device (V), and are releasable from one another in order to open the closure device (V), wherein
- the second closure part (2) is movable toward the first closure part (1) in a closing direction (S) along a connection axis (A) for closing the closure device (V),
- the closure device (V) comprises at least two magnetic elements (M1, M2; M3, M4) which, when the closure parts (1, 2) are moved toward one another, cause magnetic attraction between the first closure part (1) and the second closure part (2), and
- the first closure part (1) comprises a first connection element (12) and the second closure part (2) a second connection element (22), wherein, with the closure device (V) closed, the first and second closure parts (1, 2) are held together by means of the first and second connection elements (12, 22),
**characterized in that**
the first connection element (12) comprises at least one guide portion (121) and one holding portion (124) and the second connection element (22) at least one first and one second closure portion (221), wherein
- the guide portion (121) comprises a guide surface (1210) which is angled toward the connection axis (A), is contacted by the closure portion (221) when the second closure part (2) is moved to the first closure part (1) and which forces the second connection element (22) to rotate about the connection axis (A) relative to the connection element (12) along a first direction of rotation (D1) when the first and second closure parts (1, 2) are moved closer together,
- the guide portion (121) and the holding portion (124) each comprise an end in the first direction of rotation (D1) such that the first closure portion (221) is guidable past the guide portion (121) in the closing direction (S) and the other second closure portion past the holding portion (124) before the second connection element (22) reaches an intermediate position relative to the first connection element (12),
- the closure device (V) comprises an adjusting device (M3, M4; 24) by means of which the second connection element (22) in the intermediate position is acted upon with a force in a second direction of rotation (D2) opposite the first direction of rotation (D1) such that the second connection element (22) is automatically rotated out of the intermediate position relative to the first connection element (12) and about the connection axis (A) along the second direction of rotation (D2) into a closed position, and
- in the closed position the second closure portion (221) engages behind the holding portion (124) at least in part in order to hold the first and second connection elements (12, 22) and consequently the first and second closure parts (1, 2) together, and for releasing the first and second closure parts (1, 2) from one another, the second connection element (22) is rotatable in the first direction of rotation (D1) about the connection axis (A) relative to the first connection element (12) in order to open the closure device (V).

3. The closure device as claimed in claim 1 or 2, **characterized in that** the adjusting device (M3, M4; 24) is based on spring force and/or based on magnetic force in order to act upon the second connection element (22) in the intermediate position with a spring force and/or a magnetic force in the second direction of rotation (D2).

4. The closure device as claimed in one of claims 1 to 3, **characterized in that** at least one magnetic element (M3, M4) comprises two different magnetic poles in a plane which extends substantially perpendicularly to the closing direction (S) or at least two magnetic elements are provided on a closure part (1, 2) in such a manner that in this way two different magnetic poles are present in a plane which extends substantially perpendicularly to the closing direction (S).

5. The closure device as claimed in claim 4, **characterized in that**
- rotation of the second connection element (22) relative to the first connection element (12) out of the closed position in the first direction of rotation (D1) is permitted beyond the intermediate position and
- each closure part (1, 2) comprises at least one magnetic element (M3, M4) with two different magnetic poles or at least two magnetic poles in such a manner that two different magnetic poles are present on the closure part (1; 2) in a plane which extends substantially perpendicularly to the closing direction (S),
wherein the magnetic elements (M3, M4) of the first and second closure parts (1, 2) are arranged in such a manner on the first and second closure parts (1, 2) that the magnetic elements (M3, M4)
(a) magnetically attract when the closure device (V) is closed and, with the second connection element (12, 22) in the intermediate position, are arranged in such a manner with respect to one another that the two connection elements (12, 22), on account of the acting magnetic forces, strive to assume a relative position with respect to one another which corresponds to the closed position, and
(b) magnetically repel when the closure device (V) is opened when the second connection element (22) is rotated out of the closed position relative to the first connection element (12) in the first direction of rotation (D1) or is rotated in the second direction of rotation (D2) beyond the intermediate position.

6. The closure device as claimed in one of the preceding claims, **characterized in that** the guide portion (121) comprises two different guide surfaces (1210, 1211) in such a manner that when the closure device (V) is closed, the second connection element (22) is guided on the one guide surface (110), which is angled toward the connection axis (A), for the rotation of the second connection element (22) in the first direction of rotation (D1) and when the closure device (V) is opened, guiding occurs on the other guide surface (1211) for the rotation in the first direction of rotation (D1) in such a manner that when the closure device (V) is opened, the two closure parts (1, 2) are separated in a screwing manner in a direction opposite the closing direction.

7. The closure device as claimed in one of the preceding claims, **characterized in that** the first closure part (1) includes at least one first anti-rotation portion (123) and the second closure part (2) includes at least one second anti-rotation portion (231), wherein the second connection element (22) is rotatable on the second closure part (2) relative to the second anti-rotation portion (231) and the at least one first anti-rotation portion (123) and the at least one second anti-rotation portion (231) are non-rotatably connectable together when the closure device (V) is closed.

8. The closure device as claimed in claims 3 and 7, **characterized in that** the second closure part (2) comprises at least one spring element (24) as part of a spring force-based adjusting device, wherein the spring element (24) is supported at the one end on the connection element (22) and at the other end on an anti-rotation element (23) of the second closure part (2) comprising the second anti-rotation portion (231).

9. The closure device as claimed in claim 7 or 8, **characterized in that** the second closure part (2) comprises an actuating element (25) which is rotatable relative to the second anti-rotation portion (231) and relative to the second connection element (22) and which, with the closure device (V) closed, is rotatable in the first direction of rotation (D1) about the connection axis (A) in order to act upon the second connection element (22) and to rotate the second connection element (22) relative to the first connection element (12).

10. The closure device as claimed in one of the claims 1 to 8, **characterized in that** the closure device (V) comprises a travel limitation with at least one stop (227) on the second connection element (22) and at least one counter stop (271a, 271b) in such a manner that rotation of the second connection element (22) when opening and/or when closing the closure device (V) is limited to a predetermined maximum rotational angle as a result of contact with the stop (227) and counter stop (271a, 271b).

11. The closure device as claimed in one of the preceding claims, **characterized in that**, in the closed position, the guide portion (121) and the closure portion (221) are displaceable axially relative to one another by a defined clearance with reference to the connection axis (A), the guide portion (121) and the closure portion (221), however, are blocked against rotation relative to one another about the connection axis (A) by blocking parts (1212, 2212) of the first and second connection elements (12, 22) which interact after bridging the clearance when, with the second connection element (22) in the closed position, a force, which loads the respective closure part (1; 2) in a direction pointing away from the other closure part (2; 1), cooperates with the first and/or second closure part (1, 2) along the connection axis (A).

12. A holder for securing an object on a two-wheeler or three-wheeler, in particular in the form of a handlebar holder, having a closure device as claimed in one of the preceding claims.

13. The holder for securing an electronic device, in particular a mobile device, on a carrier element, having a closure device as claimed in one of preceding claims 1 to 11, wherein one of the closure parts (1, 2) is to be connected to the carrier element and the other of the closure parts (1, 2) to the electronic device.

14. A closure for clothes, shoes or prostheses having a closure device as claimed in one of preceding claims 1 to 11.

15. A use of a closure device as claimed in one of preceding claims 1 to 11 as part of a closure for clothes, shoes or prostheses.

## Revendications

1. Dispositif de fermeture avec au moins une première et une deuxième partie de fermeture (1, 2), qui peuvent être reliées l'une à l'autre pour fermer le dispositif de fermeture (V) et peuvent être détachées l'une de l'autre pour ouvrir le dispositif de fermeture (V), dans lequel
- la deuxième partie de fermeture (2) peut être placée contre la première partie de fermeture (1) pour fermer le dispositif de fermeture (V) le long d'un axe de liaison (A) dans une direction de fermeture (S),
- le dispositif de fermeture (V) présente au moins deux éléments magnétiques (M1, M2 ; M3, M4), qui agissent avec une attraction magnétique entre la première partie de fermeture (1) et la deuxième partie de fermeture (2) lors du placement des parties de fermeture (1, 2) l'une contre l'autre, et
- la première partie de fermeture (1) présente un premier élément de liaison (12) et la deuxième partie de fermeture (2) présente un deuxième élément de liaison (22), dans lequel lorsque le dispositif de fermeture (V) est fermé, la première et la deuxième partie de fermeture (1, 2) sont maintenues l'une contre l'autre par l'intermédiaire des premiers et deuxièmes éléments de liaison (12, 22),
**caractérisé en ce que**
le premier élément de liaison (12) présente au moins une section de guidage (121) et le deuxième élément de liaison (22) présente au moins une section de fermeture (221), dans lequel
- la section de guidage (121) présente une surface de guidage (1210) inclinée par rapport à l'axe de liaison (A), avec laquelle la section de fermeture (221) entre en contact lors du placement de la deuxième partie de fermeture (2) contre la première partie de fermeture (1) et qui impose au deuxième élément de liaison (22) une rotation autour de l'axe de liaison (A) par rapport au premier élément de liaison (12) le long d'une première direction de rotation (D1) quand les premières et deuxièmes parties de fermeture (1, 2) sont approchées davantage les unes des autres,
- la section de guidage (121) présente dans la première direction de rotation (D1) une extrémité de sorte que la section de fermeture (221) peut être guidée le long de la section de guidage (121) dans la direction de fermeture (S) avant que le deuxième élément de liaison (22) n'atteigne une position intermédiaire par rapport au premier élément de liaison (12),
- le dispositif de fermeture (V) présente un système d'ajustement (M3, M4 ; 24), par l'intermédiaire duquel le deuxième élément de liaison (22) est soumis dans la position intermédiaire à l'action d'une force dans une deuxième direction de rotation (D2) opposée à la première direction de rotation (D1) de sorte que le deuxième élément de liaison (22) est tourné depuis la position intermédiaire de manière autonome par rapport au premier élément de liaison (12) et autour de l'axe de liaison (A) le long de la deuxième direction de rotation (D2) dans une position de fermeture, et
- dans la position de fermeture, la section de fermeture (221) vient en prise par l'arrière au moins en partie avec la section de guidage (121) pour maintenir les uns contre les autres les premiers et deuxièmes éléments de liaison (12, 22) et ainsi les premières et deuxièmes parties de fermeture (1, 2) et, en vue d'un détachement des premières et deuxièmes parties de fermeture (1, 2) les unes des autres, le deuxième élément de liaison (22) peut être tourné dans la première direction de rotation (D1) autour de l'axe de liaison (A) par rapport au premier élément de liaison (12) pour ouvrir le dispositif de fermeture (V).

2. Dispositif de fermeture avec au moins une première et une deuxième partie de fermeture (1, 2), qui peuvent être reliées l'une à l'autre pour fermer le dispositif de fermeture (V) et peuvent être détachées les unes des autres pour ouvrir le dispositif de fermeture (V), dans lequel
- la deuxième partie de fermeture (2) peut être placée contre la première partie de fermeture (1) pour fermer le dispositif de fermeture (V) le long d'un axe de liaison (A) dans une direction de fermeture (S),
- le dispositif de fermeture (V) présente au moins deux éléments magnétiques (M1, M2 ; M3, M4), qui agissent avec une attraction magnétique entre la première partie de fermeture (1) et la deuxième partie de fermeture (2) lorsque les parties de fermeture (1, 2) sont placées les unes contre les autres, et
- la première partie de fermeture (1) présente un premier élément de liaison (12) et la deuxième partie de fermeture (2) présente un deuxième élément de liaison (22), dans lequel lorsque le dispositif de fermeture (V) est fermé, la première et la deuxième partie de fermeture (1, 2) sont maintenues l'une contre l'autre par l'intermédiaire des premiers et deuxièmes éléments de liaison (12, 22),
**caractérisé en ce que**
le premier élément de liaison (12) présente au moins une section de guidage (121) et une section de maintien (124) et le deuxième élément de liaison (22) présente au moins une première et une deuxième section de fermeture (221), dans lequel
- la section de guidage (121) présente une surface de guidage (1210) inclinée par rapport à l'axe de liaison (A), avec laquelle la première section de fermeture (221) entre en contact lorsque la deuxième partie de fermeture (2) est placée contre la première partie de fermeture (1) et qui impose au deuxième élément de liaison (22) une rotation autour de l'axe de liaison (A) par rapport au premier élément de liaison (12) le long d'une première direction de rotation (D1) quand les premières et deuxièmes parties de fermeture (1, 2) sont approchées davantage les unes des autres,
- la section de guidage (121) et la section de maintien (124) présentent dans la première direction de rotation (D1) respectivement une extrémité, de sorte que la première section de fermeture (221) peut être guidée dans la direction de fermeture (S) le long de la section de guidage (121) et l'autre deuxième section de fermeture peut être guidée le long de la section de maintien (124) avant que le deuxième élément de liaison (22) n'atteigne une position intermédiaire par rapport au premier élément de liaison (12),
- le dispositif de fermeture (V) présente un système d'ajustement (M3, M4 ; 24), par l'intermédiaire duquel le deuxième élément de liaison (22) est soumis dans la position intermédiaire à l'action d'une force dans une deuxième direction de rotation (D2) opposée à la première direction de rotation (D1) de sorte que le deuxième élément de liaison (22) est tourné depuis la position intermédiaire de manière autonome par rapport au premier élément de liaison (12) et autour de l'axe de rotation (A) le long de la deuxième direction de rotation (D2) dans une position de fermeture, et
- dans la position de fermeture, la deuxième section de fermeture (221) vient au moins en partie en prise par l'arrière avec la section de maintien (124) pour maintenir les uns contre les autres les premiers et deuxièmes éléments de liaison (12, 22) et ainsi les premières et deuxièmes parties de fermeture (1, 2) et, en vue d'un détachement des premières et deuxièmes parties de fermeture (1, 2) les unes des autres, le deuxième élément de liaison (22) peut être tourné dans la première direction de rotation (D1) autour de l'axe de liaison (A) par rapport au premier élément de liaison (12) pour ouvrir le dispositif de fermeture (V).

3. Dispositif de fermeture selon la revendication 1 ou 2, **caractérisé en ce que** le système d'ajustement (M3, M4 ; 24) se base sur une force élastique et/ou une force magnétique pour soumettre le deuxième élément de liaison (22) dans la position intermédiaire à l'action d'une force élastique et/ou d'une force magnétique dans la deuxième direction de rotation (D2).

4. Dispositif de fermeture selon l'une quelconque des revendications 1 à 3, **caractérisé en ce qu'**au moins un élément magnétique (M3, M4) présente deux pôles magnétiques différents dans un plan s'étendant sensiblement de manière perpendiculaire par rapport à la direction de fermeture (S) ou au moins deux éléments magnétiques sont prévus au niveau d'une partie de fermeture (1, 2) de telle manière que par cet intermédiaire deux pôles magnétiques différents sont présents dans un plan s'étendant sensiblement de manière perpendiculaire par rapport à la direction de fermeture (S).

5. Dispositif de fermeture selon la revendication 4, **caractérisé en ce que**
- une rotation du deuxième élément de liaison (22) par rapport au premier élément de liaison (12) depuis la position de fermeture dans la première direction de rotation (D1) est autorisée au-delà de la position intermédiaire, et
- chaque partie de fermeture (1, 2) présente au moins un élément magnétique (M3, M4) avec deux pôles magnétiques différents ou au moins deux éléments magnétiques de telle manière que deux pôles magnétiques différents sont présents dans un plan s'étendant sensiblement de manière perpendiculaire par rapport à la direction de fermeture (S) au niveau de la partie de fermeture (1 ; 2),
dans lequel les éléments magnétiques (M3, M4) des premières et deuxièmes parties de fermeture (1, 2) sont disposés de telle manière au niveau des premières et deuxièmes parties de fermeture (1, 2) que les éléments magnétiques (M3, M4)
(a) agissent avec une attraction magnétique lors de la fermeture du dispositif de fermeture (V) et sont disposés les uns par rapport aux autres dans la position intermédiaire du deuxième élément de liaison (12, 22) de telle manière que les deux éléments de liaison (12, 22) tendent en raison des forces magnétiques à l'œuvre à adopter l'un par rapport à l'autre une position relative correspondant à la position de fermeture, et
(b) agissent en se repoussant les uns les autres magnétiquement lors de l'ouverture du dispositif de fermeture (V) quand le deuxième élément de liaison (22) est tourné depuis la position de fermeture par rapport au premier élément de liaison (12) dans la première direction de rotation (D1) ou la deuxième direction de rotation (D2) au-delà de la position intermédiaire.

6. Dispositif de fermeture selon l'une quelconque des revendications précédentes, **caractérisé en ce que** la section de guidage (121) présente deux surfaces de guidage (1210, 1211) différentes de telle manière que lors de la fermeture du dispositif de fermeture (V), un guidage du deuxième élément de liaison (22) a lieu au niveau de la surface de guidage (1210) inclinée par rapport à l'axe de liaison (A) pour la rotation du deuxième élément de liaison (22) dans la première direction de rotation (D1) et lors de l'ouverture du dispositif de fermeture (V), un guidage au niveau de l'autre surface de guidage (1211) a lieu pour la rotation dans la première direction de rotation (D1), à savoir de telle manière que lors de l'ouverture du dispositif de fermeture (V), les deux parties de fermeture (1, 2) sont séparées par vissage dans le sens opposé à la direction de fermeture.

7. Dispositif de fermeture selon l'une quelconque des revendications précédentes, **caractérisé en ce que** la première partie de fermeture (1) comprend au moins une première section anti-rotation (123) et la deuxième partie de fermeture (2) comprend au moins une deuxième section anti-rotation (231), dans lequel le deuxième élément de liaison (22) peut être tourné par rapport à la deuxième section anti-rotation (231) au niveau de la deuxième partie de fermeture (2) et l'au moins une première section anti-rotation (123) et l'au moins une deuxième section anti-rotation (231) peuvent être reliées l'une à l'autre de manière solidaire en rotation lors de la fermeture du dispositif de fermeture (V).

8. Dispositif de fermeture selon les revendications 3 et 7, **caractérisé en ce que** la deuxième partie de fermeture (2) présente au moins un élément élastique (24) en tant que partie d'un système d'ajustement se basant sur une force élastique, dans lequel l'élément élastique (24) prend appui d'une part au niveau de l'élément de liaison (22) et d'autre part au niveau d'un élément anti-rotation (23), présentant la deuxième section anti-rotation (231), de la deuxième partie de fermeture (2).

9. Dispositif de fermeture selon la revendication 7 ou 8, **caractérisé en ce que** la deuxième partie de fermeture (2) présente un élément d'actionnement (25), qui peut tourner par rapport à la deuxième section anti-rotation (231) et par rapport au deuxième élément de liaison (22) et qui, lorsque le dispositif de fermeture (V) est fermé, peut tourner dans la première direction de rotation (D1) autour de l'axe de liaison (A) pour agir sur le deuxième élément de liaison (22) et pour faire tourner le deuxième élément de liaison (22) par rapport au premier élément de liaison (12).

10. Dispositif de fermeture selon l'une quelconque des revendications 1 à 8, **caractérisé en ce que** le dispositif de fermeture (V) présente une limitation de course de réglage avec au moins une butée (227) au niveau du deuxième élément de liaison (22) et au moins une contre-butée (271a, 271b) de telle manière qu'une rotation du deuxième élément de liaison (22) est limitée à un angle de rotation maximal prédéfini par contact de la butée (227) et de la contre-butée (271a, 271b) lors de l'ouverture et/ou lors de la fermeture du dispositif de fermeture (V).

11. Dispositif de fermeture selon l'une quelconque des revendications précédentes, **caractérisé en ce que**, dans la position de fermeture, la section de guidage (121) et la section de fermeture (221) peuvent être déplacées axialement l'une par rapport à l'autre d'un jeu défini par rapport à l'axe de liaison (A), la section de guidage (121) et la section de fermeture (221) sont bloquées toutefois pour empêcher toute rotation l'une par rapport à l'autre autour de l'axe de liaison (A) par des parties de blocage (1212, 2212), coopérant entre elles une fois le jeu surmonté, des premiers et deuxièmes éléments de liaison (12, 22) quand, dans la position de fermeture du deuxième élément de liaison (22), une force agissant le long de l'axe de liaison (A) s'engage au niveau de la première et/ou deuxième partie de fermeture (1, 2), qui contraint la partie de fermeture (1 ; 2) respective dans une direction pointant de manière à s'éloigner de l'autre partie de fermeture (2 ; 1).

12. Support pour la fixation d'un objet au niveau d'un bicycle ou d'un tricycle, en particulier sous la forme d'un support de guidon, avec un dispositif de fermeture selon l'une quelconque des revendications précédentes.

13. Support pour la fixation d'un appareil électronique, en particulier d'un appareil mobile, au niveau d'un élément porteur, avec un dispositif de fermeture selon l'une quelconque des revendications précédentes 1 à 11, dans lequel une des parties de fermeture (1, 2) est à relier à l'élément porteur et l'autre des parties de fermeture (1, 2) est à relier à l'appareil électronique.

14. Fermeture de vêtement, de chaussure ou de prothèse avec un dispositif de fermeture selon l'une quelconque des revendications 1 à 11.

15. Utilisation d'un dispositif de fermeture selon l'une quelconque des revendications 1 à 11 en tant que partie d'une fermeture de vêtement, de chaussure ou de prothèse.
